(19) Europäisches Patentamt
European Patent Office
Office européen des brevets

(11) EP 4 470 569 A1

(12) EUROPEAN PATENT APPLICATION
published in accordance with Art. 153(4) EPC

(43) Date of publication:
**04.12.2024 Bulletin 2024/49**

(21) Application number: **23746232.0**

(22) Date of filing: **19.01.2023**

(51) International Patent Classification (IPC):
***A61K 47/68*** *(2017.01)* ***A61K 31/58*** *(2006.01)*
***C07K 16/22*** *(2006.01)* ***A61P 37/00*** *(2006.01)*

(52) Cooperative Patent Classification (CPC):
**A61K 47/6849; A61K 47/68; A61K 47/6803; A61P 37/00; C07K 16/2866;** C07K 2317/24; C07K 2317/55; C07K 2317/77; C07K 2317/92

(86) International application number:
**PCT/CN2023/073057**

(87) International publication number:
**WO 2023/143351 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.01.2022 CN 202210110349**

(71) Applicants:
- **Shanghai Shengdi Pharmaceutical Co., Ltd Shanghai 201210 (CN)**
- **Shanghai Senhui Medicine Co., Ltd. Shanghai 201203 (CN)**
- **Jiangsu Hengrui Pharmaceuticals Co., Ltd. Lianyungang, Jiangsu 222047 (CN)**

(72) Inventors:
- **REN, Wenming Shanghai 201210 (CN)**
- **ZHU, Lingjian Shanghai 201203 (CN)**
- **CHEN, Hao Shanghai 201210 (CN)**
- **TANG, Manping Shanghai 201210 (CN)**
- **LIN, Yuan Shanghai 201210 (CN)**
- **ZHOU, Caihong Shanghai 201210 (CN)**
- **HUANG, Jian Shanghai 201203 (CN)**
- **LIAO, Cheng Shanghai 201210 (CN)**

(74) Representative: **Potter Clarkson Chapel Quarter Mount Street Nottingham NG1 6HQ (GB)**

Remarks:
The complete document including Reference Table(s) and the Sequence Listing(s) can be downloaded from the EPO website

# (54) DRUG CONJUGATE OF GLUCOCORTICOID

(57) Disclosed is an antibody-drug conjugate represented by formula (I), wherein Ab is an antibody or an antigen-binding fragment thereof, L is a linker covalently linking Ab to D, k is 1 to 20, and D is a glucocorticoid or a residue thereof, the glucocorticoid being selected from budesonide and ciclesonide.

$$Ab\text{-}(L\text{-}D)_k \qquad (I)$$

EP 4 470 569 A1

## Description

### TECHNICAL FIELD

The present disclosure pertains to the field of pharmaceutics and particularly relates to a drug conjugate of a glucocorticoid.

### BACKGROUND

Interleukin-4 (IL-4) is composed of 153 amino acids and has a molecular weight of approximately 17 kDa. IL-4 was initially discovered for its ability to stimulate B cell proliferation and was thus named B cell stimulating factor-1 (BSF-1). IL-4 belongs to the type I cytokine family as IL-13 does and has a quaternary structure consisting of a hydrophobic 4-α-helix bundle core. Secreted by TH2 cells, IL-4 plays a role in TH2-mediated immune responses and has a wide range of biological activities, including stimulating the proliferation of T cells, mast cells, granulocytes, megakaryocytes, and erythrocytes. Moreover, IL-4 can also stimulate B cells' expression of class 2 molecules of the major histocompatibility complex. IL-13 has about 30% amino acid sequence homology with IL-4, and they have several similar functions. Both IL-4 and IL-13 can promote B cell proliferation and, in conjunction with CD40/CD40L co-stimulation, induce the class switch from IgM to IgE. IL-4 promotes the aggregation of mast cells, upregulates the expression of high-affinity IgE receptor on mast cells and low-affinity IgE receptor CD23 (FcεRII) on B cells, upregulates the expression of vascular cell adhesion molecule (VCAM-1), and facilitates the migration of eosinophils, T lymphocytes, monocytes, and basophils. Unlike IL-13, IL-4 can promote the differentiation of naive T cells to TH2.

IL-4 needs to bind to membrane receptors to exert its biological functions. The human interleukin receptor (IL-4R) is a heterodimer formed from two polypeptide chains, including one α chain that has a very high affinity for IL-4. In IL-4R complexes, the IL-4Rα chain plays a dominant role in the binding to IL-4, leading to the frequent substitution of IL-4Rα for IL-4R in scientific research and reports. IL-4R is expressed on various cell types, including human B cells, mast cells, eosinophils, basophils, macrophages/monocytes, DC cells, fibroblasts, airway epithelial cells, smooth muscle cells, etc. IL-4Rα can form two types of receptor complexes with other subunits. In hematopoietic stem cells, the type I receptor, composed of IL-4Rα and γc, is mainly expressed. In non-hematopoietic stem cells, IL-4 mainly functions through the type II receptor, composed of IL-4Rα and IL-13Rα1. The type II receptor is a common receptor for both IL-4 and IL-13. IL-13 functions by binding to IL-13Rα1. Both the type I and type II receptors signal through the Jak/STAT pathway. IL-4Rα, γc, and IL-13Rα1 bind to Jak1, Jak3, and Tyk2, respectively, to activate downstream pathways. IL-4 and IL-13 can also signal through the insulin receptor substrate (IRS) family, ultimately activating PI3-K and NF-κB in the nucleus. Blocking IL-4R can inhibit the biological functions of both IL-4 and IL-13. Several studies have shown that IL-4 and IL-13 are associated with diseases associated with TH2 immune responses. Atopic dermatitis (AD), also known as atopic eczema or genetic allergic dermatitis, is a common skin disease in dermatology, primarily affecting children and adolescents, often concurrent with certain genetic allergic diseases such as allergic rhinitis and asthma. Research has found elevated levels of TH2 cytokines IL-4, IL-5, IL-10, and IL-13 and elevated IgE levels in AD patients. Additionally, it has been found that TH2 cytokines are related to AD progression and that mice overexpressing TH2 factors such as IL-4 and IL-13 exhibit skin protection defects and AD-like disorders[14][15] . Elevated levels of IL-4 and IL-13 in AD patients hinder epidermal differentiation and antimicrobial peptide production. IL-4 deficient mice show a reduction in skin allergic inflammation. These studies suggest that blocking IL-4R could be effective in treating AD. There have already been anti-IL-4R mAbs available abroad, showing good therapeutic effects on AD.

IL-13 and IL-4 also play important roles in asthma. Asthma is a common lung inflammation disease characterized by airway hyperresponsiveness (AHR), mucus hypersecretion, fibrosis, and elevated IgE levels. Non-specific stimuli such as cold air often increase airway hyperresponsiveness (AHR) and cause mucus hypersecretion, resulting in airway obstruction, the leading cause of death from asthma. The TH2 factor plays a crucial role in the progression of asthma. Overexpression of IL-4 and IL-13 is observed in bronchial and alveolar lavage fluids of asthma patients. Although IL-13 and IL-4 share some functional similarities, certain studies have shown that IL-13 plays a more critical role in the progression of asthma than other Th2 cytokines. IL-13 can promote the differentiation of goblet cells and fibrosis. Injecting recombinant IL-13 into the airways of mice not previously exposed to allergens can cause airway inflammation, mucus hypersecretion, and airway hyperresponsiveness. Injecting soluble IL13Rα2 can prevent AHR, mucus hypersecretion, and lung inflammation in mice. Injecting an IL-4Rα antibody in asthma models can reduce AHR and the eosinophil count in alveolar lavage fluid. Studies have shown that blocking IL-4Rα could be effective in treating asthma.

A number of pharmaceutical companies worldwide are currently developing monoclonal antibodies against IL-4R; related patent applications are, e.g., WO2010053751, WO2001092340, WO2008054606, WO2014031610, and WO2020038454.

Glucocorticoids are also relatively effective drugs for treating allergic diseases, inflammations, etc. Known glucocorticoids produced within organisms, such as cortisol and corticosterone, and synthetic glucocorticoids, such as

dexamethasone, prednisone, prednisolone, and budesonide, are typical glucocorticoids. These glucocorticoids are collectively referred to as steroids for their steroid structures. They are used in the treatment of various diseases. However, these steroids may sometimes cause side effects such as steroid peptic ulcer, steroid purpura, steroid pancreatitis, steroid diabetes, steroid cataract, and steroid glaucoma due to their use.

An antibody-drug conjugate (ADC) is a molecule in which a monoclonal antibody or an antibody fragment is linked to a biologically active drug by a stable chemical linker compound. Most ADCs in preclinical and clinical development are used for oncological indications. The cytotoxic payloads target cancer cells expressing antigens. However, the regulation of pathogenic cellular activity through ADC-mediated delivery of biologically active small molecules is also appealing to non-oncological indications, thereby leading to the widespread use of this technology.

Some drug conjugates of glucocorticoids have been disclosed in the prior art, e.g., WO2017210471, WO2019106609, and WO2019136487.

## SUMMARY

In one aspect, the present disclosure provides an antibody-drug conjugate (ADC) represented by formula (I),

$$Ab\text{-}(L\text{-}D)_k \qquad (I)$$

wherein Ab is an anti-IL-4R antibody or an antigen-binding fragment thereof;

L is a linker covalently linking Ab to D, and k is 1 to 20 (including 1, 2, 3, 4, 5, 6, 7, 8, 9, 10, 11, 12, 13, 14, 15, 16, 17, 18, 19, 20, or any value between any two values);

D is a glucocorticoid or a residue thereof, and the glucocorticoid is selected from the group consisting of dexamethasone, prednisone, prednisolone, budesonide, mometasone, beclomethasone dipropionate, fluticasone, triamcinolone acetonide, and ciclesonide; for example, the glucocorticoid may be budesonide or ciclesonide.

In some embodiments, D is represented by the following formula:

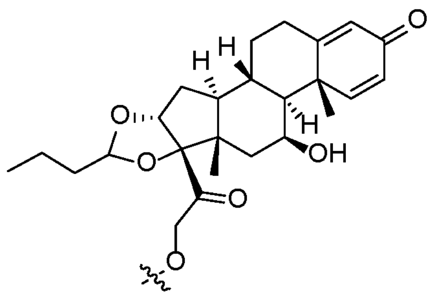

In some embodiments, the anti-IL-4R antibody or the antigen-binding fragment thereof may be known, such as those described, for example, in WO2010053751, WO2001092340, WO2008054606, WO2014031610, and WO2020038454, each of which is incorporated herein by reference.

In some embodiments, the anti-IL-4R antibody or the antigen-binding fragment thereof includes, but is not limited to, Dupixent, PRS-060, AK-120, 63 IgG1, CBP201, AMG-317, or an antigen-binding fragment thereof.

In some embodiments, the anti-IL-4R antibody or the antigen-binding fragment thereof is an antihuman IL-4R antibody or an antigen-binding fragment thereof.

In some embodiments, the anti-IL-4R antibody or the antigen-binding fragment thereof comprises a heavy chain variable region and a light chain variable region, wherein the heavy chain variable region comprises:

(I) a HCDR1, a HCDR2, and a HCDR3 set forth in SEQ ID NO: 3, SEQ ID NO: 4, and SEQ ID NO: 5, respectively; or
(II) a HCDR1, a HCDR2, and a HCDR3 set forth in SEQ ID NO: 11, SEQ ID NO: 12, and SEQ ID NO: 13, respectively;

and/or the light chain variable region of the antibody comprises:

(I) a LCDR1, a LCDR2, and a LCDR3 set forth in SEQ ID NO: 6, SEQ ID NO: 7, and SEQ ID NO: 8, respectively; or
(II) a LCDR1, a LCDR2, and a LCDR3 set forth in SEQ ID NO: 14, SEQ ID NO: 15, and SEQ ID NO: 16, respectively; or
(III) a LCDR1, a LCDR2, and a LCDR3 set forth in SEQ ID NO: 38, SEQ ID NO: 7, and SEQ ID NO: 40, respectively; or
(IV) a LCDR1, a LCDR2, and a LCDR3 set forth in SEQ ID NO: 42, SEQ ID NO: 39, and SEQ ID NO: 8, respectively.

Table 1 shows the CDR sequences of the anti-IL-4R antibody or the antigen-binding fragment thereof. Table 1. The CDR sequences of the anti-IL-4R antibody or the antigen-binding fragment thereof

| Name | Sequence | No. |
|---|---|---|
| HCDR1 | GFTFSDYGMH | SEQ ID NO: 3 |
| HCDR2 | FISSGSSIIYYADIVKG | SEQ ID NO: 4 |
| HCDR3 | GNKRGFFDY | SEQ ID NO: 5 |
| LCDR1 | NASSSVSYMY | SEQ ID NO: 6 |
| LCDR2 | LTSNLAS | SEQ ID NO: 7 |
| LCDR3 | QQWRSNPPMLT | SEQ ID NO: 8 |
| HCDR1 | GYTFTSYWMH | SEQ ID NO: 11 |
| HCDR2 | LIHPNSDTTKFSENFKT | SEQ ID NO: 12 |
| HCDR3 | SKIITTIVARHWYFDV | SEQ ID NO: 13 |
| LCDR1 | KASQSVDYGGDSYMN | SEQ ID NO: 14 |
| LCDR2 | AASNLES | SEQ ID NO: 15 |
| LCDR3 | QHSNENPPT | SEQ ID NO: 16 |
| LCDR1 | RASSSVPYMY | SEQ ID NO: 38 |
| LCDR2 | LASSRPS | SEQ ID NO: 39 |
| LCDR3 | QQWRAYPPMLT | SEQ ID NO: 40 |
| LCDR1 | RASPGVPPLA | SEQ ID NO: 42 |

In some embodiments, the anti-IL-4R antibody or the antigen-binding fragment thereof comprises a combination selected from the group consisting of any one of the following (I) to (IV):

(I) a heavy chain variable region, comprising a HCDR1, a HCDR2, and a HCDR3 set forth in SEQ ID NO: 3, SEQ ID NO: 4, and SEQ ID NO: 5, respectively; and
a light chain variable region, comprising a LCDR1, a LCDR2, and a LCDR3 set forth in SEQ ID NO: 6, SEQ ID NO: 7, and SEQ ID NO: 8, respectively; and
(II) a heavy chain variable region, comprising a HCDR1, a HCDR2, and a HCDR3 set forth in SEQ ID NO: 11, SEQ ID NO: 12, and SEQ ID NO: 13, respectively; and
a light chain variable region, comprising a LCDR1, a LCDR2, and a LCDR3 set forth in SEQ ID NO: 14, SEQ ID NO: 15, and SEQ ID NO: 16, respectively;
(III) a heavy chain variable region, comprising a HCDR1, a HCDR2, and a HCDR3 set forth in SEQ ID NO: 3, SEQ ID NO: 4, and SEQ ID NO: 5, respectively; and
a light chain variable region, comprising a LCDR1, a LCDR2, and a LCDR3 set forth in SEQ ID NO: 38, SEQ ID NO: 7, and SEQ ID NO: 40, respectively;
(IV) a heavy chain variable region, comprising a HCDR1, a HCDR2, and a HCDR3 set forth in SEQ ID NO: 3, SEQ ID NO: 4, and SEQ ID NO: 5, respectively; and
a light chain variable region, comprising a LCDR1, a LCDR2, and a LCDR3 set forth in SEQ ID NO: 42, SEQ ID NO: 39, and SEQ ID NO: 8, respectively.

In some embodiments, the anti-IL-4R antibody or the antigen-binding fragment is a murine antibody, a chimeric antibody, a fully human antibody, a humanized antibody, or a fragment thereof. In some specific embodiments, the anti-IL-4R antibody or the antigen-binding fragment is humanized.

In some embodiments, the anti-IL-4R antibody or the antigen-binding fragment thereof comprises a FR sequence derived from a human germline light chain IGKV3-11*01 (SEQ ID NO: 22, used for antibody 25G7) or a sequence with a back mutation that has at least 95% identity thereto. In some specific embodiments, the back mutation is selected from the group consisting of one or more of L46P, L47W, and F71Y. In some embodiments, the anti-IL-4R antibody or the antigen-binding fragment thereof comprises a FR sequence derived from a human germline heavy chain IGHV3-48*01 (SEQ ID NO: 21, used for antibody 25G7) or a sequence with a back mutation that has at least 95% identity thereto. In some specific embodiments, the back mutation is selected from the group consisting of one or more of

S94A, F67S, and A93T. In some embodiments, the anti-IL-4R antibody or the antigen-binding fragment thereof comprises a FR sequence derived from a human germline light chain IGKV2D-29*01 (SEQ ID NO: 24, used for antibody 7B10) or a back-mutated sequence having at least 95% identity thereto. In some specific embodiments, the back mutation is selected from the group consisting of M4L and/or V58I. In some embodiments, the anti-IL-4R antibody or the antigen-binding fragment thereof comprises a FR sequence derived from a human germline heavy chain IGHV1-2*02 (SEQ ID NO: 23, used for antibody 7B10) or a back-mutated sequence having at least 95% identity thereto. In some specific embodiments, the back mutation is selected from the group consisting of one or more of M69L, R71I, T73K, and R94K.

Human germline heavy chain IGHV3-48*01:

EVQLVESGGGLVQPGGSLRLSCAASGFTFSSYSMNWVRQAPGKGLEWVSYISSSSSTIYYA
DSVKGRFTISRDNAKNSLYLQMNSLRAEDTAVYYCAR
SEQ ID NO: 21

Human germline light chain IGKV3-11*01:

EIVLTQSPATLSLSPGERATLSCRASQSVSSYLAWYQQKPGQAPRLLIYDASNRATGIPARFSG
SGSGTDFTLTISSLEPEDFAVYYCQQRSNWP
SEQ ID NO: 22

Human germline heavy chain IGHV1-2*02:

QVQLVQSGAEVKKPGASVKVSCKASGYTFTGYYMHWVRQAPGQGLEWMGWINPNSGGT
NYAQKFQGRVTMTRDTSISTAYMELSRLRSDDTAVYYCAR
SEQ ID NO: 23

Human germline light chain IGKV2D-29*01:

DIVMTQTPLSLSVTPGQPASISCKSSQSLLHSDGKTYLYWYLQKPGQPPQLLIYEVSNRFSGV
PDRFSGSGSGTDFTLKISRVEAEDVGVYYCMQSIQLP
SEQ ID NO: 24

In some embodiments, the anti-IL-4R antibody or the antigen-binding fragment thereof comprises a heavy chain variable region and a light chain variable region, wherein:

the heavy chain variable region comprises:

(I) the sequence set forth in SEQ ID NO: 1 or a sequence having at least 70%, 80%, 90%, 95%, 98%, or 99% identity to SEQ ID NO: 1; or
(II) the sequence set forth in SEQ ID NO: 9 or a sequence having at least 70%, 80%, 90%, 95%, 98%, or 99% identity to SEQ ID NO: 9; or
(III) the sequence set forth in SEQ ID NO: 43 or a sequence having at least 70%, 80%, 90%, 95%, 98%, or 99% identity to SEQ ID NO: 43; or

and/or the light chain variable region comprises:

(I) the sequence set forth in SEQ ID NO: 2 or a sequence having at least 70%, 80%, 90%, 95%, 98%, or 99% identity to SEQ ID NO: 2; or
(II) the sequence set forth in SEQ ID NO: 10 or a sequence having at least 70%, 80%, 90%, 95%, 98%, or 99% identity to SEQ ID NO: 10; or
(III) the sequence set forth in SEQ ID NO: 37 or a sequence having at least 70%, 80%, 90%, 95%, 98%, or 99% identity to SEQ ID NO: 37; or
(IV) the sequence set forth in SEQ ID NO: 41 or a sequence having at least 70%, 80%, 90%, 95%, 98%, or 99% identity to SEQ ID NO: 41.

25G7 HCVR (25G7 heavy chain variable region)

EVQLVESGGGLVKPGGSLKLSCAASGFTFSDYGMHWVRQAPEKGLEWVAFISSGSSIIYYA
DIVKGRSTISRDNAKNTLFLQMTSLRSEDTAMYYCTRGNKRGFFDYWGQGTILTVSS
SEQ ID NO: 1

25G7 LCVR (25G7 light chain variable region)

QIVLTQSPALMSASPGEKVTMTCNASSSVSYMYWYQRKPRSSPKPWIYLTSNLASGVPVRF
SGSGSGTSYSLTISSMEAEDAATYYCQQWRSNPPMLTFGSGTKLEVK
SEQ ID NO: 2

7B10 HCVR (7B10 heavy chain variable region)

QVQLQQPGTELLKPGASVSLSCKASGYTFTSYWMHWVKQRPGQGLEWIGLIHPNSDTTKF
SENFKTRATLTIDKSSSTAYMKLSSLTSEDSAVYYCAKSKIITTIVARHWYFDVWGTGTTVTV
SS
SEQ ID NO: 9

7B10 LCVR (7B10 light chain variable region)

DIVLTQSPPSLAVSLGQRATISCKASQSVDYGGDSYMNWYQQKLGQPPKVLIYAASNLESGI
PARFSGSGSGTDFTLNIHPVEEEDVATYYCQHSNENPPTFGGGTKLEIK
SEQ ID NO: 10

hu25G7-ALCVR (hu25G7-A light chain variable region)

EIVLTQSPATLSLSPGERATLSCRASSSVPYMYWYQQKPGQAPRLLIYLTSNLASGIPARFSGS
GSGTDFTLTISSLEPEDFAVYYCQQWRAYPPMLTFGGGTKVEIK
SEQ ID NO: 37

hu25G7-B LCVR (hu25G7-B light chain variable region)

EIVLTQSPATLSLSPGERATLSCRASPGVPPLAWYQQKPGQAPRLLIYLASSRPSGIPARFSGS

GSGTDFTLTISSLEPEDFAVYYCQQWRSNPPMLTFGGGTKVEIK
SEQ ID NO: 41

hu25G7-VH (hu25G7 heavy chain variable region)

EVQLVESGGGLVQPGGSLRLSCAASGFTFSDYGMHWVRQAPGKGLEWVAFISSGSSIIYYA
DIVKGRSTISRDNAKNTLYLQMNSLRAEDTAVYYCTRGNKRGFFDYWGQGTLVTVSS
SEQ ID NO: 43

In at least one embodiment, the heavy chain variable region of the anti-IL-4R antibody or the antigen-binding fragment is set forth in the sequence SEQ ID NO: 1, and the light chain variable region is set forth in the sequence SEQ ID NO: 2; or

the heavy chain variable region is set forth in the sequence SEQ ID NO: 9, and the light chain variable region is set forth in the sequence SEQ ID NO: 10; or

the heavy chain variable region is set forth in the sequence SEQ ID NO: 43, and the light chain variable region is set forth in the sequence SEQ ID NO: 37; or
the heavy chain variable region is set forth in the sequence SEQ ID NO: 43, and the light chain variable region is set forth in the sequence SEQ ID NO: 41.

In some embodiments, the anti-IL-4R antibody or the antigen-binding fragment thereof comprises a heavy chain variable region and a light chain variable region, wherein:

the heavy chain variable region comprises:

(I) the sequence set forth in any one of SEQ ID NOs: 25-27 or a sequence having at least 70%, 80%, 90%, 95%, 98%, or 99% identity to any one of SEQ ID NOs: 25-27; or
(II) the sequence set forth in any one of SEQ ID NOs: 31-33 or a sequence having at least 70%, 80%, 90%, 95%, 98%, or 99% identity to any one of SEQ ID NOs: 31-33;

and/or the light chain variable region comprises:

(I) the sequence set forth in any one of SEQ ID NOs: 28-30 or a sequence having at least 70%, 80%, 90%, 95%, 98%, or 99% identity to any one of SEQ ID NOs: 28-30; or
(II) the sequence set forth in any one of SEQ ID NOs: 34-36 or a sequence having at least 70%, 80%, 90%, 95%, 98%, or 99% identity to any one of SEQ ID NOs: 34-36.

hu25G7-VH-a:

EVQLVESGGGLVQPGGSLRLSCAASGFTFSDYGMHWVRQAPGKGLEWVAFISSGSSIIYYA
DIVKGRFTISRDNAKNSLYLQMNSLRAEDTAVYYCARGNKRGFFDYWGQGTLVTVSS
SEQ ID NO: 25

hu25G7-VH-b:

EVQLVESGGGLVQPGGSLRLSCAASGFTFSDYGMHWVRQAPGKGLEWVAFISSGSSIIYYA
DIVKGRFTISRDNAKNSLYLQMNSLRAEDTAVYYCTRGNKRGFFDYWGQGTLVTVSS
SEQ ID NO: 26

hu25G7-VH-c:

EVQLVESGGGLVQPGGSLRLSCAASGFTFSDYGMHWVRQAPGKGLEWVAFISSGSSIIYYA
DIVKGRSTISRDNAKNSLYLQMNSLRAEDTAVYYCTRGNKRGFFDYWGQGTLVTVSS

SEQ ID NO: 27

hu25G7-VL-a:

EIVLTQSPATLSLSPGERATLSCNASSSVSYMYWYQQKPGQAPRLLIYLTSNLASGIPARFSGS
GSGTDFTLTISSLEPEDFAVYYCQQWRSNPPMLTFGGGTKVEIK
SEQ ID NO: 28

hu25G7-VL-b:

EIVLTQSPATLSLSPGERATLSCNASSSVSYMYWYQQKPGQAPRLLIYLTSNLASGIPARFSGS
GSGTDYTLTISSLEPEDFAVYYCQQWRSNPPMLTFGGGTKVEIK
SEQ ID NO: 29

hu25G7-VL-c:

EIVLTQSPATLSLSPGERATLSCNASSSVSYMYWYQQKPGQAPRPWIYLTSNLASGIPARFSG SGSGTDYTLTISSLEPEDFAVYYCQQWRSNPPMLTFGGGTKVEIK
SEQ ID NO: 30

hu7B 10-VH-a:

EVQLVQSGAEVKKPGASVKVSCKASGYTFTSYWMHWVRQAPGQGLEWMGLIHPNSDTT KFSENFKTRVTMTRDTSISTAYMELSRLRSDDTAVYYCARSKIITTIVARHWYFDVWGQGTT VTVSS
SEQ ID NO: 31

hu7B10-VH-b:

EVQLVQSGAEVKKPGASVKVSCKASGYTFTSYWMHWVRQAPGQGLEWMGLIHPNSDTT KFSENFKTRVTMTIDTSISTAYMELSRLRSDDTAVYYCAKSKIITTIVARHWYFDVWGQGTT VTVSS
SEQ ID NO: 32

hu7B 10-VH-c:

EVQLVQSGAEVKKPGASVKVSCKASGYTFTSYWMHWVRQAPGQGLEWMGLIHPNSDTT KFSENFKTRVTLTIDKSISTAYMELSRLRSDDTAVYYCAKSKIITTIVARHWYFDVWGQGTTV TVSS
SEQ ID NO: 33

hu7B 10-VL-a:

DIVMTQTPLSLSVTPGQPASISCKASQSVDYGGDSYMNWYLQKPGQPPQLLIYAASNLESG VPDRFSGSGSGTDFTLKISRVEAEDVGVYYCQHSNENPPTFGGGTKVEIK
SEQ ID NO: 34

hu7B 10-VL-b:

DIVLTQTPLSLSVTPGQPASISCKASQSVDYGGDSYMNWYLQKPGQPPQLLIYAASNLESGV PDRFSGSGSGTDFTLKISRVEAEDVGVYYCQHSNENPPTFGGGTKVEIK
SEQ ID NO: 35

hu7B 10-VL-c:

DIVMTQTPLSLSVTPGQPASISCKASQSVDYGGDSYMNWYLQKPGQPPQLLIYAASNLESGI PDRFSGSGSGTDFTLKISRVEAEDVGVYYCQHSNENPPTFGGGTKVEIK
SEQ ID NO: 36

In some specific embodiments, the heavy chain variable region is set forth in any one of the sequences SEQ ID NOs: 25-27, and the light chain variable region is set forth in any one of the sequences SEQ ID NOs: 28-30;
the heavy chain variable region is set forth in any one of the sequences SEQ ID NOs: 31-33, and the light chain variable region is set forth in any one of the sequences SEQ ID NOs: 34-36.

In some embodiments, the anti-IL-4R antibody or the antigen-binding fragment thereof comprises a heavy chain constant region selected from the group consisting of heavy chain constant regions of human IgG1, IgG2, IgG3, and IgG4, or a variant thereof. In some specific embodiments, the anti-IL-4R antibody or the antigen-binding fragment thereof comprises a heavy chain constant region of human IgG1, or a variant thereof. In some embodiments, the anti-IL-4R antibody or the antigen-binding fragment thereof comprises a constant region of a human κ or λ chain or a variant thereof.

In some embodiments, provided is the anti-IL-4R antibody or the antigen-binding fragment thereof, wherein the antibody is a humanized antibody; the heavy chain sequence is set forth in SEQ ID NO: 17 or has at least 85% sequence identity thereto, and the light chain sequence is set forth in SEQ ID NO: 18 or has at least 85% sequence identity thereto.

In some embodiments, provided is the anti-IL-4R antibody or the antigen-binding fragment thereof, wherein the antibody is a humanized antibody; the heavy chain sequence is set forth in SEQ ID NO: 19 or has at least 85% sequence identity thereto, and the light chain sequence is set forth in SEQ ID NO: 20 or has at least 85% sequence identity thereto.

In some embodiments, provided is the anti-IL-4R antibody or the antigen-binding fragment thereof, wherein the antibody is a humanized antibody; the heavy chain sequence is set forth in SEQ ID NO: 44 or has at least 85% sequence identity thereto, and the light chain sequence is set forth in SEQ ID NO: 45 or has at least 85% sequence identity thereto.

In some embodiments, provided is the anti-IL-4R antibody or the antigen-binding fragment thereof, wherein the antibody is a humanized antibody; the heavy chain sequence is set forth in SEQ ID NO: 44 or has at least 85% sequence identity thereto, and the light chain sequence is set forth in SEQ ID NO: 46 or has at least 85% sequence identity thereto.

In some embodiments, provided is an isolated anti-IL-4R antibody or an antigen-binding fragment thereof, competing for binding to human IL-4R or an epitope thereof with any one of the anti-IL-4R antibodies or the antigen-binding fragments thereof described above.

In some embodiments, provided is a bispecific antibody or a multispecific antibody, comprising the light chain variable region and/or the heavy chain variable region of any one of the anti-IL-4R antibodies or the antigen-binding fragments thereof described above. In some other embodiments, provided is a single-chain antibody, comprising the light chain variable region and/or the heavy chain variable region of any one of the anti-IL-4R antibodies or the antigen-binding fragments thereof described above.

In some embodiments, the humanized anti-IL-4R antibody or the antigen-binding fragment thereof also comprises a heavy chain constant region of human IgG1, IgG2, IgG3, or IgG4, or a variant thereof. In at least one embodiment, the humanized anti-IL-4R antibody or the antigen-binding fragment thereof comprises a human IgG2 or IgG4 heavy chain constant region, because IgG2 or IgG4 has no ADCC toxicity. In another embodiment, IgG1 that has no ADCC (antibody-dependent cell-mediated cytotoxicity) toxicity after amino acid mutation is used. In at least one embodiment, the variant comprises a heavy chain constant region mutation leading to reduced or absent ADCC effector function, such as, but not limited to N297A, L234A, and L235A of IgG1. In some embodiments, IgG1 comprises the mutations E239D and M241L.

Unless otherwise indicated, the anti-IL-4R antibody or the antigen-binding protein thereof of the present disclosure is encoded according to Kabat.

In some embodiments, the anti-IL-4R antibody or the antigen-binding fragment thereof comprises a heavy chain and a light chain, wherein the heavy chain comprises:

(I) the sequence set forth in SEQ ID NO: 17 or a sequence having at least 70%, 80%, 90%, 95%, 98%, or 99% identity to SEQ ID NO: 17; or
(II) the sequence set forth in SEQ ID NO: 19 or a sequence having at least 70%, 80%, 90%, 95%, 98%, or 99% identity to SEQ ID NO: 19; or
(III) the sequence set forth in SEQ ID NO: 44 or a sequence having at least 70%, 80%, 90%, 95%, 98%, or 99% identity to SEQ ID NO: 44;
(IV) the sequence set forth in SEQ ID NO: 47 or a sequence having at least 70%, 80%, 90%, 95%, 98%, or 99% identity to SEQ ID NO: 47;

and/or the light chain comprises:

(I) the sequence set forth in SEQ ID NO: 18 or a sequence having at least 70%, 80%, 90%, 95%, 98%, or 99% identity to SEQ ID NO: 18; or
(II) the sequence set forth in SEQ ID NO: 20 or a sequence having at least 70%, 80%, 90%, 95%, 98%, or 99% identity to SEQ ID NO: 20; or
(III) the sequence set forth in SEQ ID NO: 45 or a sequence having at least 90%, 95%, 98%, or 99% identity to SEQ ID NO: 45; or
(IV) the sequence set forth in SEQ ID NO: 46 or a sequence having at least 90%, 95%, 98%, or 99% identity to SEQ ID NO: 46.

hu25G7 HC

EVQLVESGGGLVQPGGSLRLSCAASGFTFSDYGMHWVRQAPGKGLEWVAFISSGSSIIYYA
DIVKGRSTISRDNAKNSLYLQMNSLRAEDTAVYYCTRGNKRGFFDYWGQGTLVTVSSAST
KGPSVFPLAPCSRSTSESTAALGCLVKDYFPEPVTVSWNSGALTSGVHTFPAVLQSSGLYSLS
SVVTVPSSSLGTKTYTCNVDHKPSNTKVDKRVESKYGPPCPPCPAPEFLGGPSVFLFPPKPK
DTLMISRTPEVTCVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVFSCSVMHE
ALHNHYTQ KSLSLSLGK
SEQ ID NO: 17

hu25G7 LC

EIVLTQSPATLSLSPGERATLSCNASSSVSYMYWYQQKPGQAPRLLIYLTSNLASGIPARFSGS
GSGTDFTLTISSLEPEDFAVYYCQQWRSNPPMLTFGGGTKVEIKRTVAAPSVFIFPPSDEQLKS
GTASVVCLLNNFYPREAKVQWKVDNALQSGNSQESVTEQDSKDSTYSLSSTLTLSKADYE
KHKVYACEVTHQGLSSPVTKSFNRGEC
SEQ ID NO: 18

hu7B 10 HC

EVQLVQSGAEVKKPGASVKVSCKASGYTFTSYWMHWVRQAPGQGLEWMGLIHPNSDTT
KFSENFKTRVTMTIDTSISTAYMELSRLRSDDTAVYYCAKSKIITTIVARHWYFDVWGQGTT
VTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELLG
GPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQ
QGNVFSCSVMHEALHNHYTQKSLSLSPGK
SEQ ID NO: 19

hu7B10 LC

DIVLTQTPLSLSVTPGQPASISCKASQSVDYGGDSYMNWYLQKPGQPPQLLIYAASNLESGV
PDRFSGSGSGTDFTLKISRVEAEDVGVYYCQHSNENPPTFGGGTKVEIKRTVAAPSVFIFPPS
DEQLKSGTASVVCLLNNFYPREAKVQWKVDNALQSGNSQESVTEQDSKDSTYSLSSTLTLS
KADYEKHKVYACEVTHQGLSSPVTKSFNRGEC
SEQ ID NO: 20

hu25G7-IgG4 HC

EVQLVESGGGLVQPGGSLRLSCAASGFTFSDYGMHWVRQAPGKGLEWVAFISSGSSIIYYA
DIVKGRSTISRDNAKNTLYLQMNSLRAEDTAVYYCTRGNKRGFFDYWGQGTLVTVSSAST
KGPSVFPLAPCSRSTSESTAALGCLVKDYFPEPVTVSWNSGALTSGVHTFPAVLQSSGLYSLS
SVVTVPSSSLGTKTYTCNVDHKPSNTKVDKRVESKYGPPCPPCPAPEFLGGPSVFLFPPKPK
DTLMISRTPEVTCVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVFSCSVMHE
ALHNHYTQKSLSLSLGK
SEQ ID NO: 44

hu25G7-ALC

EIVLTQSPATLSLSPGERATLSCRASSSVPYMYWYQQKPGQAPRLLIYLTSNLASGIPARFSGS
GSGTDFTLTISSLEPEDFAVYYCQQWRAYPPMLTFGGGTKVEIKRTVAAPSVFIFPPSDEQLK
SGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO: 45

hu25G7-B LC

EIVLTQSPATLSLSPGERATLSCRASPGVPPLAWYQQKPGQAPRLLIYLASSRPSGIPARFSGS
GSGTDFTLTISSLEPEDFAVYYCQQWRSNPPMLTFGGGTKVEIKRTVAAPSVFIFPPSDEQLKS
GTASVVCLLNNFYPREAKVQWKVDNALQSGNSQESVTEQDSKDSTYSLSSTLTLSKADYE
KHKVYACEVTHQGLSSPVTKSFNRGEC
SEQ ID NO: 46

hu25G7-24-IgG1 (E239D, M241L) HC

EVQLVESGGGLVQPGGSLRLSCAASGFTFSDYGMHWVRQAPGKGLEWVAFISSGSSIIYYA
DIVKGRSTISRDNAKNTLYLQMNSLRAEDTAVYYCTRGNKRGFFDYWGQGTLVTVSSAST
KGPSVFPLAPSSKSTSGGTAALGCLVKDYFPEPVTVSWNSGALTSGVHTFPAVLQSSGLYSLS
SVVTVPSSSLGTQTYICNVNHKPSNTKVDKKVEPKSCDKTHTCPPCPAPELLGGPSVFLFPP
KPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSV
MHEALHNHYTQKSLSLSPGK
SEQ ID NO: 47

In some embodiments, the heavy chain sequence is set forth in SEQ ID NO: 17, and the light chain sequence is set forth in SEQ ID NO: 18; or

the heavy chain sequence is set forth in SEQ ID NO: 19, and the light chain sequence is set forth in SEQ ID NO: 20; or
the heavy chain sequence is set forth in SEQ ID NO: 44, and the light chain sequence is set forth in SEQ ID NO: 45; or
the heavy chain sequence is set forth in SEQ ID NO: 44, and the light chain sequence is set forth in SEQ ID NO: 46; or
the heavy chain sequence is set forth in SEQ ID NO: 47, and the light chain sequence is set forth in SEQ ID NO: 45.

In some embodiments, the anti-IL-4R antibody or the antigen-binding fragment thereof comprises a heavy chain

and a light chain, wherein the heavy chain comprises:

(I) the sequence set forth in SEQ ID NO: 48 or a sequence having at least 70%, 80%, 90%, 95%, 98%, or 99% identity to SEQ ID NO: 48; or
(II) the sequence set forth in SEQ ID NO: 49 or a sequence having at least 70%, 80%, 90%, 95%, 98%, or 99% identity to SEQ ID NO: 49; or
(III) the sequence set forth in SEQ ID NO: 50 or a sequence having at least 70%, 80%, 90%, 95%, 98%, or 99% identity to SEQ ID NO: 50;
(IV) the sequence set forth in SEQ ID NO: 51 or a sequence having at least 70%, 80%, 90%, 95%, 98%, or 99% identity to SEQ ID NO: 51;

and/or the light chain comprises:

(I) the sequence set forth in SEQ ID NO: 18 or a sequence having at least 70%, 80%, 90%, 95%, 98%, or 99% identity to SEQ ID NO: 18; or
(II) the sequence set forth in SEQ ID NO: 45 or a sequence having at least 90%, 95%, 98%, or 99% identity to SEQ ID NO: 45; or
(III) the sequence set forth in SEQ ID NO: 46 or a sequence having at least 90%, 95%, 98%, or 99% identity to SEQ ID NO: 46.

hu25G7-IgG4 VH-CH1

EVQLVESGGGLVQPGGSLRLSCAASGFTFSDYGMHWVRQAPGKGLEWVAFISSGSSIIYYA
DIVKGRSTISRDNAKNSLYLQMNSLRAEDTAVYYCTRGNKRGFFDYWGQGTLVTVSSAST

KGPSVFPLAPCSRSTSESTAALGCLVKDYFPEPVTVSWNSGALTSGVHTFPAVLQSSGLYSLS
SVVTVPSSSLGTKTYTCNVDHKPSNTKVDKRV
SEQ ID NO: 48

hu25G7-IgG1 VH-CH1

EVQLVESGGGLVQPGGSLRLSCAASGFTFSDYGMHWVRQAPGKGLEWVAFISSGSSIIYYA
DIVKGRSTISRDNAKNSLYLQMNSLRAEDTAVYYCTRGNKRGFFDYWGQGTLVTVSSAST
KGPSVFPLAPSSKSTSGGTAALGCLVKDYFPEPVTVSWNSGALTSGVHTFPAVLQSSGLYSLS
SVVTVPSSSLGTQTYICNVNHKPSNTKVDKKV
SEQ ID NO: 49

hu25G7-24-IgG4 VH-CH1

EVQLVESGGGLVQPGGSLRLSCAASGFTFSDYGMHWVRQAPGKGLEWVAFISSGSSIIYYA
DIVKGRSTISRDNAKNTLYLQMNSLRAEDTAVYYCTRGNKRGFFDYWGQGTLVTVSSAST
KGPSVFPLAPCSRSTSESTAALGCLVKDYFPEPVTVSWNSGALTSGVHTFPAVLQSSGLYSLS
SVVTVPSSSLGTKTYTCNVDHKPSNTKVDKRV
SEQ ID NO: 50

hu25G7-24-IgG1 VH-CH1

EVQLVESGGGLVQPGGSLRLSCAASGFTFSDYGMHWVRQAPGKGLEWVAFISSGSSIIYYA
DIVKGRSTISRDNAKNTLYLQMNSLRAEDTAVYYCTRGNKRGFFDYWGQGTLVTVSSAST
KGPSVFPLAPSSKSTSGGTAALGCLVKDYFPEPVTVSWNSGALTSGVHTFPAVLQSSGLYSLS
SVVTVPSSSLGTQTYICNVNHKPSNTKVDKKV
SEQ ID NO: 51

In some embodiments, the heavy chain sequence is set forth in SEQ ID NO: 48, and the light chain sequence is set forth in SEQ ID NO: 18; or

the heavy chain sequence is set forth in SEQ ID NO: 49, and the light chain sequence is set forth in SEQ ID NO: 18; or
the heavy chain sequence is set forth in SEQ ID NO: 50, and the light chain sequence is set forth in SEQ ID NO: 45; or
the heavy chain sequence is set forth in SEQ ID NO: 50, and the light chain sequence is set forth in SEQ ID NO: 46; or
the heavy chain sequence is set forth in SEQ ID NO: 51, and the light chain sequence is set forth in SEQ ID NO: 45; or
the heavy chain sequence is set forth in SEQ ID NO: 51, and the light chain sequence is set forth in SEQ ID NO: 46.

In some embodiments, the anti-IL-4R antibody or the antigen-binding fragment thereof may be an antibody variant having 1 to 10 (e.g., 1, 2, 3, 4, 5, 6, 7, 8, 9, or 10) amino acid alterations in the light chain and/or 1 to 10 (e.g., 1, 2, 3, 4, 5, 6, 7, 8, 9, or 10) amino acid alterations in the heavy chain.

In some embodiments, the variant described above has identical or similar biological function or effect to the parent anti-IL-4R antibody or the fragment thereof.

In some embodiments, the antigen-binding fragment includes, but is not limited to, Fab, Fab', Fv, F(ab')2, linear antibody, scFv (single-chain Fv antibody), tandem di-scFv, tandem tri-scFv, diabody, triabody, tetrabody, sdAb (single-domain antibody or nanobody), sdFv, peptibody, domain antibody, multispecific antibody (e.g., bispecific antibody, trispecific antibody, or tetraspecific antibody), dsFv (disulfide-stabilized Fv) and ScdsFv (disulfide-stabilized single-chain Fv antibody).

In some embodiments, the antigen-binding fragment of the anti-IL-4R antibody in the antibody-drug conjugate or the pharmaceutically acceptable salt or solvate thereof of the present disclosure binds to the same IL-4R or epitope thereof as the antigen-binding fragment of the anti-IL-4R antibody described above.

In some embodiments, provided is a polynucleotide, e.g., a DNA or an RNA, encoding the anti-IL-4R antibody or the antigen-binding fragment thereof described above.

In some embodiments, provided is an expression vector, e.g., a eukaryotic expression vector, a prokaryotic expression vector, or a viral vector, comprising the polynucleotide described above.

In some embodiments, provided is a host cell, e.g., a eukaryotic cell or a prokaryotic cell, transformed with the expression vector described above. In some specific embodiments, the host cell is a bacterium (e.g., Escherichia coli), a yeast (e.g., Pichia pastoris), or a mammalian cell (e.g., a Chinese hamster ovary (CHO) cell or a human embryonic kidney (HEK) 293 cell).

In some embodiments, provided is a method for preparing the anti-IL-4R antibody or the antigen-binding fragment thereof described above, comprising the following steps: expressing the antibody or the antigen-binding fragment thereof in the host cell described above, and isolating the antibody or the antigen-binding fragment thereof from the host cell.

The three-letter and single-letter codes for amino acids in the anti-IL-4R antibody or the antigen-binding fragment thereof in the present disclosure are as described in J. Biol. Chem, 243, p3558 (1968).

In certain embodiments, k has any value between 1 and 10, preferably between 2 and 5. k may be an integer or a decimal.

In certain embodiments, the linker is stable outside a cell, such that the ADC remains intact when present in extracellular conditions but is capable of being cleaved upon internalization in a cell. In some embodiments, when the ADC enters a cell that expresses an antigen specific to the antibody moiety of the ADC, the glucocorticoid drug moiety is cleaved from the antibody moiety, and the cleavage leads to the release of an unmodified form of the glucocorticoid.

In certain embodiments, the cleavable moiety in the linker is a cleavable peptide moiety. In some embodiments, an ADC that comprises a cleavable peptide moiety exhibits a lower aggregation level and an improved antibody-to-drug ratio relative to ADCs that comprise other cleavable moieties. In certain embodiments, adding a cleavable moiety increases cytotoxicity and/or potency relative to a non-cleavable linker. In some embodiments, the cleavable peptide moiety is capable of being cleaved by an enzyme, and the linker is a linker capable of being cleaved by an enzyme. In some embodiments, the enzyme is a cathepsin, and the linker is a linker capable of being cleaved by a cathepsin. In certain embodiments, the enzyme-cleavable linker (e.g., the cathepsin-cleavable linker) exhibits one or more of the improved properties described above, as compared to other cleavage mechanisms.

In some embodiments, the linker comprises a stretcher unit, which is a chemical structural fragment one end of which is covalently linked to an antibody by a carbon atom and the other end is linked to an amino acid unit, a disulfide moiety, a sulfonamide moiety, or a non-peptide chemical moiety. Exemplary stretcher units include, but are not limited to,

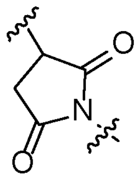, 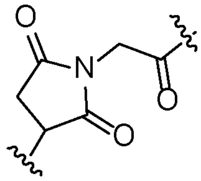, 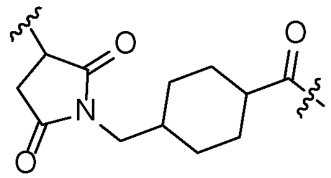, 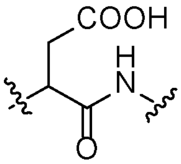, 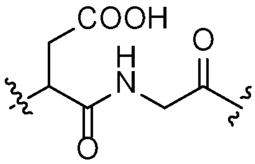,

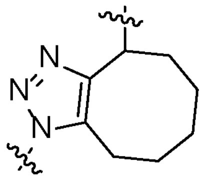, 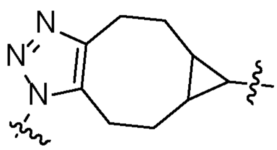, 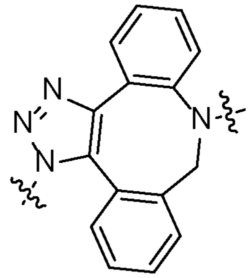, 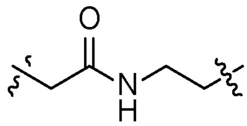,

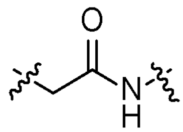, or 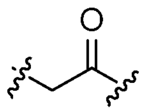

In some embodiments, the linker comprises an amino acid unit, and the amino acid unit preferably comprises a peptide residue consisting of 2 to 7 amino acids selected from the group consisting of phenylalanine, glycine, valine, lysine, citrulline, serine, glutamic acid, aspartic acid, homolysine, n-methyl-valine, and

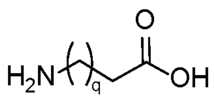

(q is an integer from 1 to 6); exemplary amino acid units include, but are not limited to, valine-citrulline (Val-Cit), alanine-phenylalanine (Ala-Phe), phenylalanine-lysine (Phe-Lys), phenylalanine-homolysine (Phe-Homolys), n-methyl-valine-citrulline (Me-Val-Cit), alanine-alanine (Ala-Ala), glycine-glutamic acid (Gly-Glu), glutamic acid-alanine-alanine (Glu-Ala-Ala), and glycine-lysine (Gly-Lys), glycine-valine-citrulline (Glv-Val-Cit), and glycine-glycine-glycine(Gly-Gly-Gly) and

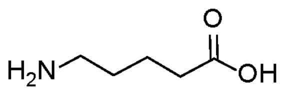

In some embodiments, the linker may comprise at least one polyethylene glycol (PEG) moiety. The PEG moiety may comprise, for example, $-(PEG)_{p1}-$,

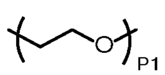,

wherein p1 is an integer from 1 to 20, e.g.,

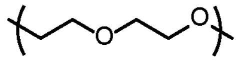,

$(PEG)_2$;

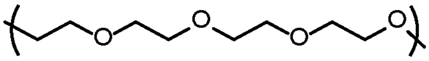

$(PEG)_4$; or

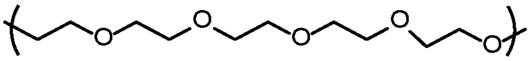

$(PEG)_5$.

In some embodiments, the linker comprises a spacer unit linking to D. In some embodiments, the spacer unit comprises p-aminobenzyloxycarbonyl (PAB),

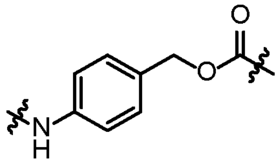

In some embodiments, the spacer unit comprises p-aminobenzoyl,

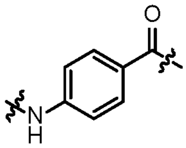

In some embodiments, the spacer unit comprises:

$-NH(CH_2)_{n1}-L^a-L^b-L^c-$, wherein n1 is selected from the group consisting of integers between 0 and 6; $L^a$ represents -C(=O)-NH-, $-NR^7-(CH_2)_{n2}-$, -O-, or a single bond; $R^7$ is selected from the group consisting of hydrogen, $C_{1-6}$ alkyl, $-(CH_2)_{n3}-COOH$, and $-(CH_2)_{n4}-OH$; n3 is selected from the group consisting of integers between 1 and 4; n4 is selected from the group consisting of integers between 1 and 6; $L^b$ represents $-CR^8(R^9)-$, -O-, $-NR^{10}-$, or a single bond; $R^8$ and $R^9$ are independently selected from the group consisting of hydrogen, $C_{1-6}$ alkyl, $C_{3-6}$ cycloalkyl, $-(CH_2)_{n5}-NH_2$, $-(CH_2)_{n6}-COOH$, and $-(CH_2)_{n7}-OH$; $R^{10}$ is selected from the group consisting of hydrogen and $C_{1-6}$ alkyl; n5 is selected from the group consisting of integers between 0 and 6, n6 is selected from the group consisting of integers between 1 and 4, and n7 is selected from the group consisting of integers between 1 and 4; and when n5 is 0, $R^8$ and $R^9$ are different, or $R^8$ and $R^9$, together with the carbon atom to which they are attached, form $C_{3-6}$ cycloalkyl; $L^c$ represents $-CH_2-$ or -C(=O)-.

In some embodiments, in $-NH(CH_2)_{n1}-L^a-L^b-L^c-$, $L^c$ represents $-NHCH_2-$.

In some embodiments, in $-NH(CH_2)_{n1}-L^a-L^b-L^c-$, $R^8$ and $R^9$ are independently selected from the group consisting of hydrogen, $C_{1-6}$ alkyl, and $C_{3-6}$ cycloalkyl, e.g., from the group consisting of hydrogen, methyl, ethyl, and cyclopropyl.

In some embodiments, in $-NH(CH_2)_{n1}-L^a-L^b-L^c-$, $L^a$ represents -O- or a single bond, $L^b$ represents - $CR^8(R^9)$- or a single bond, and $L^c$ represents -C(=O)-; $R^8$ and $R^9$, together with the carbon atom to which they are attached, form $C_{3-6}$ cycloalkyl.

In some embodiments, in $-NH(CH_2)_{n1}-L^a-L^b-L^c-$, $L^a$ represents -O- or a single bond, $L^b$ represents - $CR^8(R^9)$- or a single bond, and $L^c$ represents -C(=O)-; $R^8$ and $R^9$ are independently selected from the group consisting of hydrogen, $C_{1-6}$ alkyl, $C_{3-6}$ cycloalkyl, $-(CH_2)_{n5}-NH_2$, $-(CH_2)_{n6}-COOH$, and - $(CH_2)_{n7}-OH$; $R^{10}$ is selected from the group consisting of hydrogen and $C_{1-6}$ alkyl; n5 is selected from the group consisting of integers between 0 and 6, n6 is selected from the group consisting of integers between 1 and 4, and n7 is selected from the group consisting of integers between 1 and 4; and when n5 is 0, $R^8$ and $R^9$ are different.

In some embodiments, in $-NH(CH_2)_{n1}-L^a-L^b-L^c-$, $L^a$ represents -O- or a single bond, $L^b$ represents - $CR^8(R^9)$- or a single bond, and $L^c$ represents $-CH_2-$.

In some embodiments, in $-NH(CH_2)_{n1}-L^a-L^b-L^c-$, $L^a$ represents -O- or a single bond, $L^b$ represents - $CR^8(R^9)$- or a single bond, and $L^c$ represents $-CH_2-$; $R^8$ and $R^9$ are independently selected from the group consisting of hydrogen, $C_{1-6}$ alkyl, and $C_{3-6}$ cycloalkyl, preferably from the group consisting of hydrogen, methyl, ethyl, and cyclopropyl.

In some embodiments, in the antibody-drug conjugate, $-NH(CH_2)_{n1}-L^a-L^b-L^c-$ is: $-NHCH_2-$, - $NHCH_2CH_2-$, $-NHCH_2CH_2CH_2-$, $-NHCH_2-O-CH_2-$, $-NHCH_2CH_2-O-CH_2-$, $-NH(CH_2)_3-C(O)-$, - $NHCH_2-O-CH_2-C(O)-$, $-NH(CH_2)_2-O-CH_2-C(O)-$,

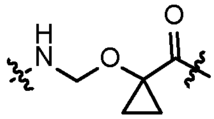,
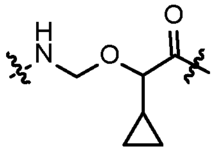,

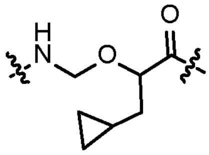
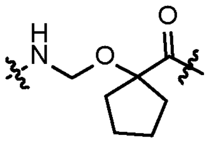,
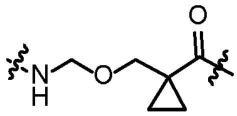,
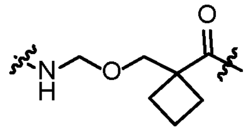, and
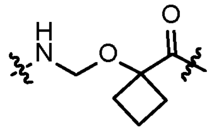

In some embodiments, $-NH-(CH_2)_{n1}L^a-L^b-L^c-$ is $-NHCH_2-$ or $-NHCH_2CH_2CH_2-$.

In some embodiments, the spacer unit in the linker comprises $(PEG)_2$. In some embodiments, the ADC comprising a shorter spacer unit (e.g., $(PEG)_2$) shows a lower aggregation level and/or a higher drug loading than the ADC comprising a longer spacer unit (e.g., $(PEG)_8$) despite the shorter linker length.

In another aspect, in the antibody conjugate (ADC) of the present disclosure, L-D is a chemical moiety represented by the following formula:

-Str-Pep-Sp-D

wherein Str is a stretcher unit covalently linked to Ab;
Sp is a spacer unit;

Pep is selected from the group consisting of an amino acid unit, a disulfide moiety, a sulfonamide moiety, and the following non-peptidic chemical moiety:

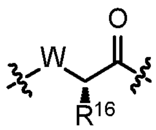,
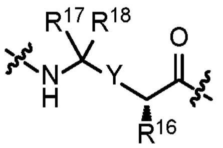, or
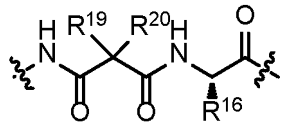,

wherein W is -NH-heterocycloalkylene- or heterocycloalkyl; Y is heteroarylene, arylene, $-C(O)C_{1-6}$ alkylene, $C_{2-6}$ alkenylene, $C_{1-6}$ alkylene, or $-C_{1-6}$ alkylene-NH-; each $R^{16}$ is independently selected from the group consisting of $C_{1-6}$ alkyl, $C_{2-6}$ alkenyl, $-(C_{1-6}$ alkylene)$NHC(NH)NH_2$, and $-(C_{1-6}$ alkylene)$NHC(O)NH_2$; $R^{17}$ and $R^{18}$ are each independently selected from the group consisting of hydrogen, $C_{1-6}$ alkyl, $C_{2-6}$ alkenyl, aryl, and heteroaryl, or $R^{17}$ and $R^{18}$ may form $C_{3-6}$ cycloalkyl together; $R^{19}$ and $R^{20}$ are each independently selected from the group consisting of $C_{1-6}$ alkyl, $C_{2-6}$ alkenyl, aryl, heteroaryl, and $(C_{1-6}$ alkyl)$OCH_2$-, or $R^{19}$ and $R^{20}$ may form a $C_{3-6}$ cycloalkyl ring together.

In some embodiments, in the antibody-drug conjugate (ADC), Str is selected from the group consisting of chemical moieties represented by the following formula:

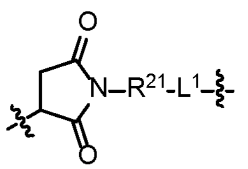
or
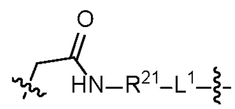,

wherein $R^{21}$ is selected from the group consisting of $-W_1-C(O)-$, $-C(O)-W_1-C(O)-$, $-(CH_2CH_2O)_{p1}C(O)-$, $-(CH_2CH_2O)_{p1}CH_2C(O)-$, and$-(CH_2CH_2O)_{p1}CH_2CH_2C(O)-$, wherein W1 is selected from the group consisting of $C_{1-6}$ alkylene, $C_{1-6}$ alkylene-cycloalkyl, and a linear heteroalkyl of 1 to 8 atoms; the heteroalkyl contains 1 to 3 heteroatoms selected from the group consisting of N, O, and S, wherein the alkyl, cycloalkyl, and linear heteroalkyl are each independently optionally substituted with one or more groups selected from the group consisting of halogen, deuterium, hydroxy, cyano, amino, $C_{1-6}$ alkyl, $C_{1-6}$ haloalkyl, deuterated $C_{1-6}$ alkyl, $C_{1-6}$ alkoxy, and $C_{3-6}$ cycloalkyl; each p1 is independently an integer from 1 to 20;

$L^1$ is selected from the group consisting of $-NR^{22}-(CH_2CH_2O)_{P2}CH_2CH_2C(O)-$, $-NR^{22}-(CH_2CH_2O)_{p2}CH_2C(O)-$,

$-S(CH_2)_{p2}C(O)-$, $-(CH_2)_{p2}C(O)-$, and a single bond, preferably from the group consisting of a single bond; p2 is an integer from 1 to 20; $R^{22}$ is selected from the group consisting of a hydrogen atom, $C_{1-6}$ alkyl, $C_{1-6}$ haloalkyl, and deuterated $C_{1-6}$ alkyl.

In some embodiments, in Str of the antibody-drug conjugate, $R^{21}$ is selected from the group consisting of $C_{1-6}$ alkylene C(O)-, $-(CH_2-CH_2O)_2C(O)-$, $-(CH_2-CH_2O)_2CH_2C(O)-$, $-(CH_2-CH_2O)_2CH_2CH_2C(O)-$, $-(CH_2-CH_2O)_3C(O)-$, and $-(CH_2-CH_2O)_4C(O)-$.

In some embodiments, the Pep is selected from the group consisting of valine-citrulline (Val-Cit), alanine-alanine-asparagine (Ala-Ala-Asn), glycine-glycine-lysine (Gly-Gly-lys), valine-lysine (Vallys), valine-alanine (Val-Ala), valine-phenylalanine (Val-Phe), and glycine-glycine-phenylalanineglycine (Gly-Gly-Phe-Gly).

In some embodiments, the Sp is selected from the group consisting of $-NHCH_2-$, $-NHCH_2CH_2-$, $-NHCH_2CH_2CH_2-$, $-NHCH_2-O-CH_2-$, $-NHCH_2CH_2-O-CH_2-$, $-NH(CH_2)_3-C(O)-$, $-NHCH_2-O-CH_2-C(O)-$, $-NH(CH_2)_2-O-CH_2-C(O)-$,

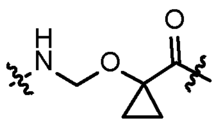,
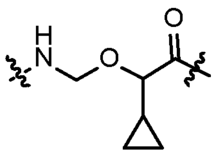,
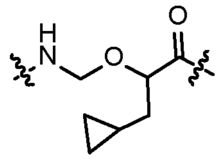,

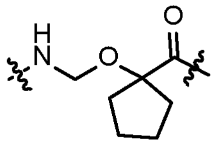,
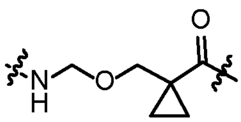,
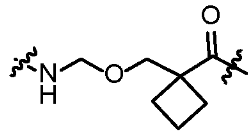, and
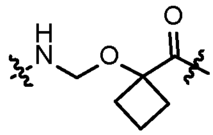

In some embodiments, in the antibody-drug conjugate, the linker L comprises: maleimide-$(PEG)_4$-$CH_2CH_2C(O)$-Gly-Gly-Phe-Gly, maleimide-$(PEG)_2$-Val-Cit, maleimide-$(PEG)_6$-Val-Cit, maleimide-$(PEG)_8$-Val-Cit, maleimide-$(PEG)_4$-$CH_2CH_2C(O)$-Val-lys, maleimide-$(CH_2)_5$-Val-Cit, maleimide-$(CH_2)_5$-Val-lys, maleimide-$(CH_2)_5$-Gly-Gly-Phe-Gly, maleimide-$(PEG)_4$-$CH_2CH_2C(O)$-Gly-Gly-Phe-Gly, maleimide-$(PEG)_2$-Ala-Ala-Asn, maleimide-$(PEG)_6$-Ala-Ala-Asn, maleimide-$(PEG)_8$-Ala-Ala-Asn, maleimide-$(PEG)_4$-triazole-$(PEG)_3$-sulfonamide, maleimide-$(PEG)_2$-$CH_2CH_2C(O)$-Val-lys, maleimide-$(PEG)_4$-triazole-$(PEG)_3$-sulfonamide, or Mal-$(PEG)_4$-triazole-$(PEG)_3$-disulfide.

In some other embodiments, the antibody-drug conjugate (ADC) of the present disclosure is represented by the following formula:

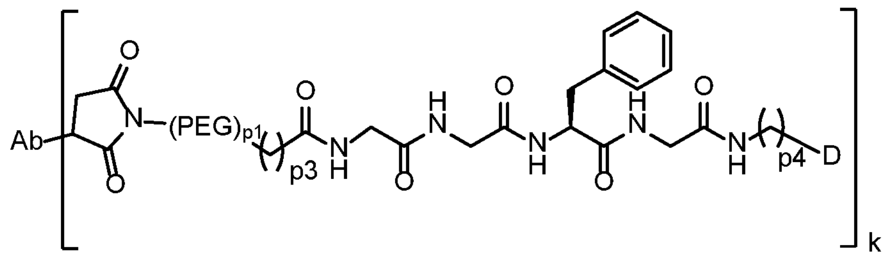,

wherein k is selected from the group consisting of 1 to 10 and may be an integer or a decimal; p1 is selected from the group consisting of 2, 4, 6, and 8; p3 and p4 are each independently selected from the group consisting of 0, 1, and 2;

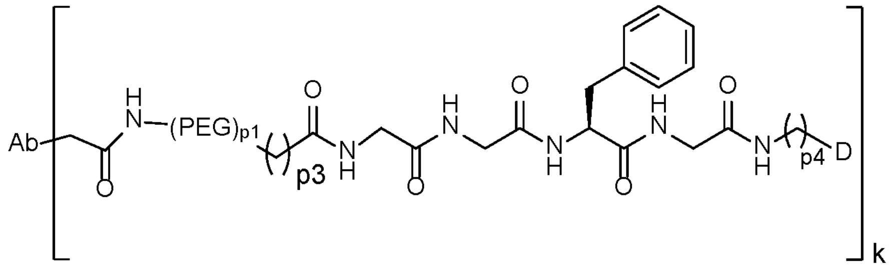

,

wherein k is selected from the group consisting of 1 to 10 and may be an integer or a decimal; p1 is selected from the group consisting of 2, 4, 6, and 8; p3 and p4 are each independently selected from the group consisting of 0, 1, and 2;

In some other embodiments, the antibody-drug conjugate (ADC) of the present disclosure is selected from the group consisting of:

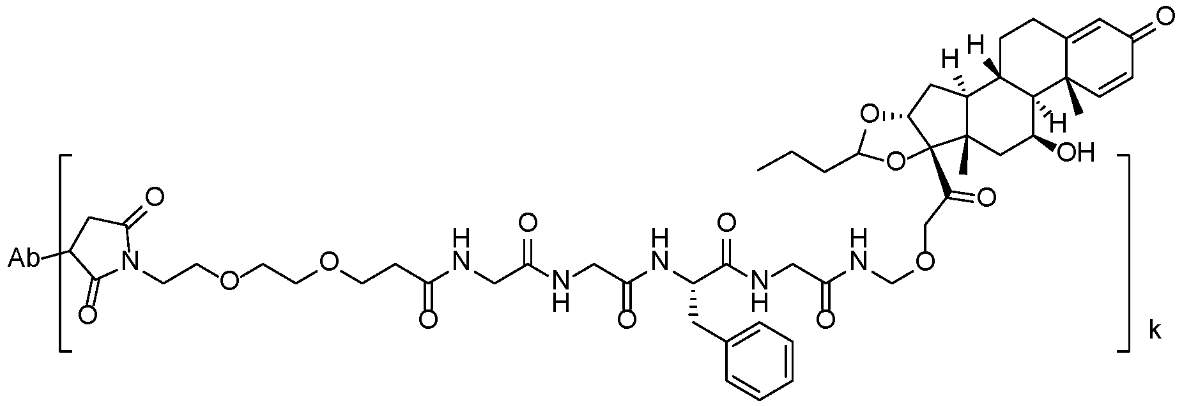

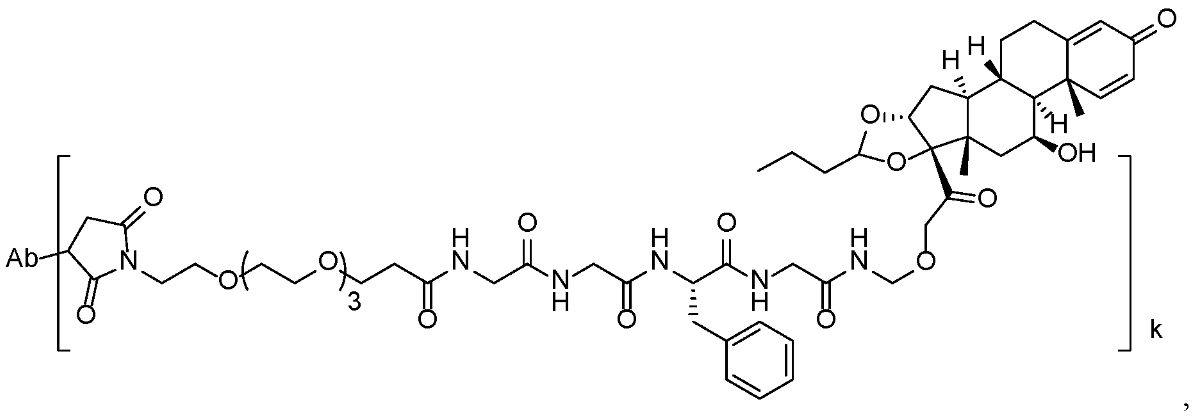

,

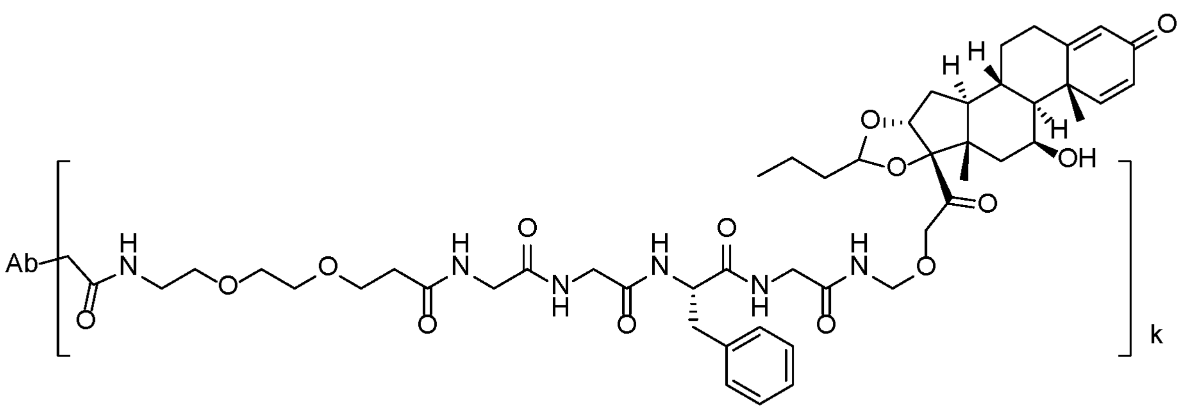

,

and

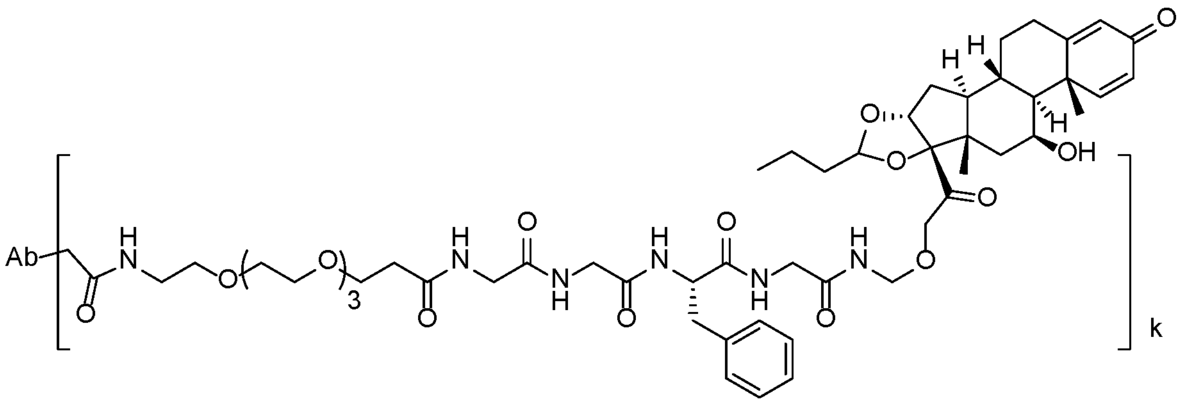

,

wherein k is selected from the group consisting of 1 to 10 and may be an integer or a decimal.

The present disclosure also provides a compound represented by formula II-A or a pharmaceutically acceptable salt thereof,

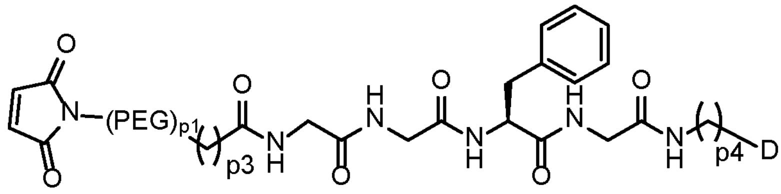

II-A

,

wherein p1 is selected from the group consisting of 2, 4, 6, and 8, and p3 and p4 are each independently selected from the group consisting of 0, 1, and 2.

The present disclosure also provides a compound represented by formula II-B or a pharmaceutically acceptable salt thereof,

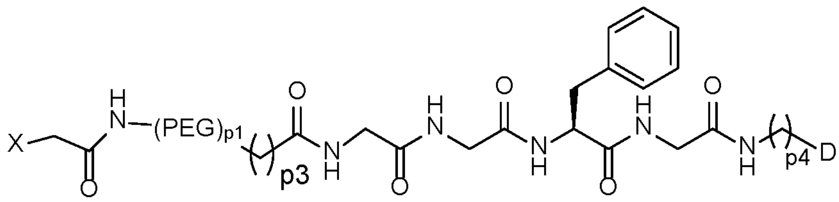

II-B

,

wherein X is halogen, p1 is selected from the group consisting of 2, 4, 6, and 8, and p3 and p4 are each independently selected from the group consisting of 0, 1, and 2.

In the structures of the present disclosure, $\overline{---}$ represents a single bond or a double bond.

The present disclosure also provides a pharmaceutical composition, comprising at least one of the antibody-drug conjugates described above, and a pharmaceutically acceptable carrier, diluent, or excipient.

In some embodiments, a unit dose of the pharmaceutical composition is 0.001 mg-1000 mg.

In some embodiments, the pharmaceutical composition comprises 0.01%-99.99% of the aforementioned compound based on the total weight of the composition. In some embodiments, the pharmaceutical composition comprises 0.1%-99.9% of the aforementioned compound. In some embodiments, the pharmaceutical composition comprises 0.5%-99.5% of the aforementioned compound. In some embodiments, the pharmaceutical composition comprises 1%-99% of the aforementioned compound. In some embodiments, the pharmaceutical composition comprises 2%-98% of the aforementioned compound.

In some embodiments, the pharmaceutical composition comprises 0.01%-99.99% of the pharmaceutically acceptable carrier, diluent, or excipient based on the total weight of the composition. In some embodiments, the pharmaceutical composition comprises 0.1%-99.9% of the pharmaceutically acceptable carrier, diluent, or excipient. In some embodiments, the pharmaceutical composition comprises 0.5%-99.5% of the pharmaceutically acceptable carrier, diluent, or excipient. In some embodiments, the pharmaceutical composition comprises 1%-99% of the pharmaceutically acceptable carrier, diluent, or excipient. In some embodiments, the pharmaceutical composition comprises 2%-98% of the pharmaceutically acceptable carrier, diluent, or excipient.
The antibody-drug conjugate of the present disclosure may be administered to a patient in a manner suitable for the indication, e.g., parenterally, topically, or by inhalation. If injected, an antagonist may be administered, e.g., via intraarticular, intravenous, intramuscular, intralesional, intraperitoneal, or subcutaneous routes, by bolus injection, or by continuous infusion. Localized administration at a site of disease or injury is considered to be percutaneous delivery and sustained release from implants. Delivery by inhalation includes, for example, nasal or oral inhalation, use of a nebulizer, inhalation of the antagonist in aerosol form, and the like. Other alternatives include eyedrops; oral formulations, including pills, syrups, lozenges, or chewing gum; and topical formulations, such as lotions, gels, sprays, and ointments.
The present disclosure also provides an inhalable pharmaceutical composition, comprising the antibody-drug conjugate represented by formula (I) and a pharmaceutically acceptable carrier,

$$Ab\text{-}(L\text{-}D)_k \qquad (I)$$

wherein Ab is an anti-IL-4R antibody or an antigen-binding fragment thereof;
L is a linker covalently linking Ab to D, and k is 1 to 20 (including 1, 2, 3, 4, 5, 6, 7, 8, 9, 10, 11, 12, 13, 14, 15, 16, 17, 18, 19, 20, or any value between any two values);
D is a glucocorticoid or a residue thereof.

Optional Ab, L, and D are as previously described.
In some embodiments, Ab is an anti-IL-4R Fab fragment.
The present disclosure also provides use of the antibody-drug conjugate and/or the pharmaceutical composition comprising the antibody-drug conjugate in the preparation of a medicament for treating or preventing an IL-4R-mediated disease or disorder.
The present disclosure also provides use of the antibody-drug conjugate and/or the pharmaceutical composition comprising the antibody-drug conjugate in the preparation of a medicament for treating or preventing an immune disease or disorder. The disease or disorder is selected from the group consisting of: asthma, nasal polyps, chronic sinusitis, allergic skin disorders, eosinophilic esophagitis, chronic obstructive pulmonary disease, allergic rhinitis, arthritis, inflammatory diseases, allergic reaction, autoimmune lymphoproliferative syndrome, autoimmune hemolytic anemia, Barrett's esophagus, autoimmune uveitis, tuberculosis, and renal disease, preferably from the group consisting of asthma and allergic skin disorders.
The present disclosure also provides a method for delivering a glucocorticoid to an IL-4R-expressing cell, comprising the step of contacting the IL-4R-expressing cell with the antibody-drug conjugate of the present disclosure.
The present disclosure further provides a kit, comprising the antibody-drug conjugate or the pharmaceutical composition of the present disclosure.

## Detailed Description of the Invention

Unless defined otherwise, all technical and scientific terms used herein have the same meaning as commonly understood by those of ordinary skill in the art to which the present disclosure belongs. Although any methods and materials similar or equivalent to those of the present disclosure can also be used to implement or test the present disclosure, preferred methods and materials are described herein. In describing and claiming the present disclosure, the following terms are used in accordance with the definitions below.
When a trade name is used in the present disclosure, the applicant intends to include the formulation of the commercial product under the trade name, and the non-patent drug and active drug component of the commercial product under the trade name.
Unless otherwise stated, the terms used in the specification and claims have the following meanings. The term "linker", "linker unit", or "linker fragment" refers to a chemical structural fragment or bond, which is linked to a ligand at one end and linked to a drug at the other end, and also may be linked to other linkers and then linked to the drug.
The linker may comprise one or more linker components. Exemplary linker components include 6-maleimidocaproyl (MC), maleimidopropionyl (MP), valine-citrulline (Val-Cit or vc), alanine-phenylalanine (ala-phe), p-aminobenzyloxycarbonyl (PAB), and those derived from coupling to a linker reagent: N-succinimidyl 4-(2-pyridylthio)pentanoate (SPP), N-succinimidyl 4-(N-maleimidomethyl)cyclohexane-1 carboxylate (SMCC, also referred to herein as MCC), and N-succinimidyl(4-iodo-acetyl)aminobenzoate (SLAB). The linker can include a stretcher unit, a spacer unit, an amino acid unit and an extension unit. The linker may be synthesized using methods known in the art, such as those described in US2005-0238649A1. A linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research 52: 127-131(1992); U.S. Patent No. 5,208,020).

The term "stretcher unit" refers to a chemical structural fragment that covalently links to an antibody through carbon atoms at one end and is linked to an amino acid unit, a disulfide moiety, a sulfonamide moiety or a non-peptide chemical moiety at the other end.

The term "spacer unit" is a bifunctional compound structural fragment that can be used to couple an amino acid unit to a glucocorticoid to form an antibody-drug conjugate, in such a way that the glucocorticoid is selectively linked to the amino acid unit.

The term "amino acid" refers to an organic compound that contains amino and carboxyl in the molecular structure and in which both amino and carboxyl are directly linked to a -CH- structure. The general formula is H2NCHRCOOH, where R is H, substituted or unsubstituted alkyl, or the like. Amino acids are classified as α, β, γ, δ, ε...-amino acids according to the position of the carbon atom to which the amino is linked in the carboxylic acid. In the biological field, the amino acids that constitute native proteins have their specific structural characteristics; that is, their amino groups are attached directly to the α-carbon atoms, namely α-amino acids, including Gly (Glycine), Ala (Alanine), Val (Valine), Leu (Leucine), Ile (Isoleucine), Phe (Phenylalanine), Trp (Tryptophan), Tyr (Tyrosine), Asp (Aspartic acid), His (Histidine), Asn (Asparagine), Glu (Glutamic acid), Lys (Lysine), Gln (Glutamine), Met (Methionine), Arg (Arginine), Ser (Serine), Thr (Threonine), Cys (Cysteine), Pro (Proline), etc. Non-natural amino acids are, for example, citrulline. As is well known to those skilled in the art, non-natural amino acids do not constitute natural proteins and are therefore not involved in the synthesis of antibodies in the present disclosure. The three-letter and single-letter codes for amino acids used in the present disclosure are as described in J. biol. chem, 243, p3558 (1968).

| For short | Abbreviation | Name | Structure |
|---|---|---|---|
| G | Gly | Glycine | 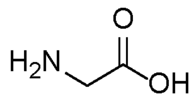 |
| A | Ala | Alanine | 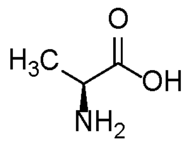 |
| V | Val | Valine | 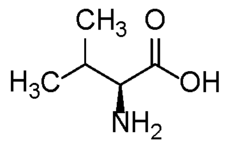 |
| L | Leu | Leucine | 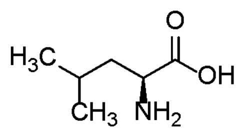 |
| I | Ile | Isoleucine | 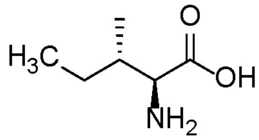 |
| F | Phe | Phenylalanine | 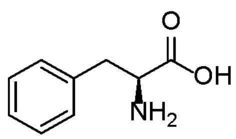 |

(continued)

| For short | Abbreviation | Name | Structure |
|---|---|---|---|
| W | Trp | Tryptophan | 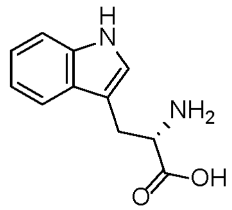 |
| Y | Tyr | Tyrosine | 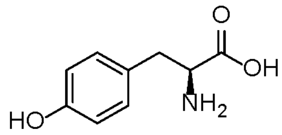 |
| D | Asp | Aspartic acid | 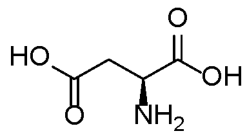 |
| H | His | Histidine | 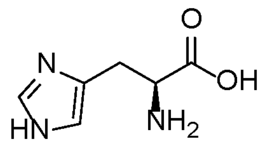 |
| N | Asn | Asparagine | 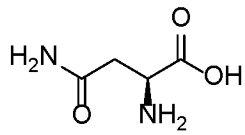 |
| E | Glu | Glutamic acid | 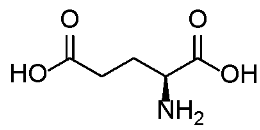 |
| K | Lys | Lysine | 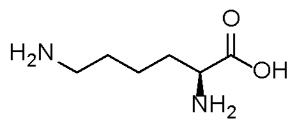 |
| Q | Gln | Glutamine | 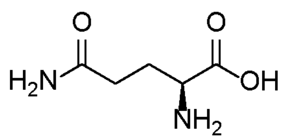 |
| C | Cit | Citrulline | 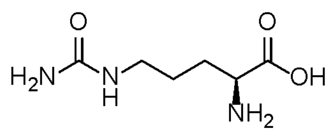 |

The term "antibody-drug conjugate" means that a ligand is linked to a biologically active drug by a stable linker unit. In the present disclosure, "antibody-drug conjugate" (ADC) means that a monoclonal antibody or an antibody fragment is linked to a biologically active glucocorticoid by a stable linker unit, wherein the antibody or the antibody fragment, through a specific group therein (such as an interchain disulfide bond), can bind to the glucocorticoid molecule comprising a linker. The term "drug loading" refers to the average number of drugs that each antibody-drug conjugate molecule carries in a population of antibody-drug conjugates, and can also be expressed as a ratio of the number of drugs to the number of antibodies. The drug loading may range from 1 to 20, preferably from 1 to 10 glucocorticoids (D) linked to

each antibody (Ab). In embodiments of the present disclosure, the drug loading is represented as k, and may illustratively be 1, 2, 3, 4, 5, 6, 7, 8, 9, 10, 11, 12, 13, 14, 15, 16, 17, 18, 19, 20, or an average of any two values; preferably an average of 1 to 10, and more preferably an average of 1 to 8, or 2 to 8, or 2 to 7, or 3 to 8, or 3 to 7, or 3 to 6, or 4 to 7, or 4 to 6, or 4 to 5. The average number of drugs per ADC molecule after coupling reactions can be characterized by conventional methods such as UV/visible spectroscopy, mass spectrometry, ELISA assays, mAb molecule size variant assay (CE-SDS), and HPLC.

The mAb molecular size variant assay (CE-SDS) of the present disclosure may be used for quantitatively determining the purity of a recombinant monoclonal antibody product by adopting capillary electrophoresis-sodium dodecyl sulfate (CE-SDS) ultraviolet assay based on the molecular weight under reduced and non-reduced conditions and according to a capillary electrophoresis method (Chinese Pharmacopoeia 0542, 2015 Edition).

In one embodiment of the present disclosure, the glucocorticoid is coupled to the N-terminal amino of the ligand and/or ε-amino of the lysine residue through a linker unit, and generally, the number of drug molecules that can be coupled to the antibody in the coupling reaction will be less than the theoretical maximum.

The loading of the antibody-drug conjugate can be controlled by the following non-limiting methods, including:

(1) controlling a molar ratio of a linking reagent to a mAb,
(2) controlling reaction time and temperature, and
(3) selecting different reaction reagents.

The term "antibody" refers to an immunoglobulin, which is of a tetrapeptide chain structure formed by connecting two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, otherwise called isotypes of immunoglobulins, namely IgM, IgD, IgG, IgA and IgE, with their corresponding heavy chains being μ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain.

In the heavy and light chains of the antibody, the sequences of about 110 amino acids near the N-terminus vary considerably and thus are referred to as variable regions (Fv regions); the remaining amino acid sequences near the C-terminus are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs arranged from the amino-terminus to the carboxyl-terminus in the following order: FR1, CDR1, FR2, CDR2, FR3, CDR3, and FR4. The 3 CDRs of the light chain refer to LCDR1, LCDR2, and LCDR3, and the 3 CDRs of the heavy chain refer to HCDR1, HCDR2, and HCDR3.

The antibody of the present disclosure includes a murine antibody, a chimeric antibody, a humanized antibody, and a fully human-derived antibody, preferably a humanized antibody and a fully human-derived antibody.

The term "murine antibody" in the present disclosure refers to an antibody prepared from mice according to the knowledge and skill in the art. During the preparation, a test subject is injected with a specific antigen, and then hybridomas expressing antibodies with desired sequences or functional properties are isolated.

The term "chimeric antibody" refers to an antibody obtained by fusing a variable region of a murine antibody to a constant region of a human antibody, which can reduce an immune response induced by the murine antibody. The chimeric antibody is established by firstly establishing a hybridoma secreting a murine specific mAb, then cloning a variable region gene from the mouse hybridoma cells, cloning a human antibody constant region gene as required, connecting the mouse variable region gene and the human constant region gene into a chimeric gene, inserting the chimeric gene into an expression vector, and finally expressing chimeric antibody molecules in a eukaryotic system or prokaryotic system.

The term "humanized antibody", also known as a CDR-grafted antibody, refers to an antibody produced by grafting murine CDR sequences into a human antibody variable region framework, i.e., a different type of human germline antibody framework sequence. Such an antibody can overcome the heterogeneous reaction induced by the chimeric antibody because of carrying a large amount of mouse protein components. Such framework sequences can be obtained from public DNA databases or published references that include germline antibody gene sequences. For example, germline DNA sequences of genes of the human heavy and light chain variable regions can be found in the "VBase" human germline sequence database (available at the Internet address www.mrccpe.com.ac.uk/vbase), as well as in Kabat, E. A. et al., 1991 Sequences of Proteins of Immunological Interest, 5th edition. To avoid the decrease in activity caused by the decrease in immunogenicity, the framework sequence in the human antibody variable region can be subjected to minimum reverse mutation or back mutation to maintain activity. The humanized antibody of the present disclosure also includes humanized antibodies formed by further affinity maturation of the CDRs by phage display. The literature further describing

methods used in humanization of accessible mouse antibodies includes, for example, Queen et al., Proc. Natl. Acad. Sci. USA, 88, 2869, 1991, and literature describing humanization using the method provided by Winter and coworkers thereof includes Jones et al., Nature, 321, 522 (1986); Riechmann et al., Nature, 332, 323-327 (1988), Verhoeyen et al, Science, 239, 1534 (1988).

The term "fully human-derived antibody", "fully human antibody" or "completely human-derived antibody", also known as "fully human-derived monoclonal antibody", may have both humanized variable regions and constant regions, thus eliminating immunogenicity and toxic and side effects. The development of monoclonal antibodies has four stages, namely murine monoclonal antibodies, chimeric monoclonal antibodies, humanized monoclonal antibodies, and fully human-derived monoclonal antibodies. The antibody of the present disclosure is the fully human-derived monoclonal antibody. Major relevant technologies for the preparation of the fully human antibody include human hybridoma technology, EBV-transformed B-lymphocyte technology, phage display technology, transgenic mouse antibody preparation technology, single B-cell antibody preparation technology, and the like.

The term "antigen-binding fragment" refers to one or more fragments of an antibody that retain the ability to specifically bind to an antigen. It is shown that a fragment of a full-length antibody can be used to perform the antigen-binding function of the antibody. The examples of the binding fragment included in the "antigen-binding fragment" include (i) Fab fragments, monovalent fragments consisting of VL, VH, CL and CH1 domains; (ii) $F(ab')_2$ fragments, bivalent fragments comprising two Fab fragments connected by disulfide bridges in the hinge regions; (iii) Fd fragments consisting of VH and CH1 domains; (iv) Fv fragments consisting of VH and VL domains of a single arm of an antibody; (v) single domains or dAb fragments (Ward et al., (1989) Nature 341: 544-546) consisting of VH domains; and (vi) isolated complementarity determining regions (CDRs) or (vii) combinations of two or more isolated CDRs which may optionally be linked by synthetic linkers. In addition, although the two domains of the Fv fragment, VL and VH, are encoded by separate genes, they can be linked by a synthetic linker by a recombination method, thus producing a single protein chain in which the VL and VH regions pair to form a monovalent molecule (referred to as single-chain Fv (scFv); see, e.g., Bird et al. (1988) Science 242: 423-426; and Huston et al. (1988) Proc. Natl. Acad. Sci. USA 85: 5879-5883). Such single-chain antibodies are also intended to be included in the term "antigen-binding fragment" of an antibody. Such antibody fragments are obtained by conventional techniques known to those skilled in the art, and screened for utility in the same manner as for intact antibodies. Antigen-binding moieties may be produced by a recombinant DNA technique or by enzyme catalysis or chemical cleavage of intact immunoglobulins. The antibody may be of different isotypes, e.g., an IgG (e.g., subtype IgG1, IgG2, IgG3 or IgG4), IgA1, IgA2, IgD, IgE or IgM antibody.

Fab is an antibody fragment having a molecular weight of about 50,000 and having antigen-binding activity, among fragments obtained by treating an IgG antibody molecule with a protease papain (cleaving the amino acid residue at position 224 of H chain), in which about half of the N-terminal side of the H chain and the entire L chain are joined together by disulfide bonds.

F(ab')2 is an antibody fragment having a molecular weight of about 100,000 and having antigen-binding activity and comprising two Fab regions linked at the hinge position, which is obtained by digesting a portion below two disulfide bonds in the IgG hinge region with the enzyme pepsin.

Fab' is an antibody fragment having a molecular weight of about 50,000 and having an antigen-binding activity, which is obtained by cleaving the disulfide bond in the hinge region of the F(ab')2 described above.

In addition, the Fab' can be produced by inserting DNA encoding the Fab' fragment of the antibody into a prokaryotic expression vector or a eukaryotic expression vector and introducing the vector into a prokaryote or a eukaryote to express the Fab'.

The term "single-chain antibody", "single-chain Fv" or "scFv" refers to a molecule comprising an antibody heavy chain variable domain (or region; VH) and an antibody light chain variable domain (or region; VL) linked by a linker. Such scFv molecules may have a general structure: $NH_2$-VL-linker-VH-COOH or $NH_2$-VH-linker-VL-COOH. Suitable linkers in the prior art consist of repeated GGGGS amino acid sequences or variants thereof, for example, 1-4 repeated variants (Holliger et al.

(1993), Proc. Natl. Acad. Sci. USA 90: 6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061; Kipriyanov et al. (1999), J. Mol. Biol. 293:41-56; and Roovers et al. (2001), Cancer Immunol.

The term "CDR" refers to one of the 6 hypervariable regions within the variable domain of an antibody which primarily contribute to antigen binding. One of the most common definitions for the 6 CDRs is provided in Kabat E.A. et al., (1991) Sequences of proteins of immunological interest. NIH Publication 91-3242. As used herein, the Kabat definition of CDRs may be only applied to CDR1, CDR2, and CDR3 of the light chain variable domain (CDR L1, CDR L2, CDR L3 or L1, L2, L3), and CDR2 and CDR3 of the heavy chain variable domain (CDR H2, CDR H3 or H2, H3). Generally, there are three CDRs (HCDR1, HCDR2, and HCDR3) in each heavy chain variable region and three CDRs (LCDR1, LCDR2, and LCDR3) in each light chain variable region. The amino acid sequence boundaries of the CDRs can be determined using any one of a variety of well-known schemes, including "Kabat" numbering scheme (see Kabat et al. (1991), "Sequences of Proteins of Immunological Interest", 5th edition, Public Health Service, National Institutes of Health, Bethesda, MD), "Chothia"

numbering scheme (see Al-Lazikani et al. (1997), JMB 273: 927-948) and ImMunoGenTics (IMGT) numbering scheme (see Lefranc M.P., Immunologist, 7, 132-136(1999); Lefranc, M.P. et al., Dev. Comp. Immunol., 27, 55-77(2003)), and the like. For example, for the classical format, according to the Kabat scheme, the CDR amino acid residues in the heavy chain variable domain (VH) are numbered 31-35 (HCDR1), 50-65 (HCDR2), and 95-102 (HCDR3); the CDR amino acid residues in the light chain variable domain (VL) are numbered 24-34 (LCDR1), 50-56 (LCDR2), and 89-97 (LCDR3). According to the Chothia scheme, the CDR amino acids in VH are numbered 26-32 (HCDR1), 52-56 (HCDR2), and 95-102 (HCDR3); and the amino acid residues in VL are numbered 26-32 (LCDR1), 50-52 (LCDR2), and 91-96 (LCDR3). According to the combination of CDR definitions provided in the Kabat scheme and the Chothia scheme, the CDR is composed of amino acid residues 26-35 (HCDR1), 50-65 (HCDR2), and 95-102 (HCDR3) in the human VH and amino acid residues 24-34 (LCDR1), 50-56 (LCDR2), and 89-97 (LCDR3) in the human VL. According to the IMGT scheme, the CDR amino acid residues in VH are roughly numbered 26-35 (CDR1), 51-57 (CDR2) and 93-102 (CDR3), and the CDR amino acid residues in VL are roughly numbered 27-32 (CDR1), 50-52 (CDR2) and 89-97 (CDR3). According to the IMGT scheme, the CDRs of the antibody can be determined using the program IMGT/DomainGap Align. The term "antibody framework" refers to a portion of a variable domain VL or VH, which serves as a framework for the antigen-binding loops (CDRs) of the variable domain. It is essentially a variable domain without CDRs.

"Bind to IL-4R" refers to the ability to interact with human IL-4R (or epitopes or fragments thereof). The term "antigen-binding site" herein refers to a three-dimensional spatial site recognized by an antibody or an antigen-binding fragment described herein.

The term "epitope" or "antigenic determinant" refers to a site on an antigen to which an immunoglobulin or antibody specifically binds. Epitopes generally comprise at least 3, 4, 5, 6, 7, 8, 9, 10, 11, 12, 13, 14, or 15 contiguous or non-contiguous amino acids in a unique spatial conformation (see, e.g., Epitope Mapping Protocols in Methods in Molecular Biology, Vol. 66, G.E.Morris, Ed. (1996)).

The terms "specific binding", "selective binding", "selectively bind to" and "specifically bind to" refer to the binding of an antibody to an epitope on a predetermined antigen. Generally, the antibody binds with an affinity (KD) of less than about $10^{-7}$ M, e.g., less than about $10^{-8}$ M, $10^{-9}$ M, or $10^{-10}$ M or less.

The term "nucleic acid molecule" refers to a DNA molecule and an RNA molecule. The nucleic acid molecule may be single-stranded or double-stranded, and is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

The term "vector" refers to a nucleic acid molecule capable of transporting another nucleic acid to which it has been linked. In one embodiment, the vector is a "plasmid" that refers to a circular double-stranded DNA loop into which additional DNA segments may be ligated. In another embodiment, the vector is a viral vector, wherein additional DNA segments may be ligated into the viral genome. The vectors disclosed herein are capable of autonomously replicating in a host cell into which they are introduced (e.g., bacterial vectors having a bacterial origin of replication and episomal mammalian vectors) or capable of integrating into the genome of a host cell after being introduced into the host cell and thus replicating with the host genome (e.g., non-episomal mammalian vectors).

Methods for producing and purifying antibodies and antigen-binding fragments are well known in the prior art, for example, those described in chapters 5-8 and 15 of "Antibodies: A Laboratory Manual", Cold Spring Harbor Press. Likewise, antigen-binding fragments can be prepared by conventional methods. The antibody or antigen-binding fragment described in the present invention is genetically engineered to contain one or more additional human-derived FRs in the non-human-derived CDRs. Human FR germline sequences can be obtained at the website http://imgt.cines.fr of ImMunoGeneTics (IMGT) or from the immunoglobulin journal, 2001ISBN012441351, by comparing the IMGT human antibody variable region germline gene database with the MOE software.

The term "host cell" refers to a cell into which an expression vector has been introduced. Host cells may include bacterial, microbial, plant, or animal cells. Bacteria susceptible to transformation include members of the *Enterobacteriaceae* family, such as strains of *Escherichia coli* or *Salmonella*; members of the *Bacillaceae* family, such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese hamster ovary cell line) and NS0 cells. The engineered antibody or antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems will result in glycosylation of antibodies, particularly at the highly conserved N-terminal site of the Fc region. Positive clones are expanded in a serum-free medium of a bioreactor to produce antibodies. The culture medium with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods,

such as molecular sieves and ion exchange. The resulting product needs to be immediately frozen, e.g., at -70 °C, or lyophilized.

The amino acid sequence "identity" refers to the percentage of amino acid residues shared by a first sequence and a second sequence, wherein in aligning the amino acid sequences and when necessary, gaps are introduced to achieve maximum percent sequence identity, and any conservative substitution is not considered as part of the sequence identity. For the purpose of determining percent amino acid sequence identity, alignments can be achieved in a variety of ways that are within the scope of skills in the art, for example, using publicly available computer software such as BLAST, BLAST-2, ALIGN, ALIGN-2 or Megalign (DNASTAR) software. Those skilled in the art can determine parameters suitable for measuring alignment, including any algorithms required to achieve maximum alignment of the full length of the aligned sequences.

The term "anti-IL-4R antibody" or "antibody that binds to IL-4R" refers to an antibody that is capable of binding to IL-4R, e.g., with sufficient affinity such that the antibody can be used as a therapeutic agent targeting IL-4R. The extent of the binding of the anti-IL-4R antibody to an unrelated non-IL-4R protein may be less than about 10% of the binding of the antibody to IL-4R as measured, e.g., by a radioimmunoassay (RIA). In some embodiments, the antibody that binds to IL-4R has a dissociation constant (Kd) of ≤1 μM, ≤100 nM, ≤10 nM, ≤1 nM, or ≤0.1 nM.

The term "peptide" refers to a compound fragment between an amino acid and a protein. The compound fragment is formed by connecting 2 or more amino acid molecules by peptide bonds, and is a structural and functional fragment of the protein, such as hormones and enzymes, which are essentially peptides.

The term "sugar" refers to biomacromolecules consisting of C, H, and O elements. They can be classified into monosaccharides, disaccharides, polysaccharides, and the like.

The term "fluorescent probe" refers to a class of fluorescent molecules that have characteristic fluorescence in the ultraviolet-visible-near infrared region and whose fluorescent properties (excitation and emission wavelengths, intensity, lifetime, polarization, etc.) can sensitively change as the properties of the environment they are in, such as polarity, refractive index, and viscosity, change. These fluorescent molecules non-covalently interact with nucleic acids (DNA or RNA), proteins or other macromolecular structures to change one or more fluorescent properties, and therefore can be used to study the properties and behavior of macromolecular substances.

The term "alkyl" refers to a saturated aliphatic hydrocarbon group which is a linear or branched group containing 1 to 20 carbon atoms, preferably alkyl containing 1 to 12 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. More preferred is an alkyl group containing 1 to 6 carbon atoms; non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted, and when it is substituted, the substituent may be substituted at any accessible point of attachment, and the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and a carboxylate group.

The term "heteroalkyl" refers to alkyl containing one or more heteroatoms selected from the group consisting of N, O, and S, wherein the alkyl is as defined above.

The term "cycloalkyl" refers to a saturated or partially unsaturated monocyclic or polycyclic hydrocarbon substituent; the cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spirocycloalkyl, fused cycloalkyl, and bridged cycloalkyl. "Carbocycle" refers to the ring system in cycloalkyl.

The term "spirocycloalkyl" refers to a 5- to 20-membered polycyclic group in which one carbon atom (referred to as a spiro atom) is shared between monocyclic rings; it may contain one or more double bonds, but none of the rings has a fully conjugated π-electron system. It is preferably 6- to 14-membered, and is more preferably 7- to 10-membered. According to the number of spiro atoms shared between rings, spirocycloalkyl may be monospirocycloalkyl, bispirocycloalkyl, or polyspirocycloalkyl, preferably monospirocycloalkyl and bispirocycloalkyl, and more preferably 4-membered/4-membered, 4-membered/5-membered, 4-membered/6-membered, 5-membered/5-membered, or 5-membered/6-membered monospirocycloalkyl. "Spirocarbocycle" refers to the ring system in spirocycloalkyl. Non-limiting examples of spirocycloalkyl include:

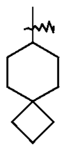 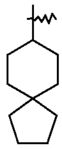 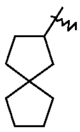 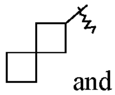 and 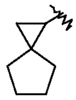

The term "fused cycloalkyl" refers to a 5- to 20-membered all-carbon polycyclic group in which each ring in the system shares a pair of adjacent carbon atoms with other rings in the system, wherein one or more rings may contain one or more double bonds, but none of the rings has a fully conjugated $\pi$-electron system. It is preferably 6- to 14-membered, and is more preferably 7- to 10-membered. According to the number of constituent rings, it may be bicyclic, tricyclic, tetracyclic, or polycyclic fused cycloalkyl, preferably bicyclic or tricyclic, and more preferably 5-membered/5-membered or 5-membered/6-membered bicycloalkyl. "Fused carbocycle" refers to the ring system in fused cycloalkyl. Non-limiting examples of fused cycloalkyl include:

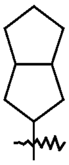 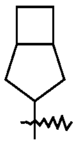 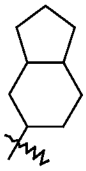 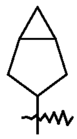 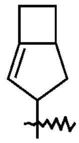 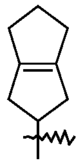 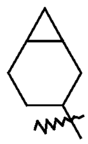 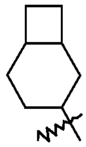 and 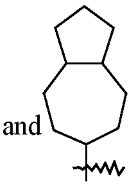

The term "bridged cycloalkyl" refers to a 5- to 20-membered all-carbon polycyclic group in which any two rings share two carbon atoms that are not directly connected; it may contain one or more double bonds, but none of the rings has a fully conjugated $\pi$-electron system. It is preferably 6- to 14-membered, and is more preferably 7- to 10-membered. According to the number of constituent rings, it may be bicyclic, tricyclic, tetracyclic, or polycyclic bridged cycloalkyl, preferably bicyclic, tricyclic, or tetracyclic, and more preferably bicyclic or tricyclic. Non-limiting examples of bridged cycloalkyl include:

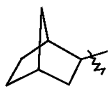 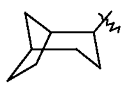 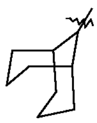 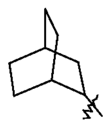 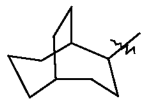

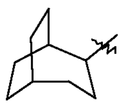 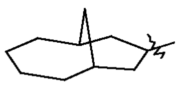 and 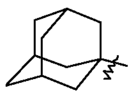

The cycloalkyl ring may be fused to an aryl, heteroaryl, or heterocycloalkyl ring, wherein the ring attached to the parent structure is cycloalkyl; non-limiting examples include indanyl, tetrahydronaphthyl, benzocycloheptyl, and the like. Cycloalkyl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and a carboxylate group.

The term "heterocyclyl" refers to a saturated or partially unsaturated, monocyclic or polycyclic hydrocarbon substituent, which contains 3 to 20 ring atoms, one or more of which are heteroatoms selected from the group consisting of nitrogen, oxygen, and $S(O)_m$ (where m is an integer from 0 to 2), but does not contain a ring moiety of -O-O-, -O-S-, or -S-S-, and the other ring atoms are carbon atoms. It preferably contains 3 to 12 ring atoms, 1 to 4 of which are heteroatoms; more preferably, it contains 3 to 6 ring atoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, and the like, preferably piperidinyl and pyrrolidinyl. Polycyclic heterocyclyl includes spiroheterocyclyl, fused heterocyclyl, and bridged heterocyclyl. "Heterocycle" refers to the ring system in heterocyclyl.

The term "spiroheterocyclyl" refers to a 5- to 20-membered polycyclic heterocyclyl group in which one atom (referred to as a spiro atom) is shared between monocyclic rings, wherein one or more ring atoms are heteroatoms selected from the group consisting of nitrogen, oxygen, and $S(O)_m$ (where m is an integer from 0 to 2), and the other ring atoms are carbon atoms. It may contain one or more double bonds, but none of the rings has a fully conjugated $\pi$-electron system. It is preferably 6- to 14-membered, and is more preferably 7- to 10-membered. According to the number of spiro atoms shared between rings, spiroheterocyclyl may be monospiroheterocyclyl, bispiroheterocyclyl, or polyspiroheterocyclyl, preferably, monospiroheterocyclyl and bispiroheterocyclyl, more preferably 4-membered/4-membered, 4-membered/5-membered, 4-membered/6-membered, 5-membered/5-membered, or 5-membered/6-membered monospiroheterocyclyl. "Spiroheterocycle" refers to the ring system in spiroheterocyclyl. Non-limiting examples of spiroheterocyclyl include:

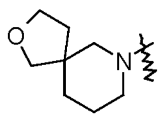 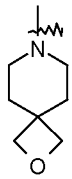 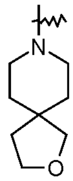 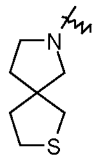 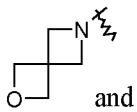 and 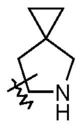

The term "fused heterocyclyl" refers to a 5- to 20-membered polycyclic heterocyclyl group in which each ring in the system shares a pair of adjacent atoms with the other rings in the system, wherein one or more rings may contain one or more double bonds, but none of the rings has a fully conjugated $\pi$-electron system; one or more ring atoms are heteroatoms selected from the group consisting of nitrogen, oxygen, and $S(O)_m$ (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic fused heterocyclyl, preferably bicyclic or tricyclic, and more preferably 5-membered/5-membered or 5-membered/6-membered bicyclic fused heterocyclyl. "Fused heterocycle" refers to the ring system in fused heterocyclyl. Non-limiting examples of fused heterocyclyl include:

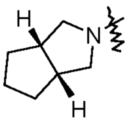 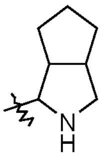 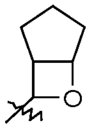 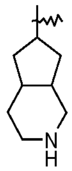 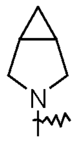 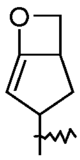 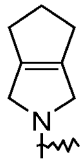 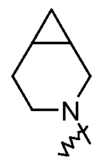

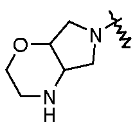, 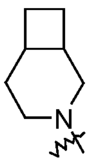, 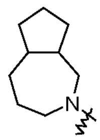, 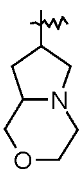, 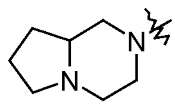, and 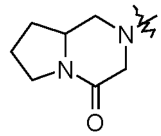

The term "bridged heterocyclyl" refers to a 5- to 14-membered polycyclic heterocyclyl group in which any two rings share two atoms that are not directly connected, which may contain one or more double bonds, but none of the rings has a fully conjugated $\pi$-electron system, wherein one or more ring atoms are heteroatoms selected from the group consisting of nitrogen, oxygen, and $S(O)_m$ (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic bridged heterocyclyl, preferably bicyclic, tricyclic, or tetracyclic, and more preferably bicyclic or tricyclic. Non-limiting examples of bridged heterocyclyl include:

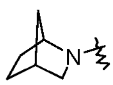 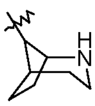 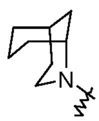 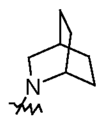

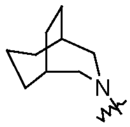 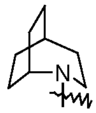 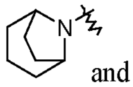 and 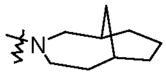

The heterocyclyl ring may be fused to an aryl, heteroaryl, or cycloalkyl ring, wherein the ring attached to the parent structure is heterocyclyl; its non-limiting examples include:

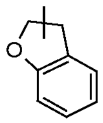, 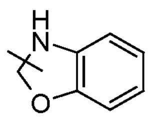, 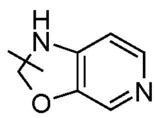, 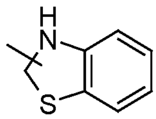,

and the like.

Heterocyclyl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and a carboxylate group.

The term "aryl" refers to a 6- to 14-membered, preferably 6- to 10-membered, all-carbon monocyclic or fused polycyclic (i.e., rings that share a pair of adjacent carbon atoms) group having a conjugated $\pi$-electron system, such as phenyl and naphthyl. The aryl ring may be fused to a heteroaryl, heterocyclyl, or cycloalkyl ring, wherein the ring attached to the parent structure is the aryl ring. "Aromatic ring" refers to the ring system in aryl. Non-limiting examples of aryl include:

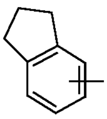, 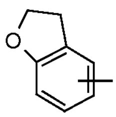, 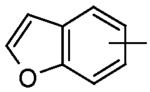, 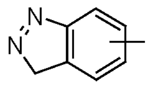, 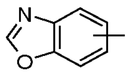, 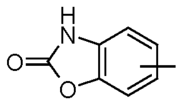, 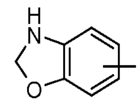,

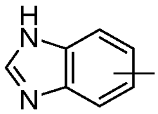, 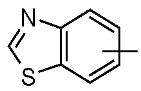, 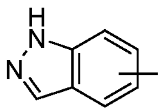, 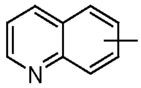, 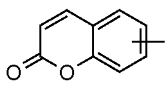, and 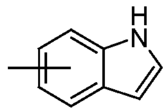

Aryl may be substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and a carboxylate group, preferably phenyl.

The term "fused-ring aryl" may be an unsaturated aromatic fused-ring structure containing 8-14 ring atoms, preferably 8-12 ring atoms, formed by connecting two or more ring structures that share two adjacent atoms with each other, including, for example, all unsaturated fused-ring aryl groups such as naphthalene and phenanthrene, and partially saturated fused-ring aryl groups such as benzo 3-8 membered saturated monocyclic cycloalkyl and benzo 3-8 membered partially saturated monocyclic cycloalkyl. "Fused aromatic ring" refers to the ring system in fused-ring aryl. Specific examples of fused-ring aryl include 2,3-dihydro-1H-indenyl, IH-indenyl, 1,2,3,4-tetrahydronaphthyl, 1,4-dihydronaphthyl, and the like.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Heteroaryl is preferably 5- to 12-membered, e.g., imidazolyl, furyl, thienyl, thiazolyl, pyrazolyl, oxazolyl, pyrrolyl, tetrazolyl, pyridyl, pyrimidinyl, thiadiazole, pyrazinyl, and the like, preferably imidazolyl, pyrazolyl, pyrimidinyl, or thiazolyl, and more preferably pyrazolyl or thiazolyl. The heteroaryl ring may be fused to an aryl, heterocyclyl, or cycloalkyl ring, wherein the ring attached to the parent structure is the heteroaryl ring. "Heteroaromatic ring" refers to the ring system in heteroaryl. Non-limiting examples of heteroaryl include:

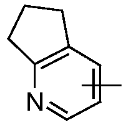 , 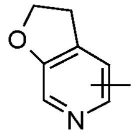 , 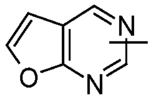 , 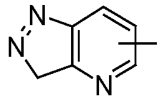 , 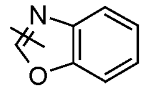 , 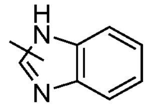 ,

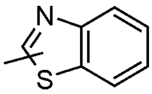 , 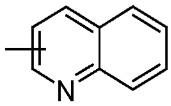 , 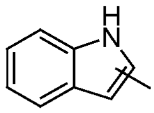 , and 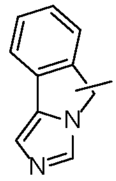

Heteroaryl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and a carboxylate group.

The term "fused heteroaryl" may be an unsaturated aromatic fused-ring structure containing 5-14 ring atoms (at least one heteroatom) formed by connecting two or more ring structures that share two adjacent atoms with each other, including the case where a carbon atom, a nitrogen atom, and a sulfur atom may be oxidized, preferably "5-12 membered fused heteroaryl", "7-12 membered fused heteroaryl", "9-12 membered fused heteroaryl", and the like, for example, benzofuranyl, benzoisothiafuranyl, benzothienyl, indolyl, isoindolyl, benzoxazolyl, benzimidazolyl, indazolyl, benzotriazolyl, quinolyl, 2-quinolinone, 4-quinolinone, 1-isoquinolinone, isoquinolinyl, acridinyl, phenanthridinyl, benzopyridazinyl, phthalazinyl, quinazolinyl, quinoxalinyl, phenazinyl, pteridinyl, purinyl, naphthyridinyl, phenazine, phenothiazine, and the like. "Fused heteroaromatic ring" refers to the ring system in fused heteroaryl.

Fused heteroaryl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and a carboxylate group.

The term "alkylene ($-CH_2-$)" refers to the remainder of an alkane molecule after removal of 2 hydrogen atoms, and includes linear and branched ylene groups of 1 to 20 carbon atoms. Non-limiting examples of alkylene containing 1 to 6 carbon atoms include methylene ($-CH_2-$), ethylene (e.g., $-CH_2CH_2-$ or $-CH(CH_3)-$), propylene (e.g, $-CH_2CH_2CH_2-$ or $-CH(CH_2CH_3)-$), and butylene (e.g., $-CH_2CH_2CH_2CH_2-$). Alkylene may be substituted or unsubstituted, and when it is substituted, the substituent may be substituted at any accessible point of attachment, preferably one or more of the following groups, independently selected from the group consisting of halogen, deuterium, hydroxy, nitro, cyano, and amino. Similarly, "alkenylene", "heteroarylene", "arylene", "heterocycloalkylene", and the like are as defined above.

The term "alkenylene" refers to a linear alkenyl group having 2 to 8 carbon atoms, preferably 2 to 6 carbon atoms, and more preferably 2 to 4 carbon atoms, and having at least one double bond at any position, including, for example, ethenylene, allylene, propenylene, butenylene, prenylene, butadienylene, pentenylene, pentadienylene, hexenylene, hexadienylene, and the like.

The term "alkynylene" refers to a linear alkynylene group having 2 to 8 carbon atoms, preferably 2 to 6 carbon atoms, and more preferably 2 to 4 carbon atoms, and having at least one triple bond at any position, including, for example, ethynylene, propynylene, butynelene, pentynylene, hexynylene, and the like.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and a carboxylate group.

The term "alkylthio" refers to -S-(alkyl) and -S-(unsubstituted cycloalkyl), wherein the alkyl is as defined above. Non-limiting examples of alkylthio include: methylthio, ethylthio, propylthio, butylthio, cyclopropylthio, cyclobutylthio, cyclopentylthio, and cyclohexylthio. Alkylthio may be optionally substituted or unsubstituted, and when it is substituted, the substituent is preferably one or more of the following groups, and it is substituted with one or more substituents independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "hydroxyalkyl" refers to alkyl substituted with hydroxy, wherein the alkyl group is as defined above.

The term "haloalkyl" refers to alkyl substituted with halogen, wherein the alkyl group is as defined above.

The term "deuterated alkyl" refers to alkyl substituted with a deuterium atom, wherein the alkyl group is as defined above.

The term "hydroxy" refers to the -OH group.

The term "oxo" refers to the =O group. For example, a carbon atom and an oxygen atom are connected by a double bond to form a ketone or aldehyde group.

The term "thio" refers to the =S group. For example, a carbon atom and a sulfur atom are connected by a double bond to form thiocarbonyl-C(S)-.

The term "halogen" refers to fluorine, chlorine, bromine, or iodine.

The term "amino" refers to $-NH_2$.

The term "cyano" refers to -CN.

The term "nitro" refers to $-NO_2$.

The term "carboxyl" refers to -C(O)OH.

The term "aldehyde" refers to -CHO.

The term "carboxylate group" refers to -C(O)O(alkyl) or -C(O)O(cycloalkyl), wherein the alkyl and cycloalkyl are as defined above.

The term "acyl halide" refers to a compound containing a -C(O)-halogen group.

The term "sulfonyl" refers to -S(O)(O)-.

The term "sulfinyl" refers to -S(O)-.

"Amino protecting group" is a suitable group known in the art for amino protection; see the literature ("Protective Groups in Organic Synthesis", 5Th. Ed. T. W. Greene & P. G. M. Wuts) for the amino protecting groups. Preferably, the amino protecting group may be ($C_{1-10}$ alkyl or aryl)acyl, e.g., formyl, acetyl, benzoyl, or the like; ($C_{1-6}$ alkyl or $C_{6-10}$ aryl) sulfonyl; ($C_{1-6}$ alkoxy or $C_{6-10}$ aryloxy)carbonyl, e.g., Boc or Cbz; or substituted or unsubstituted alkyl, e.g., trityl (Tr), 2,4-dimethoxybenzyl (DMB), p-methoxybenzyl (PMB), or benzyl (Bn).

"Optional" or "optionally" means that the event or circumstance subsequently described may, but does not necessarily, occur; this description includes instances where the event or circumstance occurs or does not occur. For example, "a heterocycloalkyl group optionally substituted with alkyl" means that alkyl may, but does not necessarily, exist; this description includes an instance where the heterocycloalkyl group is substituted with alkyl and an instance where it is not.

The term "pharmaceutical composition" refers to a mixture containing one or more of the compounds or the physiologically/pharmaceutically acceptable salts or pro-drugs thereof described herein, and other chemical components, as well as other components such as physiologically/pharmaceutically acceptable carriers and excipients. The pharmaceutical composition is intended to promote administration to an organism and facilitate the absorption of the active ingredient so that it can exert its biological activity.

The term "drug carrier" for the drug of the present disclosure refers to a system that can alter how the drug gets into a human body and the distribution of the drug in the human body, control the release rate of the drug, and deliver the drug to a targeted organ. The drug carrier release and targeted system can reduce drug degradation and loss, reduce side effects, and improve bioavailability. For example, polymeric surfactants that can be used as carriers can self-assemble due to their unique amphiphilic structures to form various forms of aggregates; preferred examples are, e.g., micelles, microemulsions, gels, liquid crystals, and vesicles. The aggregates have the capability of encapsulating drug molecules and have good permeability for membranes, and therefore can be used as excellent drug carriers.

The term "excipient" is an addition, besides the main drug, to a pharmaceutical formulation. It may also be referred to as an auxiliary material. For example, binders, fillers, disintegrants, and lubricants in tablets; the matrix part in semisolid ointment and cream preparations; preservatives, antioxidants, corrigents, fragrances, cosolvents, emulsifiers, solubilizers, osmotic pressure regulators, colorants, and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug's compression moldability and the like. When the drug in the tablet form contains oily components, an absorbent is necessarily added to absorb the oily components so as to maintain a "dry" state and thus facilitate the preparation of the tablet.

The compound of the present disclosure may contain one or more asymmetric centers and thus enantiomers and diastereomers may be generated. The enantiomers and diastereomers may be defined in terms of absolute stereochemistry as (*R*)- or (*S*)-, or other stereoisomeric forms of (*D*)- or (*L*)- for amino acids. The present disclosure includes all possible isomers as well as racemic and optically pure forms thereof. Optically active (+) and (-), (*R*)- and (*S*)-, or (*D*)- and (*L*)-isomers may be prepared by using chiral synthons or chiral reagents, or may be prepared by using conventional methods such as chromatography and fractional crystallization. Conventional methods for the preparation/separation of enantiomers include chiral synthesis from suitable optically pure precursors or resolution of the racemate (or the racemate of a salt or derivative) by using, for example, chiral high performance liquid chromatography (HPLC). When a compound

described herein contains an olefinic double bond or other geometric asymmetric centers, it is meant that the compound includes both E and Z geometric isomers, unless otherwise specified. Moreover, all tautomeric forms are also intended to be included.

In the chemical structures of the compounds of the present disclosure, a bond " 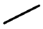 " represents an unspecified configuration; that is, if chiral isomers exist in the chemical structures, the bond " 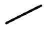 " may be " 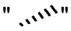 " or " 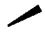 ", or both the configurations of " 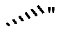 " and " 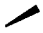 " are included simultaneously. In the chemical structures of the compounds of the present disclosure, a bond " 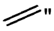 " is not specified with a configuration; that is, they may be in a Z configuration or an E configuration, or contain both configurations.

"Stereoisomer" refers to compounds composed of identical atoms bonded by the same bond but with different three-dimensional structures, which are not interchangeable. The present disclosure contemplates various stereoisomers and mixtures thereof, including "enantiomers" that refer to a pair of stereoisomers that are non-superimposable mirror images of one another.

"Tautomer" refers to the transfer of a proton from one atom of a molecule to another atom of the same molecule. Tautomers of any of the compounds are included in the present disclosure.

Any isotopically-labeled derivative of the compound or the pharmaceutically acceptable salt or the isomer thereof of the present disclosure is encompassed by the present disclosure. Atoms that can be isotopically labeled include, but are not limited to, hydrogen, carbon, nitrogen, oxygen, phosphorus, fluorine, chlorine, iodine, and the like. They may be separately replaced by the isotopes $^{2}H$ (D), $^{3}H$, $^{11}C$, $^{13}C$, $^{14}C$, $^{15}N$, $^{18}F$, $^{31}P$, $^{32}P$, $^{35}S$, $^{36}Cl$, $^{125}I$, etc. Unless otherwise stated, when a position is specifically designated as deuterium (D), that position shall be understood to be deuterium having an abundance that is at least 3000 times greater than the natural abundance of deuterium (which is 0.015%) (i.e., at least 45% deuterium incorporation).

## BRIEF DESCRIPTION OF THE DRAWINGS

FIG. 1: dose-response curves for the whole-cell binding activity of the test proteins to cells expressing human IL-4R$\alpha$
FIG. 2: dose-response curves for the blocking of the IL-4/IL-13 signaling pathway by the test proteins
FIG. 3: endocytosis ratio-time curves of the test proteins in TF-1 cells
FIG. 4: time-concentration curves of ADC-2 and free budesonide in tissues after intratracheal administration
FIG. 5: leukocyte and differential cell counts in the bronchoalveolar lavage fluid of Example 6
FIG. 6: leukocyte and differential cell counts in the bronchoalveolar lavage fluid of Example 7

## DETAILED DESCRIPTION

The preparation of the compound described herein and a pharmaceutically acceptable salt thereof is further described below in conjunction with examples, which are not intended to limit the scope of the present disclosure.

Experimental methods without conditions specified in the examples of the present disclosure were generally conducted under conventional conditions, or conditions recommended by the manufacturers of the starting materials or commercial products. Reagents without specific sources indicated are commercially available conventional reagents.

The NMR shifts ($\delta$) are given in $10^{-6}$ (ppm). The NMR analyses were performed on a Bruker AVANCE-400 nuclear magnetic resonance instrument, with dimethyl sulfoxide-D6 (DMSO-$d^6$), chloroform-D ($CDCl_3$), and methanol-D4 ($CD_3OD$) as solvents and tetramethylsilane (TMS) as an internal standard.

The MS analyses were performed on a Shimadzu 2010 Mass Spectrometer or Agilent 6110A MSD Mass Spectrometer.

The HPLC analyses were performed on Shimadzu LC-20A systems, Shimadzu LC-2010HT series, or Agilent 1200 LC high-performance liquid chromatograph (Ultimate XB-C18 3.0 $\times$ 150 mm chromatography column or Xtimate C18 2.1 $\times$ 30 mm chromatography column).

In the chiral HPLC analyses, Chiralpak IC-3 100 $\times$ 4.6 mm I.D., 3 $\mu$m, Chiralpak AD-3 150 $\times$ 4.6 mm I.D., 3 $\mu$m, Chiralpak AD-3 50 $\times$ 4.6 mm I.D., 3 $\mu$m, Chiralpak AS-3 150 $\times$ 4.6 mm I.D., 3 $\mu$m, Chiralpak AS-3 100 $\times$ 4.6 mm I.D., 3 $\mu$m, ChiralCel OD-3 150 $\times$ 4.6 mm I.D., 3 $\mu$m, Chiralcel OD-3 100 $\times$ 4.6 mm I.D., 3 $\mu$m, ChiralCel OJ-H 150 $\times$ 4.6 mm I.D., 5 $\mu$m, and Chiralcel OJ-3 150 $\times$ 4.6 mm I.D., 3 $\mu$m chromatography columns were used. The thin-layer chromatography silica gel plates used were Yantai Huanghai HSGF254 or Qingdao GF254 silica gel plates: the silica gel plates used in the thin-layer chromatography (TLC) analyses had a layer thickness of 0.15 mm-0.2 mm, and those used in the thin-layer chromatography separation and purification had a layer thickness of 0.4 mm-0.5 mm.

In the column chromatography steps, 100-200 mesh, 200-300 mesh, or 300-400 mesh Yantai Huanghai silica gel was generally used as a carrier.

In the preparative chiral chromatography steps, a DAICEL CHIRALPAK IC (250 mm × 30 mm, 10 μm) or Phenomenex-Amylose-1 (250 mm × 30 mm, 5 μm) column was used.

The CombiFlash preparative flash chromatograph used was Combiflash Rf150 (TELEDYNE ISCO). The kinase mean inhibition rates and $IC_{50}$ values were measured using a NovoStar microplate reader (BMG, Germany).

The known starting materials in the present disclosure may be synthesized by using or following methods known in the art, or may be purchased from companies such as ABCR GmbH & Co. KG, Acros Organics, Aldrich Chemical Company, Accela ChemBio Inc., and Chembee Chemicals.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 rounds of vacuumization and hydrogen filling. The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The monitoring of reaction progress in the examples was conducted using thin-layer chromatography (TLC). The developing solvents used in the reactions, the column chromatography eluent systems used in the compound purification, and the thin-layer chromatography developing solvent systems include: A: a dichloromethane/methanol system, B: a n-hexane/ethyl acetate system, C: a petroleum ether/ethyl acetate system, and D: a petroleum ether/ethyl acetate/methanol system. The volume ratio of the solvents was adjusted depending on the polarity of the compound, or adjusted by adding a small amount of basic or acidic reagents such as triethylamine and acetic acid.

Preparation of 1.0 M Tris buffer, pH = 8.30 ± 0.1:
6.0 g of tris was weighed into a 50 mL volumetric flask and dissolved by shaking in 40 mL of purified water, 1.2 mL of concentrated hydrochloric acid was added dropwise to adjust the pH to 8.30, and the solution was brought to volume with purified water.

Preparation of buffer A:
$KH_2PO4$ (8.50 g), $K_2HPO4$ (8.56 g), NaCl (5.86 g), and EDTA (1.50 g) were added to a 2.0 L vessel and completely dissolved in 1.6 L of water for injection by half an hour's stirring, and the solution was then brought to volume (2.0 L) with water for injection. The solution measured 6.30 ± 0.1 on the pH scale.

The abbreviations used in the experiments below have the following meanings:
DAST: diethylaminosulfur trifluoride; THF: tetrahydrofuran; NMP: N-methylpyrrolidone; DCM: dichloromethane; m-CPBA: m-chloroperoxybenzoic acid; DIEA: *N,N*-diisopropylethylamine; TEA: triethylamine; Boc: tert-butyloxycarbonyl; MeOH: methanol; $Et_2O$: diethyl ether.

Methods for preparing and purifying the 25G7 and 7B10 antibodies in the present application are described in patent application No. WO2020038454A1, which is incorporated herein by reference in its entirety.

**Example 1: Preparation of N-((10*S*)-10-benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5]indeno[1,2-d][1,3]dioxol-8b-yl)-1,6,9,12,15-pentaoxo-3-oxa-5,8,11,14-tetraazahexadecan-16-yl)-1-(2-bromoacetamido)-3,6,9,12-tetraoxapentadecan-15-amide**

(compound 1)

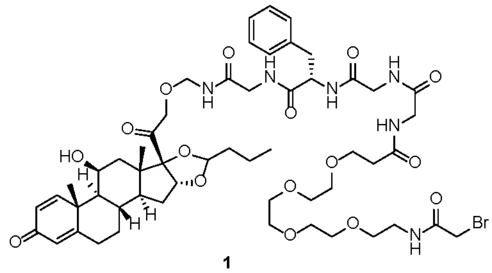

1

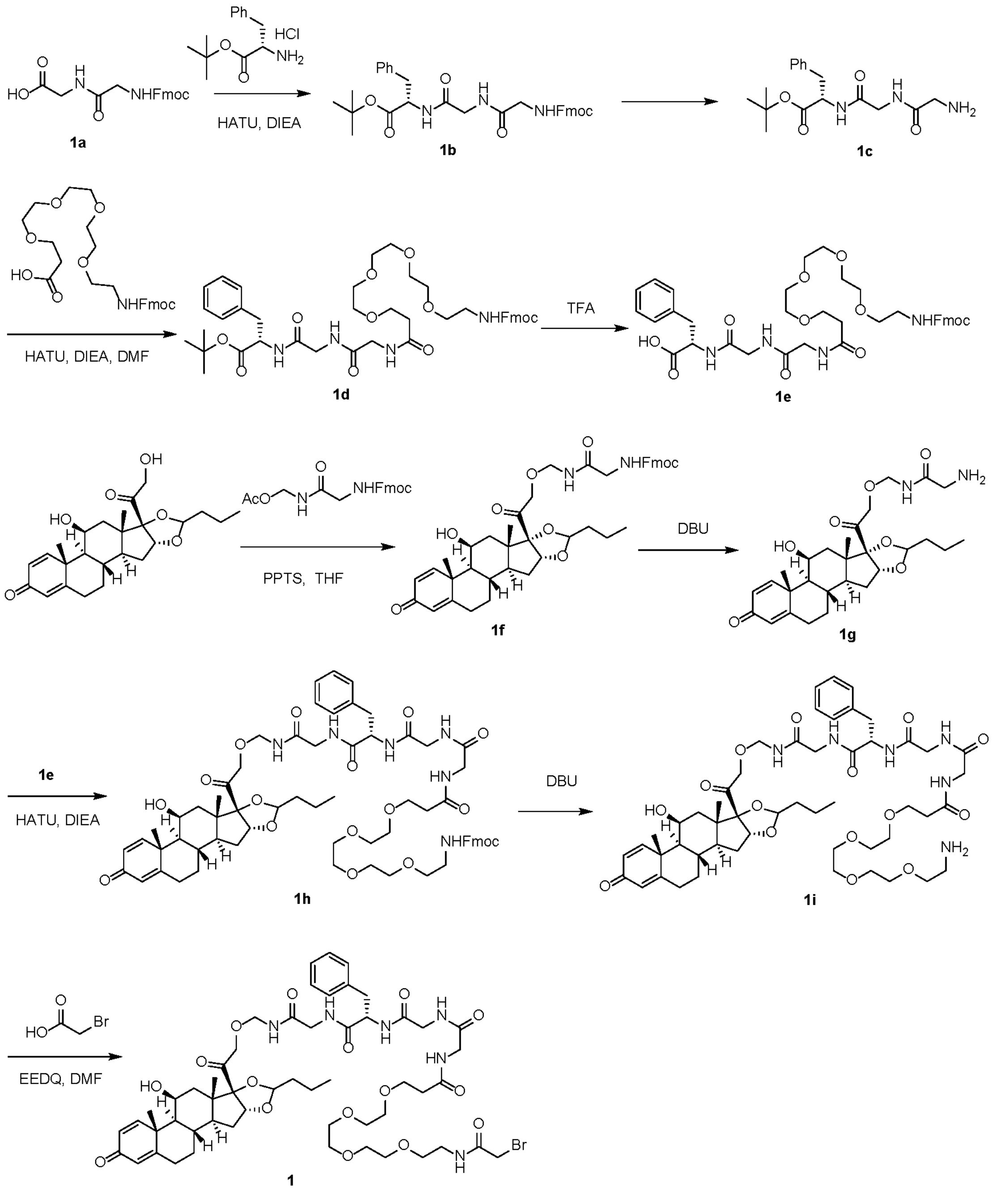


1a 1b 1c 1d 1e 1f 1g 1h 1i 1

**Step 1) Preparation of tert-butyl (((9*H*-fluoren-9-yl)methoxy)carbonyl)glycylglycyl-L-phenylalaninate** (compound 1b)

Compound **1a** (5.14 g, 20.0 mmol, 1.0 eq) and tert-butyl L-phenylalaninate hydrochloride (7.08 g, 20.0 mmol, 1.0 eq) were placed in a 250 mL three-necked flask and completely dissolved in anhydrous DMF (60 mL). The solution was cooled in an ice bath, and HATU (9.12 g, 24.0 mmol, 1.2 eq) and DIEA (7.74 g, 60.0 mmol, 3.0 eq) were then added. The mixture was left to react in the ice bath for 2 h. Water was added to the reaction mixture, and extraction was performed with ethyl acetate. The organic phase was washed with saturated brine, dried, and concentrated under reduced pressure to give compound **1b** (11.5 g, 100% yield). The crude product was directly used in the next step.

MS (ESI): m/z 580.3 $[M+Na]^+$.

$^1$H NMR (400 MHz, DMSO-d6) δ 8.21-8.16(m, 1H), 8.12-8.05 (m, 1H), 7.91-7.87 (m, 2H), 7.72-7.68 (m, 2H), 7.64-7.59 (m, 1H), 7.44-7.38 (m, 2H), 7.36-7.25 (m, 4H), 7.23-7.18 (m, 3H), 4.41-4.18 (m, 4H), 3.76-3.72 (m, 2H), 3.68-3.61 (m, 2H), 2.98-2.89 (m, 2H), 2.69 (s, 2H), 1.30 (s, 9H).

**Step 2) Preparation of tert-butyl glycylglycyl-L-phenylalaninate** (compound 1c)

Compound **1b** (5.57 g, 10.0 mmol, 1.0 eq) was placed in a 250 mL three-necked flask and completely dissolved in anhydrous DCM (60 mL). The solution was cooled in an ice bath. Piperidine (8.5 g, 100.0 mmol, 10.0 eq) was slowly added. After the addition, the mixture was stirred at room temperature for about 2 h. The reaction mixture was directly concentrated to dryness, and the crude product was purified by column chromatography to give compound **1c** (2.70 g, 81% yield).

MS (ESI): m/z 358.3[M+H]$^+$.

**Step 3) Preparation of tert-butyl (1-(9*H*-fluoren-9-yl)-3-oxo-2,7,10,13,16-pentaoxa-4-azanonadecan-19-oyl)glycylglycyl-L-phenylalaninate** (compound 1d)

The starting materials 1-(9*H*-fluoren-9-yl)-3-oxo-2,7,10,13,16-pentaoxa-4-azanonadecan-19-oic acid (1.0 g, 2.05 mmol, 1.0 eq; from Tonglai Biochemical) and **1c** (0.69 g, 2.05 mmol, 1.0 eq) were added to a 100 mL three-necked flask and completely dissolved in anhydrous DMF (25 mL). The solution was cooled in an ice bath. HATU (1.01 g, 2.67 mmol, 1.3 eq) and DIEA (529 mg, 4.10 mmol, 2.0 eq) were added, and the mixture was left to react in the ice bath for 1 h. Water was added to the reaction mixture, and extraction was performed with ethyl acetate. The organic phase was washed with saturated brine, dried, and concentrated under reduced pressure to give compound **1d** (1.60 g, 97% yield).

MS (ESI): m/z 805.3 [M+H]$^+$.

**Step 4) Preparation of (1-(9*H*-fluoren-9-yl)-3-oxo-2,7,10,13,16-pentaoxa-4-azanonadecan-19-oyl)glycylglycyl-L-phenylalanine** (compound 1e)

Compound **1d** (1.60 g, 1.99 mmol, 1.0 eq) was added to a 100 mL single-necked flask and dissolved in anhydrous dichloromethane (16 mL). Trifluoroacetic acid (8 mL) was added at room temperature, and the mixture was stirred for 2 h. The reaction mixture was directly concentrated to dryness, and ethyl acetate was added to dissolve the residue. The solution was washed with saturated brine, dried, and concentrated under reduced pressure to give compound **1e** (1.23 g, 83% yield);

MS (ESI): m/z 749.3 [M+H]$^+$.

**Step 5) Preparation of (9*H*-fluoren-9-yl)methyl (2-(((2-((6aR,6bS,7S,8aS,8bS,llaR,12aS,12bS)-7-hydroxy-6a,8a-dimethyl-4-oxo-10-propyl-1,2,4,6a,6b,7,8,8a,11a,12,12a,12b-dodecahydro-8bH-naphtho[2',1':4,5]indeno[1,2-d][1,3]dioxol-8b-yl)-2-oxoethoxy)methyl)amino)-2-oxoethyl)carbamate** (compound 1f)

The starting materials budesonide (1.29 g, 3.0 mmol, 1.0 eq) and (2-((((9H-fluoren-9-yl)methoxy)carbonyl)amino)acetamido)methyl acetate (1.16 g, 3.15 mmol, 1.0 eq; prepared using the method in "Tetrahedron, 2018, 74(15), 1951-1956") and pyridinium p-toluenesulfonate (75 mg, 0.30 mmol, 0.1 eq) were added to a 100 mL three-necked flask. Anhydrous tetrahydrofuran (20 mL) was added at room temperature, and the mixture was heated at reflux for 4 h. The reaction mixture was directly concentrated to dryness, and the crude product was purified by column chromatography to give **If** (0.54 g, 24% yield).

MS (ESI): m/z 739.4 [M+H]$^+$.

$^1$H NMR (400 MHz, DMSO-d6) δ 8.76-8.69 (m, 1H), 7.91-7.84 (m, 2H), 7.73-7.67 (m, 2H), 7.63-7.57 (m, 1H), 7.43-7.58 (m, 2H), 7.35-7.23 (m, 3H), 6.13 (d, *J* = 7.5 Hz, 1H), 5.91 (s, 2H), 5.15-4.97 (m, 1H), 4.73-4.45 (m, 5H), 4.30-4.09 (m, 5H), 3.65-3.59 (m, 2H), 2.29-2.21 (m, 1H), 2.11-1.87 (m, 2H), 1.74-1.21 (m, 11H), 1.09-0.77 (m, 8H).

**Step 6) Preparation of 2-amino-N-((2-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8bH-naphtho[2',1':4,5]indeno[1,2-d][1,3]dioxol-8b-yl)-2-oxoethoxy)methyl)acetamide** (compound 1g)

Compound **If** (540 mg, 0.73 mmol, 1.0 eq) was placed in a 25 mL single-necked flask and dissolved in anhydrous dichloromethane (10 mL). The solution was cooled in an ice bath, and DBU (167 mg, 1.10 mmol, 1.5 eq) was added. The mixture was stirred for 30 min. Water was added, and the aqueous phase was separated and extracted with dichloromethane twice. The organic phase was washed with saturated brine and concentrated under reduced pressure to give compound **Ig** (450 mg, 100% yield). The crude product was directly used in the next step.

MS (ESI): m/z 517.4 [M+H]$^+$.

**Step 7) Preparation of (9*H* fluoren-9-yl)methyl ((10*S*)-10-benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8bH naphtho[2',1':4,5] indeno[1,2-d][1,3]dioxol-8b-yl)-1,6,9,12,15,18-hexaoxo-3,21,24,27,30-pentaoxa-5,8,11,14,17-pentaazadotriacontan-32-yl)carbamate** (compound 1h)

Compounds **lg** (0.45 g of crude product, equivalent to 0.73 mmol, 1.0 eq) and **1e** (0.55 g, 0.73 mmol, 1.0 eq) were placed in a 100 mL three-necked flask and completely dissolved in anhydrous DMF (9 mL). The solution was cooled in an ice bath, and HATU (361 mg, 0.95 mmol, 1.3 eq) and DIEA(189 mg, 1.46 mmol, 2.0 eq) were then added sequentially. The mixture was stirred for 1 h. Water was added to the reaction mixture, and extraction was performed with dichloromethane twice. The organic phases were combined, washed with saturated brine once, dried, and concentrated under reduced pressure to give a crude product. The crude product was purified by trituration in a solvent mixture of petroleum ether and ethyl acetate to give compound **1h** (710 mg, 78% yield).

MS (ESI): m/z 1269.7 $[M+Na]^+$.

**Step 8) Preparation of 1-amino-*N*-((10*S*)-10-benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5]indeno[1,2-d] [1,3]dioxol-8b-yl)-1,6,9,12,15-pentaoxo-3-oxa-5,8,11,14-tetraazahexadecan-16-yl)-3,6,9,12-tetraoxapentadecan-15-amide** (compound 1i)

Compound **1h** (350 mg, 0.28 mmol, 1.0 eq) was placed in a 25 mL single-necked flask and completely dissolved in anhydrous dichloromethane (10 mL). The solution was cooled in an ice bath, and DBU (64 mg, 0.42 mmol, 1.5 eq) was added. The mixture was stirred for 1 h. The reaction mixture was directly concentrated to dryness to give a crude product, and the crude product was then purified by column chromatography to give compound **li** (289 mg, 89% yield).

MS (ESI): m/z 1025.6 $[M+H]^+$.

**Step 9) Preparation of N-((10*S*)-10-benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho [2',1' :4,5] indeno [1,2-d] [1,3] dioxol-8b-yl)-1,6,9,12,15-pentaoxo-3-oxa-5,8,11,14-tetraazahexadecan-16-yl)-1-(2-bromoacetamido)-3,6,9,12-tetraoxapentadecan-15-amide** (compound 1)

Solid bromoacetic acid (35.2 mg, 0.127 mmol, 1.0 eq) and EEDQ (63 mg, 0.25 mmol, 2.0 eq) were placed in a 25 mL Schlenk-Tube and completely dissolved in anhydrous DMF (5 mL). The solution was stirred at room temperature for 1 h. Compound **li** (130 mg, 0.127 mmol, 1.0 eq) was dissolved in DMF (2 mL), and the resulting solution was added dropwise to the active ester solution prepared above. After the addition, the mixture was left to react at room temperature for 2 h. Water was added to the reaction system, and extraction was performed with ethyl acetate twice. The organic phases were combined, washed with saturated brine, dried, and concentrated under reduced pressure to give a crude product, and the crude product was then purified by column chromatography to give compound **1** (39 mg, 27% yield).

MS (ESI): m/z 1145.5/1147.6 $[M+1]^+$.

$^1H$ NMR (400 MHz, $CDCl_3$) δ 8.62-8.57 (m, 1H), 8.34-8.28 (m, 2H), 8.19-8.11(m, 2H), 8.03-7.99(m, 1H), 7.30-7.17(m, 6H), 6.15 (d, *J* = 9.6 Hz, 1H), 5.92 (s, 2H), 5.18-5.03 (m, 1H), 4.72-4.47 (m, 5H), 4.29-4.15 (m, 2H), 3.78-3.42 (m, 27H), 3.27-3.21 (m, 2H), 3.06-2.84 (m, 2H), 2.41-2.28 (m, 3H), 2.06-1.73 (m, 2H), 1.60-1.24 (m, 10H), 1.01-0.82 (m, 8H).

**Example 2:**

**N-((10*S*)-10-Benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho [2',1' :4,5] indeno [1,2-d] [1,3] dioxol-8b-yl)-1,6,9,12,15-pentaoxo-3-oxa-5,8,11,14-tetraazahexadecan-16-yl)-1-(2,5-dioxo-2,5-dihydro-1*H*-pyrrol-1-yl)-3,6,9,12-tetraoxapentadecan-15-amide**

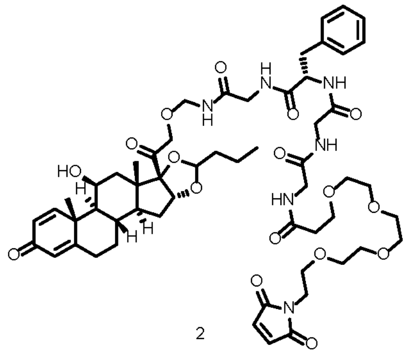

2

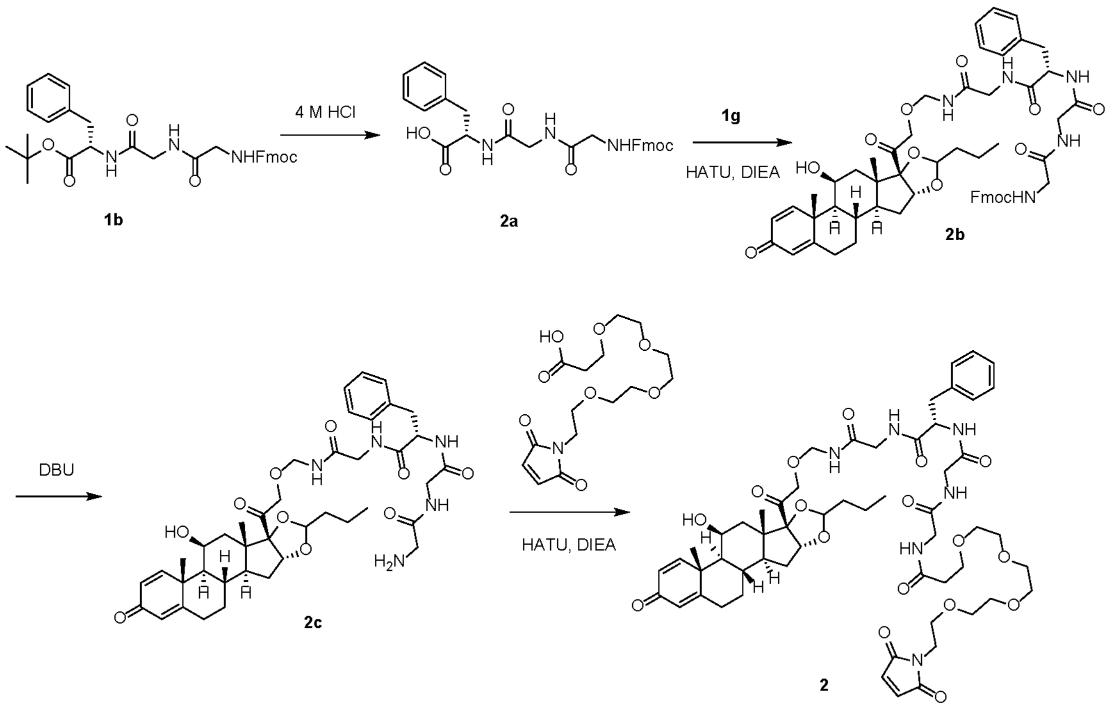


1b 2a 2b 2c 2

**Step 1) Preparation of (((9*H*-fluoren-9-yl)methoxy)carbonyl)glycylglycyl-L-phenylalanine** (compound 2a)

Compound **1b** (1.50 g, 2.69 mmol, 1.0 eq) was placed in a 100 mL single-necked flask and dissolved in a 4 M solution of HCl in 1,4-dioxane (20 mL). The solution was stirred at room temperature for 4 h until the starting material **1b** was completely consumed. The reaction mixture was directly concentrated to dryness to give compound **1a** (1.34 g, 100% yield).

MS (ESI): m/z 502.3 $[M+1]^+$

**Step 2) Preparation of (9*H* fluoren-9-yl)methyl ((10*S*)-10-benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5] indeno[1,2-d] [1,3]dioxol-8b-yl)-1,6,9,12,15-pentaoxo-3-oxa-5,8,11,14-tetraazahexadecan-16-yl)carbamate** (compound 2b)

Compounds **2a** (0.97 g, 1.63 mmol, 1.0 eq) and **1g** (1.0 g, 1.95 mmol, 1.2 eq) were placed in a 100 mL three-necked flask. DMF (20 mL) was added, and the mixture was cooled in an ice bath. HATU (927 mg, 2.44 mmol, 1.5 eq) and DIEA(420 mg, 3.26 mmol, 2.0 eq) were sequentially added. After the addition, the mixture was left to react in the ice bath for 1-2 h. The reaction mixture was concentrated and purified by column chromatography to give compound **2b** (860 mg, 53% yield).

MS (ESI): m/z 1000.5 $[M+1]^+$

**Step 3) Preparation of (2*S*)-2-(2-(2-aminoacetamido)acetamido)-*N*-(2-(((2-((6a*R*,6b*S*,7*S*,8a*S*,8b-*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5]indeno[1,2-d] [1,3]dioxol-8b-yl)-2-oxoethoxy)methyl)amino)-2-oxoethyl)-3-phenylpropanamide** (compound 2c)

Compound **2b** (860 mg, 0.86 mmol, 1.0 eq) was placed in a 25 mL single-necked flask and completely dissolved in anhydrous DMF (10 mL). The solution was cooled in an ice bath, and DBU (196 mg, 1.29 mmol, 1.5 eq) was added. The mixture was stirred for 30 min. The reaction mixture was directly concentrated and purified by column chromatography to give compound **2c** (485 mg, 72% yield).

MS (ESI): m/z 778.4 $[M+1]^+$

**Step 4) Preparation of *N*-((10*S*)-10-Benzyl-1-((6a*R*,6b*S*,7*S*,8a*S*,8b*S*,11a*R*,12a*S*,12b*S*)-7-hydroxy-6a,8a-dimethyl-4-oxo-10-propyl-1,2,4,6a,6b,7,8,8a,11a,12,12a,12b-dodecahydro-8b*H*-naphtho[2',1':4,5]indeno[1,2-d] [1,3]dioxol-8b-yl)-1,6,9,12,15-pentaoxo-3-oxa-5,8,11,14-tetraazahexadecan-16-yl)-1-(2,5-dioxo-2,5-dihydro-1*H* pyrrol-1-yl)-3,6,9,12-tetraoxapentadecan-15-amide** (compound 2d)

Compound **2c** (480 mg, 0.62 mmol, 1.0 eq) and 1-(2,5-dioxo-2,5-dihydro-1H-pyrrol-1-yl)-3,6,9,12-tetraoxapentadecan-15-oic acid (213 mg, 0.62 mmol, 1.0 eq; from Tonglai Biochemical) were placed in a 100 mL three-necked flask and completely dissolved in anhydrous DMF (10 mL). The solution was cooled in an ice bath, and HATU (352 mg, 0.93 mmol, 1.5 eq) and DIEA (159 mg, 1.23 mmol, 2.0 eq) were then added sequentially. The mixture was stirred for 1 h. The reaction mixture was directly concentrated and purified by column chromatography to give compound **2d** (360 mg, 53% yield).

MS (ESI): m/z 1105.6 $[M+1]^+$

$^1$H NMR (400 MHz, DMSO-d6) δ 8.61-8.56 (m, 1H), 8.32-8.26 (m, 1H), 8.22-8.13 (m, 2H), 8.03-7.96(m, 1H), 7.39-7.20(m, 6H), 7.02 (s, 2H), 6.17-6.13 (m, 1H), 5.92 (s, 2H), 5.16-4.99 (m, 1H), 4.76-4.40 (m, 6H), 4.29-4.16 (m, 2H), 3.64-3.19 (m, 26H), 3.09-3.02 (m, 1H), 2.86-2.79 (m, 1H), 2.41-2.26 (m, 3H), 2.12-1.90 (m, 2H), 1.78-1.25 (m, 10H), 1.03-0.83 (m, 8H).

**Example 3: Process of Preparing 25G7-Fab Protein**

The gene sequence of the antibody was synthesized and subcloned into the pcDNA3.4 vector. Top10 competent cells were transformed with the ligation products, and positive clones were picked for expansion culture. The clones were inoculated into a culture medium for expansion culture. Plasmids containing the deoxyribonucleotide sequence of 25G7-Fab antibody were extracted by the Maxi preparation method. The liposome transfection method was adopted: the expression plasmids and a transfection reagent were mixed and added to Expi 293 cells, and after the cells were cultured for 5 days in a thermostatic incubator, the cell culture was harvested. The cell culture was centrifuged, the supernatant was collected and filtered to remove cell debris, and the clarified liquid was collected. The protein of interest was captured using a kappa select chromatography column. After filtration, the concentration of the protein of interest was obtained by measuring the A280 absorbance with a Nanodrop and dividing it by the extinction coefficient, and then multiplied by the volume to obtain the final yield. The protein was subjected to gel electrophoresis (SDS-PAGE) analysis, size exclusion chromatography (SEC-HPLC) analysis, and endotoxin testing, and the final product was confirmed as 25G7-Fab.

**Example 4: Process of Preparing AZD1402-TAG Protein**

AZD1402-TAG (the protein sequence is from SEQ ID NO: 1 in the patent WO2020200960A1; the sequence has a His tag fused at the C-terminus for facilitating protein preparation and purification, and the sequence was finally designated AZD1402-TAG).
AZD1402-TAG

ASDEEIQDVSGTWYLKAMTVDSRCPRAVYNSVTPMTLTTLEGGNLEAKFTAQRKGRWQK
YKLVLEKTDEPGKYTASGGRHVAYIIRSHVKDHYIFHSEGLCPGQPVPGVWLVGRDPKNNL
EALEDFEKAAGARGLSTESILIPRQSETSSPGSDHHHHHH
SEQ ID NO: 52

The coding gene sequence for the protein above was synthesized and subcloned into pcDNA3.4. The expression

plasmid was mixed with a transfection reagent, and the mixture was incubated at 37 °C for 15 min and added dropwise to a HEK293 cell solution. After the cell solution was incubated on a 37 °C shaker for one week, the cell culture was centrifuged, and the supernatant was collected. An imidazole-free buffer was used to equilibrate a nickel affinity chromatography column, and the protein sample was then loaded onto the nickel affinity chromatography column. After the column was equilibrated again with the imidazole-free buffer, a buffer containing a high concentration of imidazole was used to elute the column-bound protein. The eluted protein was transferred to a dialysis bag and dialyzed against 1 × PBS to substitute the PBS storage buffer for the elution buffer. Analysis showed that the protein of interest was obtained.

**Examples 5: Preparation of Antibody-Drug Conjugate ADC-1**

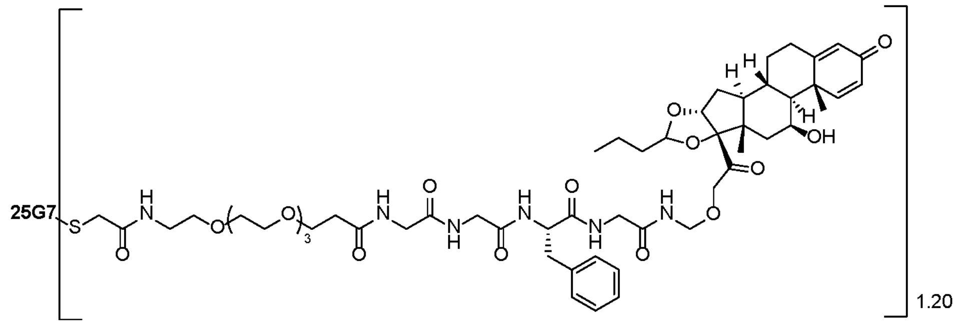

ADC-1

Preparation of buffer A: $KH_2PO_4$ (8.50 g), $K_2HPO_4$ (8.56 g), NaCl (5.86 g), and EDTA (1.50 g) were added to a 2.0 L vessel and completely dissolved in 1.6 L of water for injection by half an hour's stirring, and the solution was then brought to volume (2.0 L) with water for injection. The solution measured 6.30 ± 0.1 on the pH scale.

To an aqueous buffer solution of antibody 25G7-Fab (the heavy chain sequence is set forth in SEQ ID NO: 50 and the light chain sequence is set forth in SEQ ID NO: 45) in buffer A (a pH 6.3, 0.05 M aqueous buffer solution; 10.0 mg/mL, 0.5 mL, 0.125 mmol) at 37 °C was added a prepared aqueous solution of tris(2-carboxyethyl)phosphine (TCEP) (2.5 mM, 100.0 μL, 0.250 mmol). The mixture was left to react on a water-bath shaker at 37 °C for 3 h, and the reaction was stopped. The reaction mixture was cooled to 25 °C in a water bath.

1.0 M Tris buffer (50 μL) was added to the above reaction mixture. Compound **1** (1.43 mg, 1.250 mmol) was dissolved in 25 μL of DMSO, and the solution was added to the above reaction mixture. The mixture was left to react on a water-bath shaker at 25 °C for 3 h, and the reaction was stopped. The reaction mixture was desalted and purified using a Sephadex G25 gel column (eluent phase: buffer A) and concentrated using an ultrafiltration tube to give the title product ADC-1 in buffer A (6.06 mg/mL, 0.60 mL). The product was refrigerated at 4 °C.

**Examples 6: Preparation of Antibody-Drug Conjugate ADC-2**

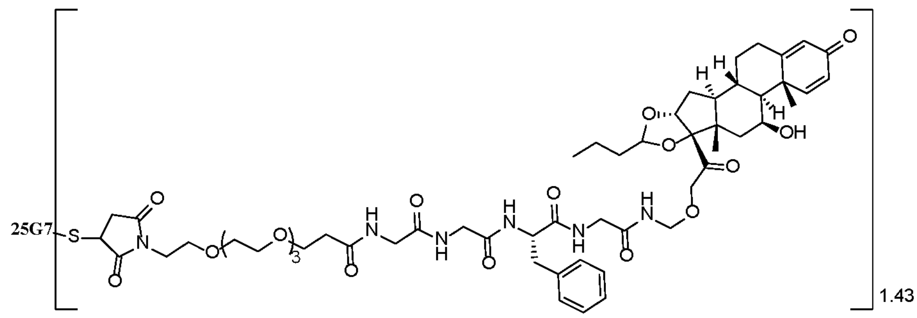

ADC-2

To an aqueous buffer solution of antibody 25G7-Fab in buffer A (a pH 6.3, 0.05 M aqueous buffer solution; 10.0 mg/mL, 6.0 mL, 1.50 mmol) at 37 °C was added a prepared aqueous solution of tris(2-carboxyethyl)phosphine (TCEP)

(10.0 mM, 1.50 mL, 15.0 mmol). The mixture was left to react on a water-bath shaker at 37 °C for 3 h, and the reaction was stopped. The reaction mixture was cooled to 25 °C in a water bath.

Compound 2 (12.2 mg, 12.0 mmol) was dissolved in 300 μL of DMSO, and the solution was added to the above reaction mixture. The mixture was left to react on a water-bath shaker at 25 °C for 3 h, and the reaction was stopped. The reaction mixture was desalted and purified using a Sephadex G25 gel column (eluent phase: buffer A) and concentrated using an ultrafiltration tube to give the title product ADC-2 in buffer A (3.65 mg/mL, 12.8 mL). The product was refrigerated at 4 °C.

**Biological Evaluations**

The present disclosure is further described and explained below with reference to test examples, which are not intended to limit the scope of the present disclosure.

**Test Example 1: Affinity of Antibody-Drug Conjugate ADC-2 for Human IL-4Rα Protein**

1. Test proteins

Antibody-drug conjugate ADC-2, 25G7-Fab, and AZD1402-TAG

2. Experimental method

The affinities of antibody-drug conjugate ADC-2, 25G7-Fab, and AZD1402-TAG for human IL-4Rα antigen protein were measured using surface plasmon resonance (SPR) technology. An SA sensor chip (Cytiva) was placed in Biacore 8K (Cytiva), and Biotin-labeled human IL-4Rα protein (SinoBiological, CAT# 10402-H08H-B) was captured on the SA sensor chip. HBS-EP + buffer solution (10 mM HEPES, 150 mM NaCl, 3 mM EDTA, 0.05% surfactant P20) was used as the mobile phase. Each test protein was serially diluted 2-fold with HBS-EP + buffer solution and then injected as an analyte. The flow rate was set to 30 μL/min. For ADC-2, 25G7-Fab, and AZD1402-TAG, the association time was 100 s, and the dissociation time was 600 s. Analysis was performed using Biacore 8K evaluation software (Cytiva), and data on the affinities of the test proteins were obtained.

3. Experimental results

Table 1. The affinity KD values for the binding of the test proteins to human IL-4Rα protein

| Captured protein | Test protein | Kon (1/Ms) | Koff (1/s) | KD (pM) |
|---|---|---|---|---|
| hIL-4Rα | ADC-2 | 8.78E+06 | 9.62E-05 | 11.0 |
| | 25G7-Fab | 4.69E+06 | 2.06E-04 | 43.9 |
| | AZD 1402-TAG | 4.77E+06 | 1.02E-04 | 21.3 |

The results are shown in Table 1. ADC-2 bound to human IL-4Rα antigen protein with a KD value of 11.0 pM, which was slightly better than those of AZD1402-TAG and 25G7-Fab.

**Test Example 2: Whole-Cell Binding Activity of Antibody-Drug Conjugate ADC-2 to Cells Expressing Human IL-4Rα**

1. Test proteins

Antibody-drug conjugate ADC-2, 25G7-Fab, hu25G7 (the heavy chain sequence is set forth in SEQ ID NO: 44 and the light chain sequence is set forth in SEQ ID NO: 45), and AZD1402-TAG.

2. Experimental method

It was confirmed that the TF-1 and Karpas 299 cell strains can express IL-4Rα. TF-1 and Karpas299 cells in the logarithmic growth phase were harvested and counted, and the cell density was adjusted. The cells were inoculated onto a 96-well round-bottom plate at 1 × $10^5$ cells/well. The cells were incubated with different concentrations of anti-IL-4R

antibodies (25G7, and naked antibody 25G7-Fab), antibody-drug conjugate ADC-2, and AZD1402-TAG at 4 °C for 45 min. After the antibodies were washed off with FACS buffer (PBS containing 2% FBS), a fluorescently labeled secondary antibody was added for staining. For 25G7-Fab, hu25G7, and ADC-2, an FITC-conjugated anti-human IgG (Fab-specific) secondary antibody (Sigma, CAT#F5512-1ML) was used, and for AZD1402-TAG, an FITC-conjugated anti-His antibody (GenScript, CAT#A01620) was used. The cells were incubated with the secondary antibodies at 4 °C for 30 min, then washed with FACS buffer twice, and assayed on a flow cytometer (BD, FACS Celesta), and the results were analyzed using FlowJo (FlowJo, LLC) software.

3. Experimental results

Table 2. $EC_{50}$ values for the whole-cell binding activity of the test proteins to cells expressing human IL-4Rα

| Test protein | ADC-2 | 25G7-Fab | hu25G7 | AZD1402-TAG |
|---|---|---|---|---|
| Secondary antibody for assay | Anti-hFab-FITC | | | Anti-His-FITC |
| $EC_{50}$ (nM, TF-1) | 0.107 | 0.243 | 0.247 | 0.0124 |

The results are shown in FIG. 1 and Table 2. ADC-2 bound to TF-1 cells with an $EC_{50}$ value of 0.107 nM, which was comparable to those of naked antibody 25G7-Fab and hu25G7. The binding effect of AZD1402-TAG on TF-1 cells was strong; however, the data are not comparable with those for ADC-2 and 25G7-Fab, among others, due to the different assay antibodies.

**Test Example 3: Blocking of IL-4/IL-13 Signaling Pathway by Antibody-Drug Conjugate ADC-**2

1. Test proteins

Antibody-drug conjugate ADC-2 and 25G7-Fab.

2. Experimental method

STAT6 activation is a key step in the activation of the IL-4/IL-13 signaling pathway. In this example, the blocking effect of ADC-2 on the IL-4/IL-13 signaling pathway was evaluated through the HEK-Blue™ IL-4/IL-13 reporter gene cell strain. The cells were purchased from Invivogen (Cat#hkb-il413). In the cells, the human STAT6 gene and the secreted alkaline phosphatase (SEAP) reporter gene, the expression of which is induced by phosphorylated STAT6, are overexpressed. The level of activation of the IL-4/IL-13 signaling pathway can be evaluated by measuring the content of SEAP secreted in the cell culture supernatant with the SEAP substrate QUANTI-Blue.

HEK-Blue™ IL-4/IL-13 cells in the logarithmic growth phase were harvested, and the density was adjusted to $5 \times 10^5$ cells/mL. The cells were added to a 96-well flat-bottom culture plate at 100 μL/well, and the plate was incubated at 37 °C with 5% $CO_2$ for 24 h. Then the test proteins, serially diluted, and recombinant human IL-4 or IL-13 were added at 20 μL/well, and the cell culture plate was incubated in a 37 °C, 5% $CO_2$ incubator for another 20-24 h. After the culture was complete, the cell culture plate was taken out, and 20 μL of the cell culture supernatant was transferred from each well to another 96-well plate. QUANTI-Blue solution, pre-heated at 37 °C, was added at 180 μL/well. After 1 h of incubation at 37 °C, the absorbance at the wavelength of 620 nm was measured.

3. Experimental results

Table 3. $IC_{50}$ values for the blocking of the IL-4/IL-13 signaling pathway by the test proteins

| Test protein | ADC-2 | 25G7-Fab |
|---|---|---|
| $IC_{50}$ value (nM) for blocking of IL-4 signaling pathway | 0.68 | 0.61 |
| $IC_{50}$ value (nM) for blocking of IL-13 signaling pathway | 10.60 | 14.59 |

The results are shown in FIG. 2 and Table 3. Each test protein exhibited a blocking effect on STAT6 activation

induced by recombinant human IL-4 and IL-13: ADC-2 blocked the IL-4 signaling pathway with an $IC_{50}$ value of 0.68 nM, which is close to the $IC_{50}$ value of 25G7-Fab, which was 0.61 nM; ADC-2 blocked the IL-13 signaling pathway with an $IC_{50}$ value of 10.60 nM, which is comparable to the $IC_{50}$ of 25G7-Fab, which was 14.59 nM.

**Test Example 4: Endocytic Activity of Antibody-Drug Conjugate ADC-2 in TF-1 Cells**

1. Test proteins

Antibody-drug conjugate ADC-2, 25G7-Fab, hu25G7, and AZD 1402-TAG.

2. Experimental method

TF-1 cells in a good growth state were harvested, and the density was adjusted to $1 \times 10^6$ cells/mL. The cells were added to a 96-well cell culture plate at 100 μL/well, and the plate was left at 4 °C. The test proteins, with a final concentration of 2 nM, were added, and the plate was incubated at 4 °C for 1 h and then continued to be incubated in a 4 °C, 5% $CO_2$ cell incubator. For each time point, a culture plate was set. The plates were taken out at corresponding incubation time points, washed with FACS buffer, and centrifuged. A fluorescently labeled secondary antibody was then added, and incubation was performed. For 25G7-Fab, hu25G7, and ADC-2, an FITC-conjugated anti-human IgG (Fab-specific) secondary antibody was used for labeling, and for AZD1402-TAG, an FITC-conjugated anti-His antibody was used for labeling. The cells were incubated with the secondary antibodies at 4 °C for 30 min, then washed with FACS buffer twice, and assayed on a flow cytometer (BD, FACS Celesta), and the results were analyzed using FlowJo (FlowJo, LLC) software. The endocytosis ratio at a specific time point was calculated by the formula:

$$(gMFI_{\text{Test Ab at time X}} - gMFI_{\text{ISO Ab at time X}})/(gMFI_{\text{Test Ab at time zero}} - gMFI_{\text{ISO Ab at time zero}})*100\%$$

3. Experimental results

The experimental results are shown in FIG. 3. The endocytosis signal of each test protein in TF-1 cells was detected. The endocytosis activity of ADC-2 was comparable to that of hu25G7, and the 3-hour endocytosis ratio was about 70%. The endocytosis of 25G7-Fab was faster, and its endocytosis ratio was also higher. Both the endocytosis rates and the endocytosis ratios of ADC-2, 25G7-Fab, and hu25G7 were significantly better than those of AZD1402-TAG, plateauing at about 2 h. The endocytic activity of ADC-2 was slightly weaker than that of 25G7-Fab.

**Test Example 5: Pharmacokinetic Testing of Antibody-Drug Conjugate ADC-2 on Mouse Model**

1. Test protein

Antibody-drug conjugate ADC-2

2. Experimental method

2.1. Experimental animals

C57BL6/J mice, 8 weeks old, male, Cyagen (Suzhou) Biosciences Co., Ltd.

2.2. Mice administration procedure

Mice were given medication via intratracheal nebulization (i.t.). At different time points post-administration, 300-400 μL of blood was collected from a mandibular blood vessel, left to stand for 2 h, and then centrifuged at 7500 rpm and 4 °C for 10 min, and the serum was collected. Following the blood collection, the mice were anesthetized and immobilized, the neck muscles of the mice were bluntly isolated to expose the trachea, and an incision was horizontally made in the trachea. A lavage syringe needle was slowly inserted, the normal saline pre-loaded, pre-cooled at 4 °C, was slowly injected into the lung tissue for lavage, and the bronchoalveolar lavage fluid (BALF) was collected. Lavage was performed 3 times, with 300 μL of normal saline used for each of the first two lavages and 400 μL for the third. The collected bronchoalveolar lavage fluid was centrifuged at 1000 rpm and 4 °C for 10 min, and the supernatant was collected. After the mice were euthanized, their thoracic cavities were opened, and the complete lung tissues were isolated and collected. The tissues were rinsed with PBS to remove blood stains on the surfaces, and the surface PBS was dried off with paper towels

before weighing the lung tissues. The lung tissues were placed in 2 mL centrifuge tubes, and 1 mL of cell lysis buffer (CST, #9803) was added. The tissues were homogenized using a homogenizer (Shanghai Jing Xin, Tissuelyser-48) at 60 Hz for 2 min and then centrifuged at 10,000× g and 4 °C for 10 min. The supernatants were lung tissue homogenates. The protein concentrations in the homogenates were measured (Pierce, 23227). The serum, BALF, and lung tissue homogenates were stored at -80°C. The administration regimen is shown in Table 4, and the sampling protocol is shown in Table 5.

Table 4. Administration regimen

| Group | Number of animals | Test protein | Dose (μg) | Route of administration | Frequency of administration |
|---|---|---|---|---|---|
| ADC-2 group | 15 | ADC-2 | 100 | i.t. | Once |

Table 5. Sampling protocol

<table>
<tr><td colspan="7">Time post-administration</td></tr>
<tr><td>5 min</td><td>1 h</td><td>6 h</td><td>24 h</td><td>48 h</td><td>Pre-dose</td><td>30 min</td></tr>
<tr><td colspan="7">Animal No.</td></tr>
<tr><td>1, 2, 3</td><td>4, 5, 6</td><td>7, 8, 9</td><td>10, 11, 12</td><td>13, 14, 15</td><td>10, 11, 12</td><td>13, 14, 15</td></tr>
<tr><td colspan="5">At these time points, serum was collected first, and BALF and lung tissue were collected after the animal was sacrificed</td><td colspan="2">At these time points, only serum was collected</td></tr>
</table>

2.3. Measurement of ADC-2 content

(1) Coating: Antigen hIL-4Rα was diluted to 1 μg/mL with carbonate buffer, (Acro, ILR-H5221) and added to an ELISA plate at 100 μL/well, and the plate was incubated at 4 °C overnight (16-18 h).
(2) Plate washing: The ELISA plate was warmed to room temperature and washed 3 times with 0.05% PBST (PBS + 0.05% Tween20) buffer (300 μL/well).
(3) Blocking: PBS containing 3% BSA was added at 300 μL/well, and the plate was incubated at room temperature on a shaker at 400 rpm for 2 h.
(4) Plate washing: The plate was washed 3 times with 0.05% PBST (300 μL/well).
(5) Sample incubation: 100 μL of diluted serum, BALF, or lung tissue homogenate was added to the ELISA plate, and the plate was incubated at room temperature on a shaker at 400 rpm for 1.5 h.
(6) Plate washing: The plate was washed 3 times with 0.05% PBST (300 μL/well).
(7) Assay: Assay antibody (Invitrogen, A18811) was diluted 10,000-fold with assay buffer (0.05% PBST containing 0.5% BSA) and added to the ELISA plate at 100 μL/well, and the plate was incubated at room temperature on a shaker at 400 rpm for 1 h.
(8) Plate washing: The plate was washed 3 times with 0.05% PBST (300 μL/well).
(9) Color development: TMB substrate (Sera care, 5120-0080) was added at 100 μL/well, and the plate was incubated at room temperature for 15 min.
(10) Stop: Stop solution for substrate reactions (Solarbio, C1058) was added at 100 μL/well, and the absorbance at the wavelength of 620 nm was measured.

2.4. Measurement of free budesonide content

30 μL of serum, BALF, or lung tissue homogenate was taken, and 30.0 μL of internal standard working solution (5.00 ng/mL budesonide-D8) and 120 μL of acetonitrile were added. The mixture was vortexed for 1 min and centrifuged at 13,000 rpm and 4 °C for 5 min. 100 μL of the supernatant was added to a 96-well plate, and 100 μL of 0.3% FA dilution was added. The plate was incubated at room temperature on a shaker at 1,000 rpm for 15 min, and a 20 μL sample was taken for LC-MS/MS analysis (Shimadzu Japan, Shimadzu liquid chromatography system; AB Sciex, Triple Quad 6500$^+$ triple quadrupole tandem mass spectrometer).

3. Experimental results

The pharmacokinetic parameters of antibody-drug conjugate ADC-2 and free budesonide are shown in Table 6,

and the plasma concentration-time curves are shown in FIG. 4.

Table 6. The PK parameters of ADC-2 and free budesonide

| | ADC-2 | | | Free budesonide | | |
|---|---|---|---|---|---|---|
| **Pharmacokinetic parameter** | **Serum** | **BALF** | **Lung tissue** | **Serum** | **BALF** | **Lung tissue** |
| Peak concentration of drug ($C_{max}$, ng/mL) | 2427 | 31377 | 7293 | 0.7 | 1.1 | 1.88 |
| Time to peak of drug ($T_{max,}$ h) | 1.0 | 1.0 | 1.0 | 1.0 | 0.083 | 6.0 |
| Area under plasma concentration-time curve ($AUC_{last,}$ ng/mL · h) | 28526 | 451418 | 133942 | 3.43 | 0.046 | 53.72 |
| Half-life ($T_{1/2,}$ h) | 9.4 | 13.8 | 10.7 | / | / | / |
| Clearance rate (CL, g/h) | 3.41 | 0.20 | 0.72 | / | / | / |
| Apparent volume of distribution (Vz, g) | 46.46 | 4.06 | 11.17 | / | / | / |

The experimental results show that after antibody-drug conjugate ADC-2 was intratracheally administrated, the drug was mainly distributed in serum and BALF, and the serum exposure was low. The exposure of budesonide was low in all these tissues.

**Test Example 6:** *In Vivo* Efficacy of ADC-2 in OVA-Induced Mouse Asthma Model

1. Test substances

Antibody-drug conjugate ADC-2, 25G7-Fab, AZD1402-TAG, and budesonide.

2. Experimental method

2.1. Experimental animals

IL-4 hu/hu-IL-4Rα hu/hu double transgenic C57BL6/J mice, 8 weeks old, female, Cyagen (Suzhou) Biosciences Co., Ltd.

2.2. Mice administration procedure

The animals were randomly divided into a group of normal control animals and a group of molded animals. The mice were intraperitoneally injected with 100 μL of PBS solution containing 200 μg of OVA (Sigma, A5503) at days 0, 7, and 14, and sensitized with an equal volume of aluminum hydroxide as an adjuvant. From day 21 to day 27, the mice were each given one inhalation of a nebulized solution containing 3% OVA daily, with each inhalation lasting for 35 min. From day 21, the mice were intratracheally given a nebulized drug half an hour prior to the inhalation of the nebulized OVA solution. The specific administration regimen is shown in Table 7.

At day 28, after the mice were euthanized, the neck muscles of the mice were bluntly isolated to expose the trachea, and an incision was horizontally made in the trachea. A lavage syringe needle was slowly inserted, the normal saline pre-loaded, pre-cooled at 4 °C, was slowly injected into the lung tissue for lavage, and the bronchoalveolar lavage fluid was collected. Lavage was performed 3 times, with 300 μL of normal saline used for each of the first two lavages and 400 μL for the third. After 10 min of centrifugation at 1,000 rpm and 4 °C, the cell pellet was resuspended in 350 μL of pre-cooled normal saline, and leukocyte and differential cell counting was performed using a fully automatic blood analyzer.

Table 7. Administration regimen

| Group | Number of animals | Test protein | Dose (μg) | Route of administration | Frequency of administration |
|---|---|---|---|---|---|
| Normal control group | 6 | PBS | / | i.t. | QD |
| Model group | 6 | PBS | / | i.t. | QD |
| ADC-2 group | 8 | ADC-2 | 100 | i.t. | QD |

(continued)

| Group | Number of animals | Test protein | Dose (μg) | Route of administration | Frequency of administration |
|---|---|---|---|---|---|
| AZD1402-TAG group | 8 | AZD1402-TAG | 30 | i.t. | QD |
| 25G7-Fab group | 8 | 25G7-Fab | 100 | i.t. | QD |
| Budesonide group | 6 | Budesonide | 1 | i.t. | QD |

2.3. Experimental measures

Leukocyte and differential cell counts in BALF.

2.4. Statistical analysis

Unless otherwise stated, a comparison of the cell counts of two groups was conducted using one-way ANOVA test, with $p < 0.05$ defined as the presence of a statistically significant difference.

3. Experimental results

The experimental results are shown in FIG. 5. Compared to the model group, under the conditions of an equimolar dose, antibody-drug conjugate ADC-2 significantly reduced the number of leukocytes, eosinophils, and neutrophils in BALF, and ADC-2 also exhibited some inhibition of the number of monocytes. The efficacy of ADC-2 was better than that of AZD1402-TAG, and was significantly better than that of 25G7-Fab and budesonide. ADC-2 showed the synergistic effect of 25G7-Fab and budesonide in terms of efficacy.

**Test Example 7:** Dose Exploration of *In Vivo* Efficacy of ADC-2 in OVA-Induced Mouse Asthma Model

1. Test substances

Antibody-drug conjugate ADC-2, AZD1402-TAG, and Budesonide.

2. Experimental method

2.1. Experimental animals

IL-4 hu/hu-IL-4Rα hu/hu double transgenic C57BL6/J mice, 8 weeks old, female, Cyagen (Suzhou) Biosciences Co., Ltd.

2.2. Mice administration procedure

Drug administration and BALF sampling were performed as in Test Example 6. The specific administration regimen is shown in Table 8.

| Group | Number of animals | Test protein | Dose (μg) | Route of administration | Frequency of administration |
|---|---|---|---|---|---|
| Normal control group | 8 | PBS | / | i.t. | QD |
| Model group | 8 | PBS | / | i.t. | QD |
| ADC-2 low-dose group | 8 | ADC-2 | 10 | i.t. | QD |
| ADC-2 medium-dose group | 8 | ADC-2 | 30 | i.t. | QD |
| ADC-2 high-dose group | 8 | ADC-2 | 100 | i.t. | QD |
| AZD1402-TAG group | 8 | AZD1402-TAG | 30 | i.t. | QD |

(continued)

| Group | Number of animals | Test protein | Dose (μg) | Route of administration | Frequency of administration |
|---|---|---|---|---|---|
| Budesonide group | 6 | Budesonide | 1 mpk | i.t. | QD |

2.3. Experimental measures

Leukocyte and differential cell counts in BALF.

2.4. Statistical analysis

Unless otherwise stated, a comparison of the cell counts of two groups was conducted using one-way ANOVA test, with $p < 0.05$ defined as the presence of a statistically significant difference.

3. Experimental results

The experimental results are shown in FIG. 6. Compared to the model group, a mere 10 μg of ADC-2 significantly reduced the number of eosinophils in bronchoalveolar lavage fluid, and the effect was comparable to that of AZD1402-TAG and was slightly better than that of a dose of budesonide that is far higher than the clinically equivalent dose. In addition, 10 μg of ADC-2 exhibited a very significant capacity to inhibit the number of leukocytes, and also exhibited some inhibition of the number of monocytes and neutrophils. These results indicate that a mere 1/10 molar dose of ADC-2 can achieve an effect that is comparable to that of AZD1402-TAG.

## Claims

**1.** An antibody-drug conjugate represented by formula (I),

$$Ab\text{-}(L\text{-}D)_k \qquad (I)$$

wherein Ab is an anti-IL-4R antibody or an antigen-binding fragment thereof;
L is a linker covalently linking Ab to D, and k is 1 to 20;
D is a glucocorticoid or a residue thereof, and the glucocorticoid is selected from the group consisting of budesonide and ciclesonide.

**2.** The antibody-drug conjugate according to claim 1, wherein D is:

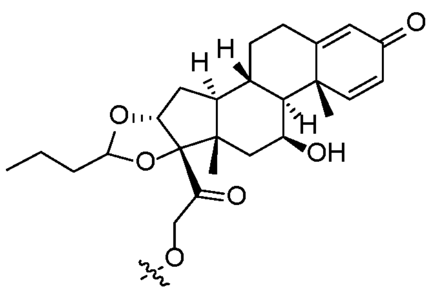

**3.** The antibody-drug conjugate according to claim 1 or 2, wherein the anti-IL-4R antibody or the antigen-binding fragment thereof comprises a combination selected from the group consisting of any one of the following (I) to (IV):

(I) a heavy chain variable region, comprising a HCDR1, a HCDR2, and a HCDR3 set forth in SEQ ID NO: 3, SEQ ID NO: 4, and SEQ ID NO: 5, respectively; and
a light chain variable region, comprising a LCDR1, a LCDR2, and a LCDR3 set forth in SEQ ID NO: 38, SEQ ID NO: 7, and SEQ ID NO: 40, respectively;

(II) a heavy chain variable region, comprising a HCDR1, a HCDR2, and a HCDR3 set forth in SEQ ID NO: 11, SEQ ID NO: 12, and SEQ ID NO: 13, respectively; and
a light chain variable region, comprising a LCDR1, a LCDR2, and a LCDR3 set forth in SEQ ID NO: 14, SEQ ID NO: 15, and SEQ ID NO: 16, respectively;
(III) a heavy chain variable region, comprising a HCDR1, a HCDR2, and a HCDR3 set forth in SEQ ID NO: 3, SEQ ID NO: 4, and SEQ ID NO: 5, respectively; and
a light chain variable region, comprising a LCDR1, a LCDR2, and a LCDR3 set forth in SEQ ID NO: 6, SEQ ID NO: 7, and SEQ ID NO: 8, respectively; and
(IV) a heavy chain variable region, comprising a HCDR1, a HCDR2, and a HCDR3 set forth in SEQ ID NO: 3, SEQ ID NO: 4, and SEQ ID NO: 5, respectively; and
a light chain variable region, comprising a LCDR1, a LCDR2, and a LCDR3 set forth in SEQ ID NO: 42, SEQ ID NO: 39, and SEQ ID NO: 8, respectively.

**4.** The antibody-drug conjugate according to any one of claims 1-3, wherein the anti-IL-4R antibody is selected from the group consisting of any one of the following: a murine antibody, a chimeric antibody, a fully human antibody, and a humanized antibody;

preferably, the anti-IL-4R antibody or the antigen-binding fragment thereof comprises a FR derived from a human germline light chain IGKV3-11*01 or a FR having at least 95% identity thereto; the FR preferably comprises a back mutation, and the back mutation is selected from the group consisting of one or more of 46P, 47W, and 71Y; and/or comprises a FR derived from a human germline heavy chain IGHV3-48*01 or a FR having at least 95% identity thereto; the FR preferably comprises a back mutation, and the back mutation is selected from the group consisting of one or more of 49A, 67S, and 93T;
or preferably, the anti-IL-4R antibody or the antigen-binding fragment thereof comprises a FR derived from a human germline light chain IGKV2D-29*01 or a FR having at least 95% identity thereto; the FR preferably comprises a back mutation, and the back mutation is selected from the group consisting of 4L and/or 58I; and/or comprises a FR derived from a human germline heavy chain IGHV1-2*02 or a FR having at least 95% identity thereto; the FR preferably comprises a back mutation, and the back mutation is selected from the group consisting of one or more of 69L, 71I, 73K, and 94K.

**5.** The antibody-drug conjugate according to any one of claims 1-4, wherein the anti-IL-4R antibody or the antigen-binding fragment thereof comprises a combination selected from the group consisting of any one of the following (i) to (vi):

(i) a heavy chain variable region, comprising the amino acid sequence set forth in SEQ ID NO: 43 or an amino acid sequence having at least 90%, 95%, 98%, or 99% identity to SEQ ID NO: 43; and
a light chain variable region, comprising the amino acid sequence set forth in SEQ ID NO: 37 or an amino acid sequence having at least 90%, 95%, 98%, or 99% identity to SEQ ID NO: 37;
(ii) a heavy chain variable region, comprising the amino acid sequence set forth in SEQ ID NO: 9 or an amino acid sequence having at least 90%, 95%, 98%, or 99% identity to SEQ ID NO: 9; and
a light chain variable region, comprising the amino acid sequence set forth in SEQ ID NO: 10 or an amino acid sequence having at least 90%, 95%, 98%, or 99% identity to SEQ ID NO: 10;
(iii) a heavy chain variable region, comprising the amino acid sequence set forth in SEQ ID NO: 1 or an amino acid sequence having at least 90%, 95%, 98%, or 99% identity to SEQ ID NO: 1; and
a light chain variable region, comprising the amino acid sequence set forth in SEQ ID NO: 2 or an amino acid sequence having at least 90%, 95%, 98%, or 99% identity to SEQ ID NO: 2;
(iv) a heavy chain variable region, comprising the amino acid sequence set forth in SEQ ID NO: 43 or an amino acid sequence having at least 90%, 95%, 98%, or 99% identity to SEQ ID NO: 43; and a light chain variable region, comprising the amino acid sequence set forth in SEQ ID NO: 41 or an amino acid sequence having at least 90%, 95%, 98%, or 99% identity to SEQ ID NO: 41;
(v) a heavy chain variable region, comprising a sequence set forth in any one of SEQ ID NOs: 25-27 or a sequence having at least 90%, 95%, 98%, or 99% identity to any one of SEQ ID NOs: 25-27; and
a light chain variable region, comprising a sequence set forth in any one of SEQ ID NOs: 28-30 or a sequence having at least 90%, 95%, 98%, or 99% identity to any one of SEQ ID NOs: 28-30; and
(vi) a heavy chain variable region, comprising a sequence set forth in any one of SEQ ID NOs: 31-33 or a sequence having at least 90%, 95%, 98%, or 99% identity to any one of SEQ ID NOs: 31-33; and
a light chain variable region, comprising a sequence set forth in any one of SEQ ID NOs: 34-36 or a sequence having at least 90%, 95%, 98%, or 99% identity to any one of SEQ ID NOs: 34-36; preferably, the anti-IL-4R

antibody or the antigen-binding fragment thereof comprises a combination selected from the group consisting of any one of the following (vii) to (xii):
(vii) a heavy chain variable region the sequence of which is set forth in SEQ ID NO: 43, and a light chain variable region the sequence of which is set forth in SEQ ID NO: 37;
(viii) a heavy chain variable region the sequence of which is set forth in SEQ ID NO: 9, and a light chain variable region the sequence of which is set forth in SEQ ID NO: 10;
(ix) a heavy chain variable region the sequence of which is set forth in SEQ ID NO: 1, and a light chain variable region the sequence of which is set forth in SEQ ID NO: 2;
(x) a heavy chain variable region the sequence of which is set forth in SEQ ID NO: 43, and a light chain variable region the sequence of which is set forth in SEQ ID NO: 41;
(xi) a heavy chain variable region the sequence of which is set forth in any one of SEQ ID NOs: 25-27, and a light chain variable region the sequence of which is set forth in any one of SEQ ID NOs: 28-30; and
(xii) a heavy chain variable region the sequence of which is set forth in any one of SEQ ID NOs: 31-33, and a light chain variable region the sequence of which is set forth in any one of SEQ ID NOs: 34-36.

**6.** The antibody-drug conjugate according to any one of claims 1-5, wherein the anti-IL-4R antibody or the antigen-binding fragment thereof comprises a heavy chain constant region, and the heavy chain constant region is a heavy chain constant region of human IgG1, IgG2, IgG3, or IgG4, or a variant thereof; and/or
the antigen-binding fragment is a Fab, Fv, scFv, Fab', or F(ab')2.

**7.** The antibody-drug conjugate according to any one of claims 1-6, wherein the anti-IL-4R antibody or the antigen-binding fragment thereof comprises a combination selected from the group consisting of any one of (a)-(d):

(a) a heavy chain, comprising the amino acid sequence set forth in SEQ ID NO: 44 or an amino acid sequence having at least 90%, 95%, 98%, or 99% identity to SEQ ID NO: 44; and
a light chain, comprising the amino acid sequence set forth in SEQ ID NO: 45 or an amino acid sequence having at least 90%, 95%, 98%, or 99% identity to SEQ ID NO: 45;
(b) a heavy chain, comprising the amino acid sequence set forth in SEQ ID NO: 19 or an amino acid sequence having at least 90%, 95%, 98%, or 99% identity to SEQ ID NO: 19; and
a light chain, comprising the amino acid sequence set forth in SEQ ID NO: 20 or an amino acid sequence having at least 90%, 95%, 98%, or 99% identity to SEQ ID NO: 20;
(c) a heavy chain, comprising the amino acid sequence set forth in SEQ ID NO: 17 or an amino acid sequence having at least 90%, 95%, 98%, or 99% identity to SEQ ID NO: 17; and
a light chain, comprising the amino acid sequence set forth in SEQ ID NO: 18 or an amino acid sequence having at least 90%, 95%, 98%, or 99% identity to SEQ ID NO: 18; and
(d) a heavy chain, comprising the amino acid sequence set forth in SEQ ID NO: 44 or an amino acid sequence having at least 90%, 95%, 98%, or 99% identity to SEQ ID NO: 44; and
a light chain, comprising the amino acid sequence set forth in SEQ ID NO: 46 or an amino acid sequence having at least 90%, 95%, 98%, or 99% identity to SEQ ID NO: 46;

more preferably, the anti-IL-4R antibody or the antigen-binding fragment thereof comprises a combination selected from the group consisting of any one of (e)-(i):

(e) a heavy chain the sequence of which is set forth in SEQ ID NO: 44, and a light chain the sequence of which is set forth in SEQ ID NO: 45;
(f) a heavy chain the sequence of which is set forth in SEQ ID NO: 47, and a light chain the sequence of which is set forth in SEQ ID NO: 45;
(g) a heavy chain the sequence of which is set forth in SEQ ID NO: 17, and a light chain the sequence of which is set forth in SEQ ID NO: 18;
(h) a heavy chain the sequence of which is set forth in SEQ ID NO: 19, and a light chain the sequence of which is set forth in SEQ ID NO: 20; and
(i) a heavy chain the sequence of which is set forth in SEQ ID NO: 44, and a light chain the sequence of which is set forth in SEQ ID NO: 46.

**8.** The antibody-drug conjugate according to any one of claims 1-6, wherein the anti-IL-4R antibody or the antigen-binding fragment thereof comprises a combination selected from the group consisting of any one of (a)-(f):

(a) a heavy chain, comprising the amino acid sequence set forth in SEQ ID NO: 48 or an amino acid sequence

having at least 90%, 95%, 98%, or 99% identity to SEQ ID NO: 48; and
a light chain, comprising the amino acid sequence set forth in SEQ ID NO: 18 or an amino acid sequence having at least 90%, 95%, 98%, or 99% identity to SEQ ID NO: 18;
(b) a heavy chain, comprising the amino acid sequence set forth in SEQ ID NO: 49 or an amino acid sequence having at least 90%, 95%, 98%, or 99% identity to SEQ ID NO: 49; and
a light chain, comprising the amino acid sequence set forth in SEQ ID NO: 18 or an amino acid sequence having at least 90%, 95%, 98%, or 99% identity to SEQ ID NO: 18;
(c) a heavy chain, comprising the amino acid sequence set forth in SEQ ID NO: 50 or an amino acid sequence having at least 90%, 95%, 98%, or 99% identity to SEQ ID NO: 50; and
a light chain, comprising the amino acid sequence set forth in SEQ ID NO: 45 or an amino acid sequence having at least 90%, 95%, 98%, or 99% identity to SEQ ID NO: 45;
(d) a heavy chain, comprising the amino acid sequence set forth in SEQ ID NO: 50 or an amino acid sequence having at least 90%, 95%, 98%, or 99% identity to SEQ ID NO: 50; and
a light chain, comprising the amino acid sequence set forth in SEQ ID NO: 46 or an amino acid sequence having at least 90%, 95%, 98%, or 99% identity to SEQ ID NO: 46;
(e) a heavy chain, comprising the amino acid sequence set forth in SEQ ID NO: 51 or an amino acid sequence having at least 90%, 95%, 98%, or 99% identity to SEQ ID NO: 51; and
a light chain, comprising the amino acid sequence set forth in SEQ ID NO: 45 or an amino acid sequence having at least 90%, 95%, 98%, or 99% identity to SEQ ID NO: 45; and
(f) a heavy chain, comprising the amino acid sequence set forth in SEQ ID NO: 51 or an amino acid sequence having at least 90%, 95%, 98%, or 99% identity to SEQ ID NO: 51; and
a light chain, comprising the amino acid sequence set forth in SEQ ID NO: 46 or an amino acid sequence having at least 90%, 95%, 98%, or 99% identity to SEQ ID NO: 46;

more preferably, the anti-IL-4R antibody or the antigen-binding fragment thereof comprises a combination selected from the group consisting of any one of (h)-(m):

(h) a heavy chain the sequence of which is set forth in SEQ ID NO: 48, and a light chain the sequence of which is set forth in SEQ ID NO: 18;
(i) a heavy chain the sequence of which is set forth in SEQ ID NO: 49, and a light chain the sequence of which is set forth in SEQ ID NO: 18;
(j) a heavy chain the sequence of which is set forth in SEQ ID NO: 50, and a light chain the sequence of which is set forth in SEQ ID NO: 45;
(k) a heavy chain the sequence of which is set forth in SEQ ID NO: 50, and a light chain the sequence of which is set forth in SEQ ID NO: 46;
(l) a heavy chain the sequence of which is set forth in SEQ ID NO: 51, and a light chain the sequence of which is set forth in SEQ ID NO: 45; and
(m) a heavy chain the sequence of which is set forth in SEQ ID NO: 51, and a light chain the sequence of which is set forth in SEQ ID NO: 46.

**9.** The antibody-drug conjugate according to any one of claims 1-8, wherein L-D is a chemical moiety represented by the following formula:

-Str-Pep-Sp-D,

wherein Str is a stretcher unit covalently linked to Ab;
Sp is a spacer unit;
Pep is selected from the group consisting of an amino acid unit, a disulfide moiety, a sulfonamide moiety, and the following non-peptidic chemical moiety:

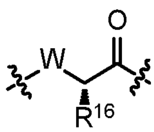 , 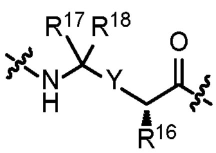 , or 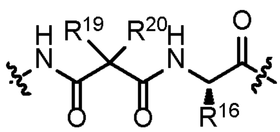 ,

wherein W is -NH-heterocycloalkylene- or heterocycloalkyl; Y is heteroarylene, arylene, -C(O)$C_{1-6}$ alkylene, $C_{2-6}$ alkenylene, $C_{1-6}$ alkylene, or -$C_{1-6}$ alkylene-NH-; each $R^{16}$ is independently selected from the group consisting of

$C_{1-6}$ alkyl, $C_{2-6}$ alkenyl, -($C_{1-6}$ alkylene)NHC(NH)$NH_2$, and -($C_{1-6}$ alkylene)NHC(O)$NH_2$; $R^{17}$ and $R^{18}$ are each independently selected from the group consisting of hydrogen, $C_{1-6}$ alkyl, $C_{2-6}$ alkenyl, aryl, and heteroaryl, or $R^{17}$ and $R^{18}$ may form $C_{3-6}$ cycloalkyl together; $R^{19}$ and $R^{20}$ are each independently selected from the group consisting of $C_{1-6}$ alkyl, $C_{2-6}$ alkenyl, aryl, heteroaryl, and ($C_{1-6}$ alkyl)$OCH_2$-, or $R^{19}$ and $R^{20}$ may form a $C_{3-6}$ cycloalkyl ring together.

**10.** The antibody-drug conjugate according to claim 9, wherein Str is selected from the group consisting of chemical moieties represented by the following formula:

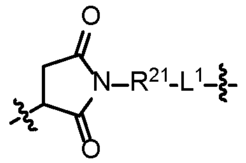 or 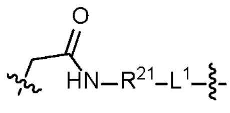,

wherein $R^{21}$ is selected from the group consisting of $-W_1-C(O)-$, $-C(O)-W_1-C(O)-$, $-(CH_2CH_2O)_{p1}C(O)-$, $-(CH_2CH_2O)_{p1}CH_2C(O)-$, and $-(CH_2CH_2O)_{p1}CH_2CH_2C(O)-$, wherein $W_1$ is selected from the group consisting of $C_{1-6}$ alkylene, $C_{1-6}$ alkylene-cycloalkyl, and a linear heteroalkyl of 1 to 8 atoms; the heteroalkyl contains 1 to 3 heteroatoms selected from the group consisting of N, O, and S, wherein the alkyl, cycloalkyl, and linear heteroalkyl are each independently optionally substituted with one or more groups selected from the group consisting of halogen, deuterium, hydroxy, cyano, amino, $C_{1-6}$ alkyl, $C_{1-6}$ haloalkyl, deuterated $C_{1-6}$ alkyl, $C_{1-6}$ alkoxy, and $C_{3-6}$ cycloalkyl; each p1 is independently an integer from 1 to 20;

$L^1$ is selected from the group consisting of $-NR^{22}-(CH_2CH_2O)_{p2}CH_2CH_2C(O)-$, $-NR^{22}-(CH_2CH_2O)_{p2}CH_2C(O)-$, $-S-(CH_2)_{p2}C(O)-$, $-(CH_2)_{p2}C(O)-$, and a single bond, preferably from the group consisting of a single bond; each p2 is independently an integer from 1 to 20; $R^{22}$ is selected from the group consisting of a hydrogen atom, $C_{1-6}$ alkyl, $C_{1-6}$ haloalkyl, and deuterated $C_{1-6}$ alkyl.

**11.** The antibody-drug conjugate according to claim 10, wherein $R^{21}$ is selected from the group consisting of $-C_{1-6}$ alkylene C(O)-, $-(CH_2-CH_2O)_2C(O)-$, $-(CH_2-CH_2O)_2CH_2C(O)-$, $-(CH_2-CH_2O)_2CH_2CH_2C(O)-$, $-(CH_2-CH_2O)_3C(O)-$, and $-(CH_2-CH_2O)_4C(O)-$.

**12.** The antibody-drug conjugate according to claim 10 or 11, wherein the Pep is selected from the group consisting of valine-citrulline, alanine-alanine-asparagine, glycine-glycine-lysine, valine-lysine (Val-lys), valine-alanine, valine-phenylalanine, and glycine-glycine-phenylalanine-glycine.

**13.** The antibody-drug conjugate according to any one of claims 10-12, wherein Sp is selected from the group consisting of $-NHCH_2-$, $-NHCH_2CH_2-$, $-NHCH_2CH_2CH_2-$, $-NHCH_2-O-CH_2-$, $-NHCH_2CH_2-O-CH_2-$, $-NH(CH_2)_3-C(O)-$, $-NHCH_2-O-CH_2-C(O)-$, $-NH(CH_2)_2-O-CH_2-C(O)-$,

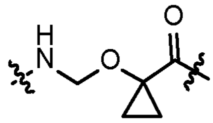,

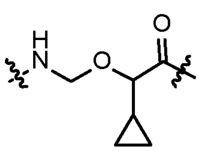, 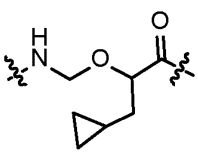, 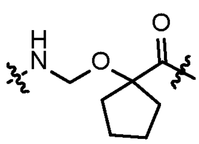, 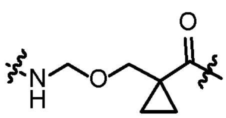, 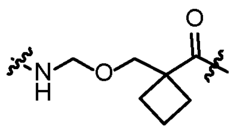,

and

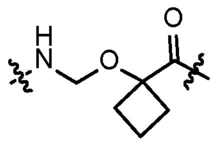

**14.** The antibody-drug conjugate according to any one of claims 1-13, wherein the linker L in the antibody-drug conjugate comprises: maleimide-$(PEG)_4$-$CH_2CH_2C(O)$-Gly-Gly-Phe-Gly, maleimide-$(PEG)_2$-Val-Cit, maleimide-$(PEG)_6$-Val-Cit, maleimide-$(PEG)_8$-Val-Cit, maleimide-$(PEG)_4$-$CH_2CH_2C(O)$-Val-lys, maleimide-$(CH_2)_5$-Val-Cit, maleimide-$(CH_2)_5$-Val-lys, maleimide-$(CH_2)_5$-Gly-Gly-Phe-Gly, maleimide-$(PEG)_4$-$CH_2CH_2C(O)$-Gly-Gly-Phe-Gly, maleimide-$(PEG)_2$-Ala-Ala-Asn, maleimide-$(PEG)_6$-Ala-Ala-Asn, maleimide-$(PEG)_8$-Ala-Ala-Asn, maleimide-$(PEG)_4$-triazole-$(PEG)_3$-sulfonamide, maleimide-$(PEG)_2$-$CH_2CH_2C(O)$-Val-lys, maleimide-$(PEG)_4$-triazole-$(PEG)_3$-sulfonamide, or Mal-$(PEG)_4$-triazole-$(PEG)_3$-disulfide.

**15.** The antibody-drug conjugate according to claim 1, being represented by the following formula:

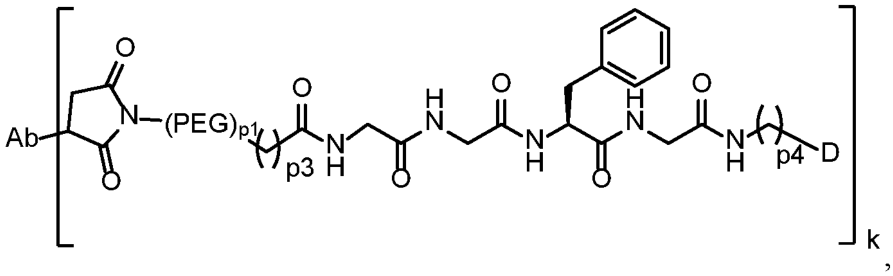

wherein k is selected from the group consisting of 1 to 10, p1 is selected from the group consisting of 2, 4, 6, and 8, and p3 and p4 are each independently selected from the group consisting of 0, 1, and 2; or

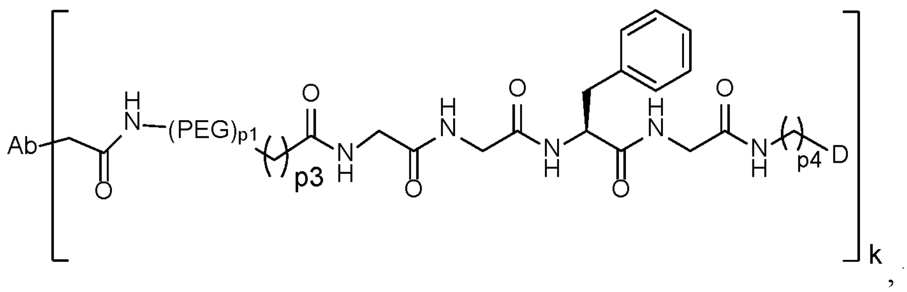

wherein k is selected from the group consisting of 1 to 10, p1 is selected from the group consisting of 2, 4, 6, and 8, and p3 and p4 are each independently selected from the group consisting of 0, 1, and 2.

**16.** The antibody-drug conjugate according to claim 15, being selected from the group consisting of:

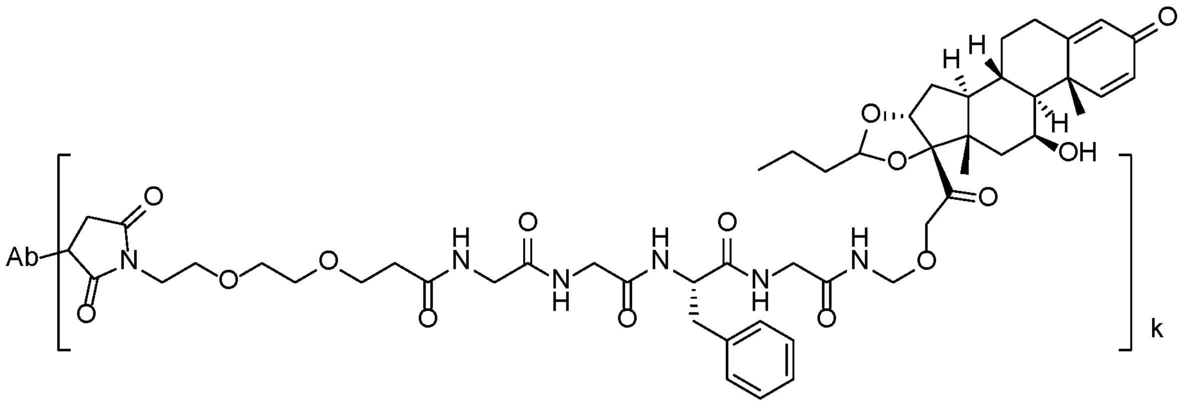

,

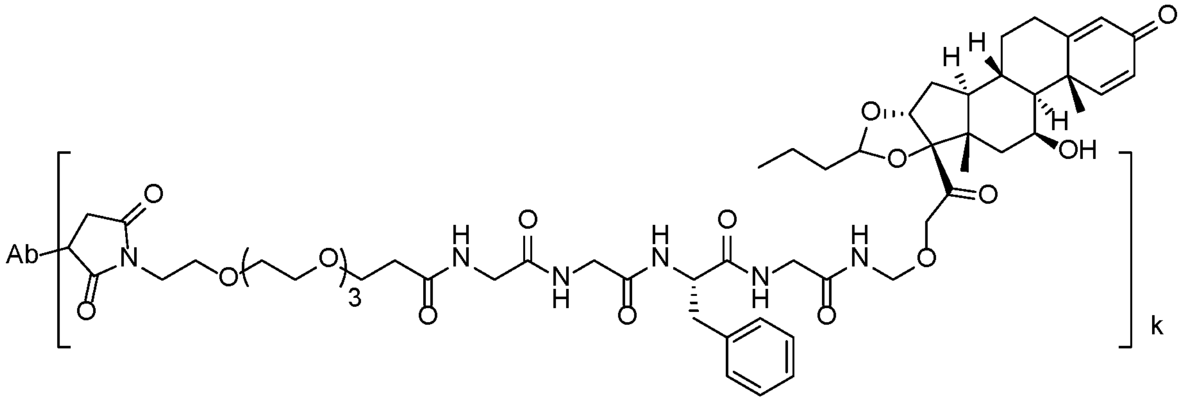

,

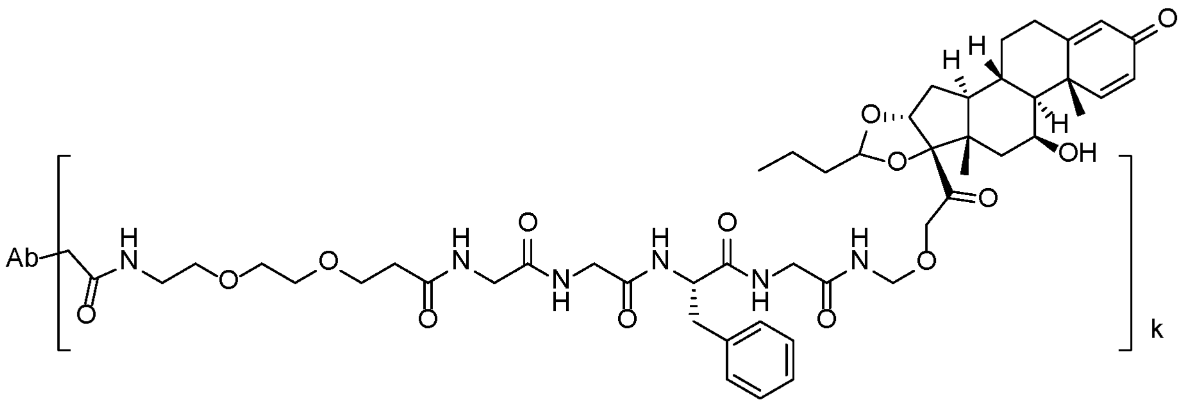

,

and

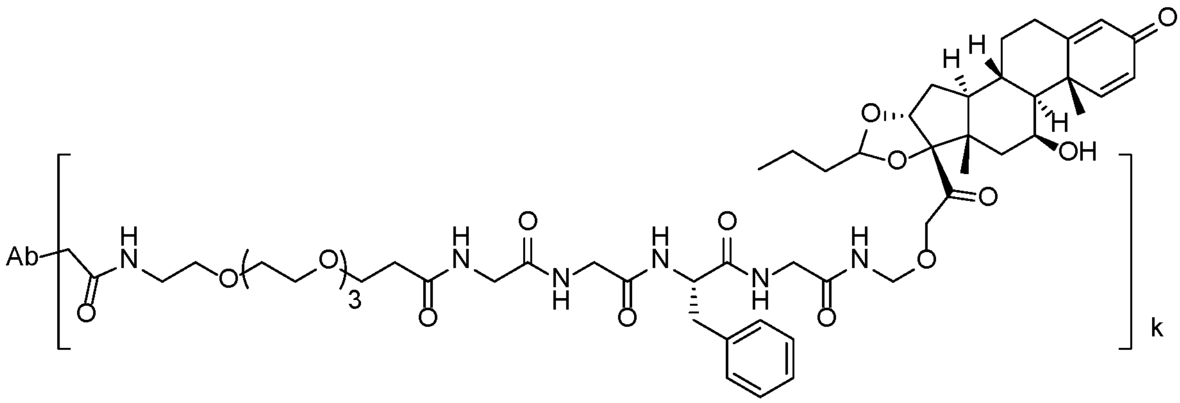

,

wherein k is selected from the group consisting of 1 to 10.

**17.** A compound represented by formula II-A or a pharmaceutically acceptable salt thereof,

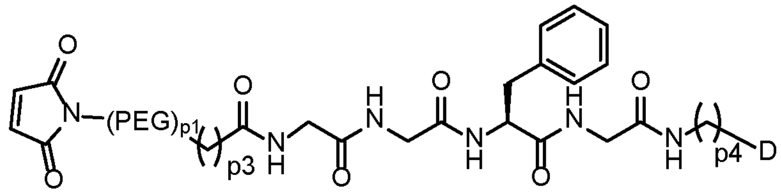

II-A

wherein p1 is selected from the group consisting of 2, 4, 6, and 8, p3 and p4 are each independently selected from the group consisting of 0, 1, and 2, and D is budesonide or ciclesonide.

**18.** A compound represented by formula II-B or a pharmaceutically acceptable salt thereof,

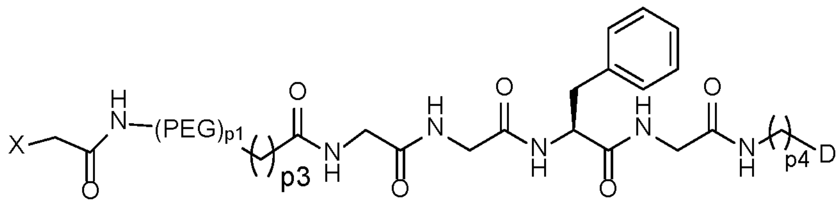

II-B

wherein X is halogen, preferably bromine, p1 is selected from the group consisting of 2, 4, 6, and 8, p3 and p4 are each independently selected from the group consisting of 0, 1, and 2, and D is budesonide or ciclesonide.

**19.** A pharmaceutical composition, comprising the antibody-drug conjugate according to any one of claims 1-16 and a pharmaceutically acceptable excipient.

**20.** An inhalable pharmaceutical composition, comprising an antibody-drug conjugate represented by formula (I) and a pharmaceutically acceptable carrier,

$$Ab\text{-}(L\text{-}D)_k \qquad (I)$$

wherein Ab is an anti-IL-4R antibody or an antigen-binding fragment thereof, preferably an anti-IL-4R Fab fragment;

L is a linker covalently linking Ab to D, and k is 1 to 20;
D is a glucocorticoid or a residue thereof.

**21.** Use of the antibody-drug conjugate according to any one of claims 1-16 or the pharmaceutical composition according to claim 19 or 20 in the preparation of a medicament for treating or preventing an immune disease or disorder, wherein the disease or disorder is preferably: asthma, nasal polyps, chronic sinusitis, allergic skin disorders, eosinophilic esophagitis, chronic obstructive pulmonary disease, allergic rhinitis, arthritis, inflammatory diseases, allergic reaction, autoimmune lymphoproliferative syndrome, autoimmune hemolytic anemia, Barrett's esophagus, autoimmune uveitis, tuberculosis, and renal disease, and is more preferably, asthma or allergic skin disorders.

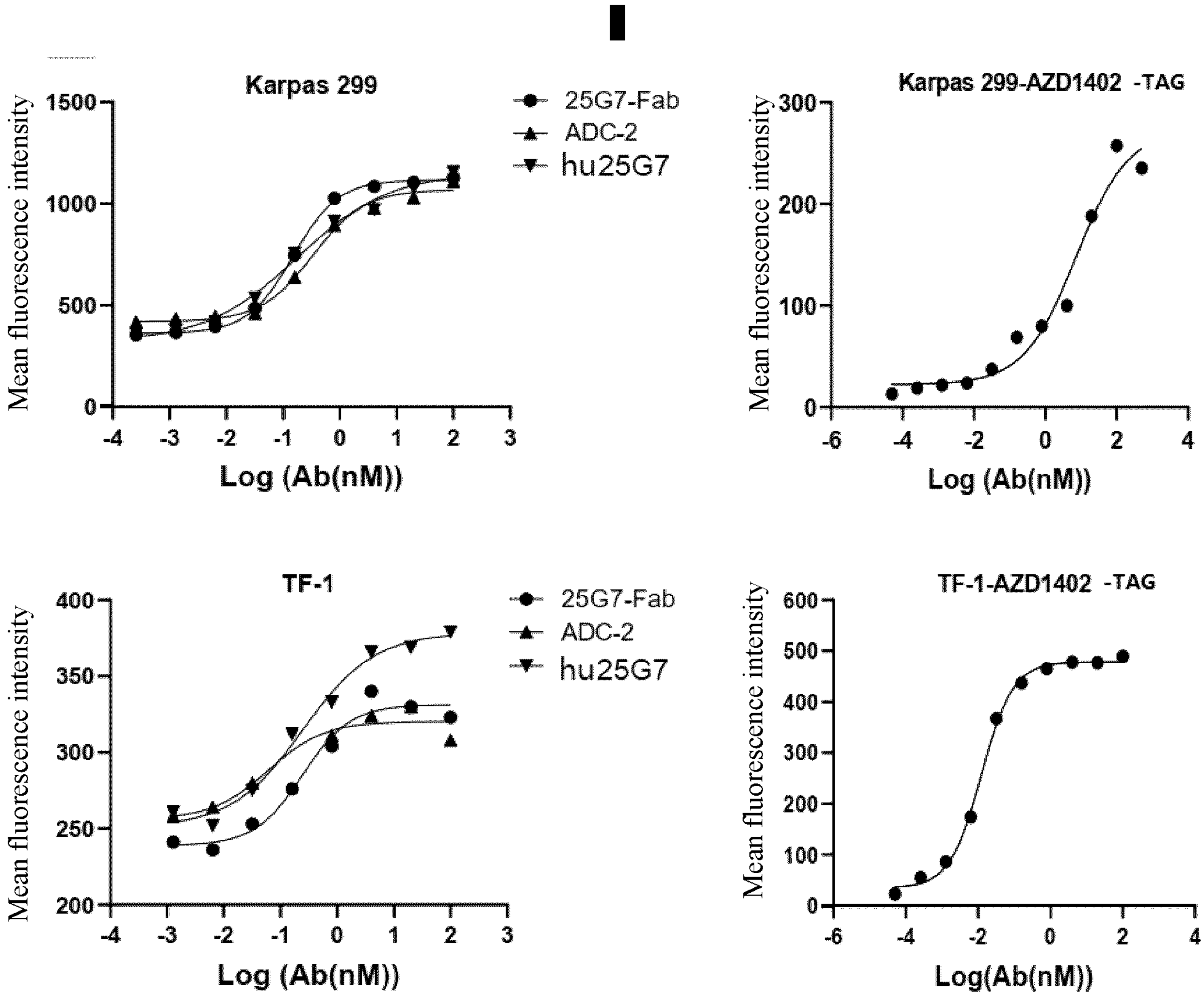
Karpas 299
25G7-Fab
ADC-2
hu25G7
Mean fluorescence intensity
Log (Ab(nM))
Karpas 299-AZD1402 -TAG
Mean fluorescence intensity
Log (Ab(nM))
TF-1
25G7-Fab
ADC-2
hu25G7
Mean fluorescence intensity
Log (Ab(nM))
TF-1-AZD1402 -TAG
Mean fluorescence intensity
Log(Ab(nM))

FIG. 1

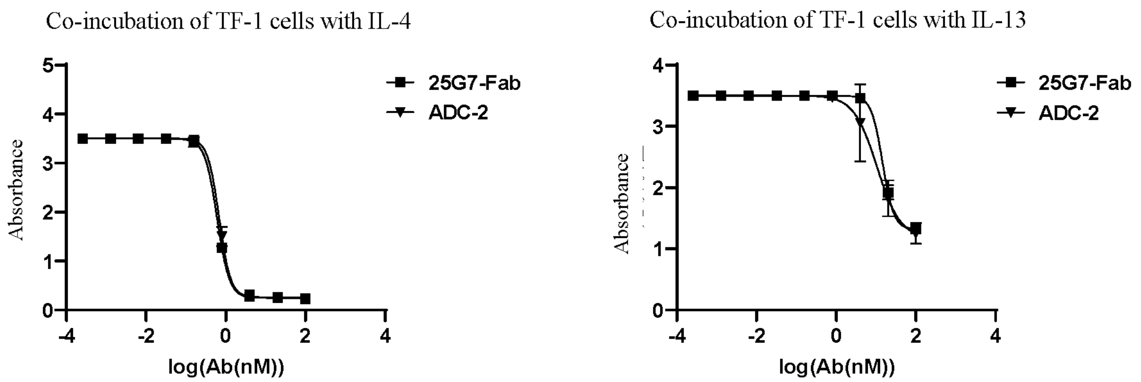
Co-incubation of TF-1 cells with IL-4
Co-incubation of TF-1 cells with IL-13
Absorbance
log(Ab(nM))
25G7-Fab
ADC-2

FIG. 2

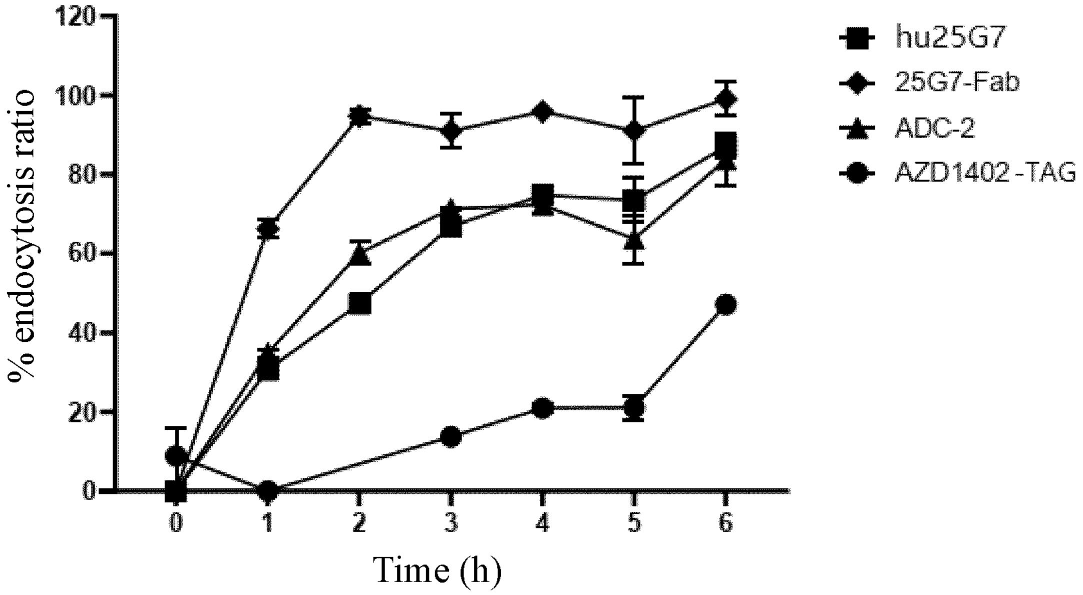
% endocytosis ratio
Time (h)
hu25G7
25G7-Fab
ADC-2
AZD1402-TAG

FIG. 3

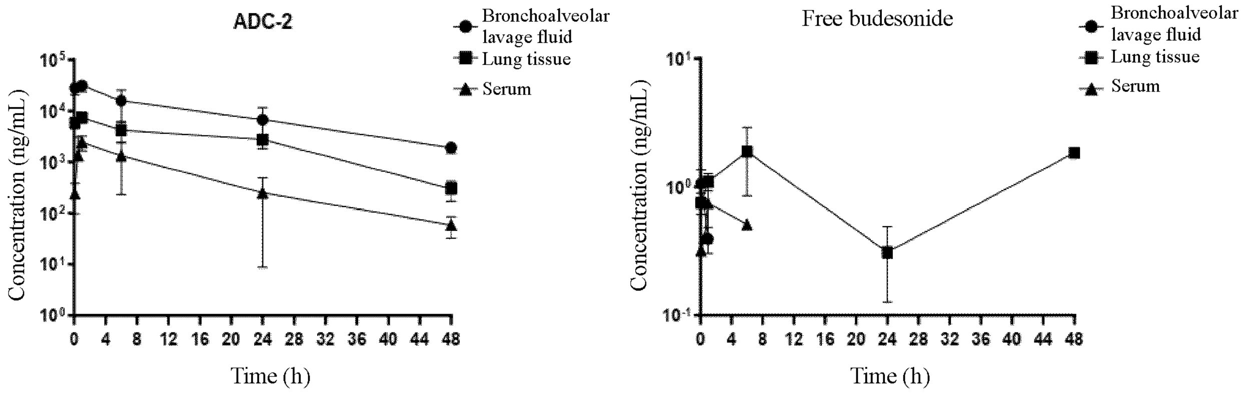
ADC-2
Bronchoalveolar lavage fluid
Lung tissue
Serum
Concentration (ng/mL)
Time (h)
Free budesonide
Bronchoalveolar lavage fluid
Lung tissue
Serum
Concentration (ng/mL)
Time (h)

FIG. 4

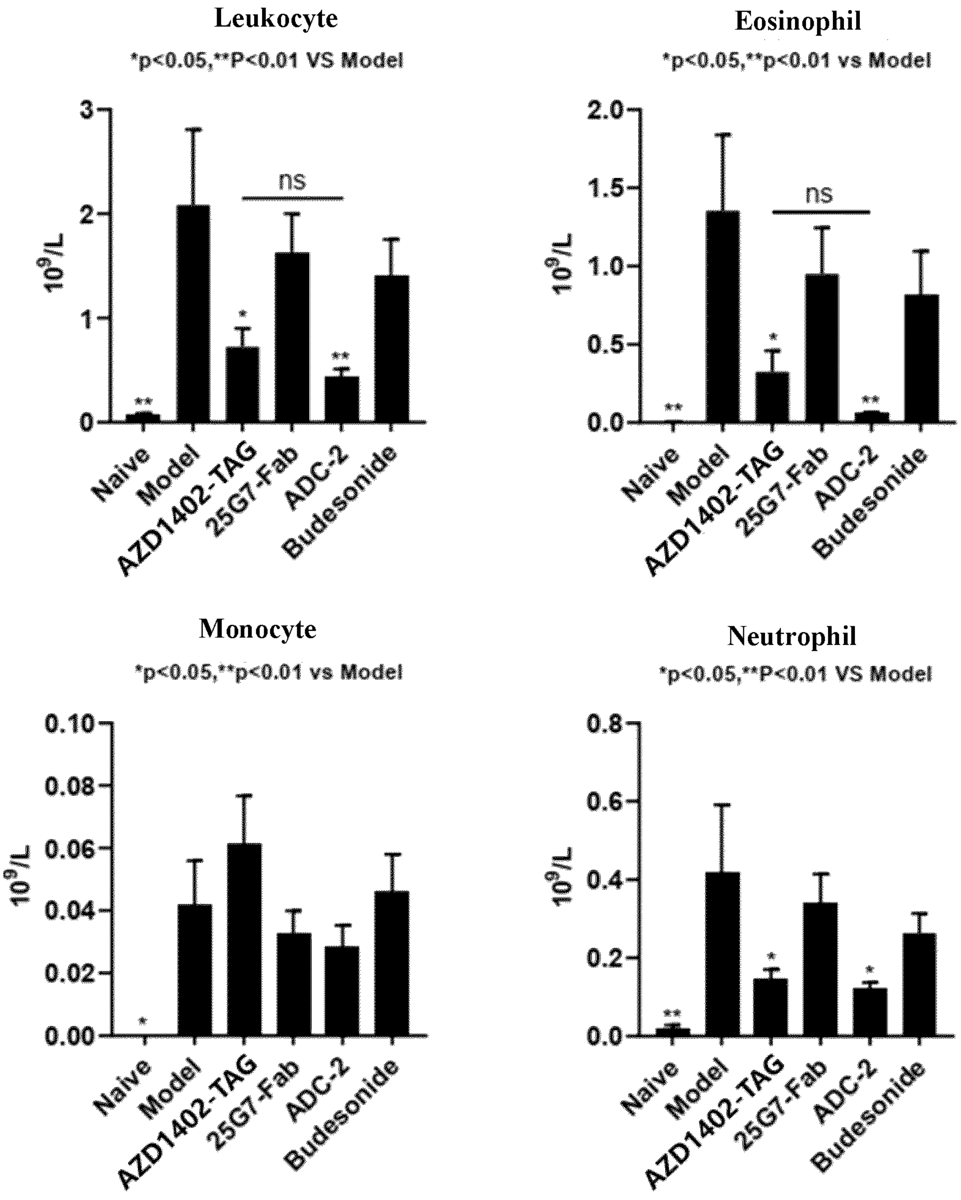
Leukocyte
*p<0.05,**P<0.01 VS Model
ns
10⁹/L
Naive
Model
AZD1402-TAG
25G7-Fab
ADC-2
Budesonide
Eosinophil
*p<0.05,**p<0.01 vs Model
ns
10⁹/L
Monocyte
*p<0.05,**p<0.01 vs Model
10⁹/L
Neutrophil
*p<0.05,**P<0.01 VS Model
10⁹/L

FIG. 5

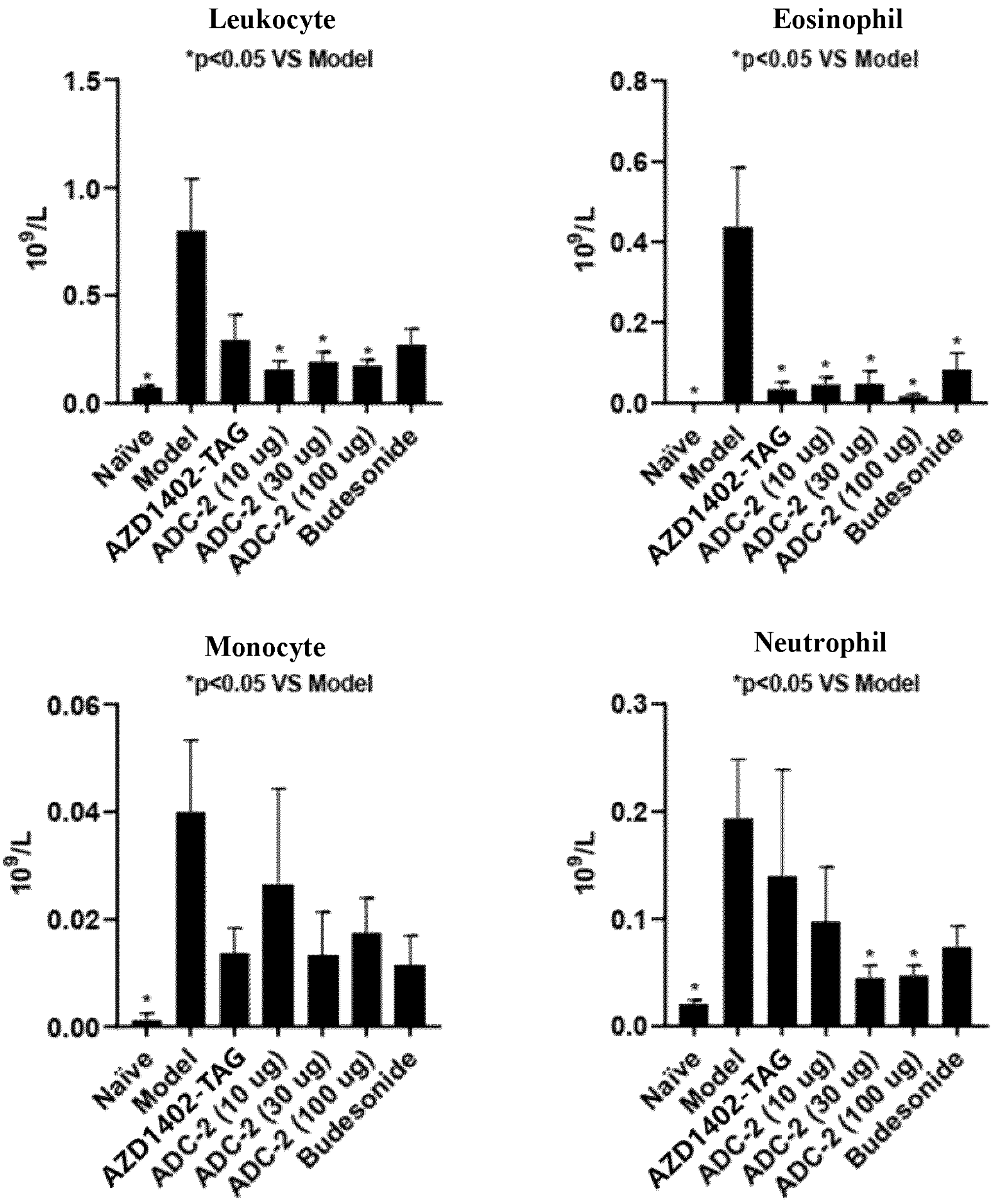
Leukocyte
*p<0.05 VS Model
Eosinophil
*p<0.05 VS Model
Monocyte
*p<0.05 VS Model
Neutrophil
*p<0.05 VS Model
10^9/L
Naïve
Model
AZD1402-TAG
ADC-2 (10 ug)
ADC-2 (30 ug)
ADC-2 (100 ug)
Budesonide

FIG. 6

## INTERNATIONAL SEARCH REPORT

International application No.
**PCT/CN2023/073057**

**A. CLASSIFICATION OF SUBJECT MATTER**

A61K47/68 (2017.01)i; A61K31/58 (2006.01)i; C07K16/22(2006.01)i;A61P37/00(2006.01)i

According to International Patent Classification (IPC) or to both national classification and IPC

**B. FIELDS SEARCHED**

Minimum documentation searched (classification system followed by classification symbols)

IPC: A61K C07K A61P

Documentation searched other than minimum documentation to the extent that such documents are included in the fields searched

Electronic data base consulted during the international search (name of data base and, where practicable, search terms used)

DWPI; CNABS; WPABS; WPABSC; ENTXTC; CNTXT; CJFD; CNKI; VEN; PubMed; STN: 抗IL-4R, 白介素, PRLR, 抗体, 偶联, 缀合, 布地奈德, 环索奈德, 糖皮质激素, 序列检索, 结构检索, II-A结构, II-B结构, 哮喘, 过敏性皮肤病, ADC, Antibody, Drug, Conjugation, Asthma, Allergic dermatose?, Budesonide, Ciclesonide, Glucocorticoids

**C. DOCUMENTS CONSIDERED TO BE RELEVANT**

| Category* | Citation of document, with indication, where appropriate, of the relevant passages | Relevant to claim No. |
|---|---|---|
| PX | TW 202245796 A (SHANGHAI SENHUI MEDICINE CO., LTD.; SHANGHAI SHENGDI PHARMACEUTICAL CO., LTD.; JIANGSU HENGRUI MEDICINE CO., LTD.) 01 December 2022 (2022-12-01)<br>claims 1, 17, and 21-23, and example 7 | 1, 19-21 |
| PA | TW 202245796 A (SHANGHAI SENHUI MEDICINE CO., LTD.; SHANGHAI SHENGDI PHARMACEUTICAL CO., LTD.; JIANGSU HENGRUI MEDICINE CO., LTD.) 01 December 2022 (2022-12-01)<br>claims 1, 17, and 21-23, and example 7 | 2-18 |
| X | CN 113905767 A (REGENERON PHARMACEUTICALS INC.) 07 January 2022 (2022-01-07)<br>claims 72 and 114, description, page 13, the second-to-last structure and page 16, the third structure, and description, paragraphs [0175], [0185], [0316, [0318], and [0325] | 1, 2, 9, 19-21 |
| Y | CN 113905767 A (REGENERON PHARMACEUTICALS INC.) 07 January 2022 (2022-01-07)<br>claims 72 and 114, description, page 13, the second-to-last structure and page 16, the third structure, and description, paragraphs [0175], [0185], [0316, [0318], and [0325] | 10-18, |

✓ Further documents are listed in the continuation of Box C. ✓ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 April 2023** | **07 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/073057**

**C. DOCUMENTS CONSIDERED TO BE RELEVANT**

| Category* | Citation of document, with indication, where appropriate, of the relevant passages | Relevant to claim No. |
|---|---|---|
| A | CN 109476699 A (ABBVIE INC.) 15 March 2019 (2019-03-15) claims 1 and 14-21 | 1-21 |
| Y | WO 2021148003 A1 (SHANGHAI SENHUI MEDICINE CO., LTD.; SHANGHAI SHENGDI PHARMACEUTICAL CO., LTD.; SHANGHAI HENGRUI PHARMACEUTICAL CO., LTD.; JIANGSU HENGRUI MEDICINE CO., LTD.) 29 July 2021 (2021-07-29) claims 1 and 12-24 | 10-18 |
| A | US 2015056221 A1 (REGENERON PHARMA) 26 February 2015 (2015-02-26) embodiment 9 | 1-21 |
| A | US 2019167804 A1 (ABBVIE INC.) 06 June 2019 (2019-06-06) claims 1-11 | 1-21 |
| A | US 2020164085 A1 (MERCK SHARP & DOHME; AMBRX INC.) 28 May 2020 (2020-05-28) claims 1-3 and 17-21 | 1-21 |
| A | WO 2020038454 A1 (JIANGSU HENGRUI MEDICINE CO., LTD.; SHANGHAI HENGRUI PHARMACEUTICAL CO., LTD.) 27 February 2020 (2020-02-27) claims 1-9, 14, and 16-17 | 1-21 |

Form PCT/ISA/210 (second sheet) (July 2022)

**INTERNATIONAL SEARCH REPORT**

International application No.
**PCT/CN2023/073057**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/073057**

| Patent document cited in search report | | | Publication date (day/month/year) | Patent family member(s) | | | Publication date (day/month/year) |
|---|---|---|---|---|---|---|---|
| TW | 202245796 | A | 01 December 2022 | WO | 2022166779 | A1 | 11 August 2022 |
| CN | 113905767 | A | 07 January 2022 | SG | 11202107212 | QA | 29 July 2021 |
| | | | | CA | 3125998 | A1 | 16 July 2020 |
| | | | | KR | 20210114008 | A | 17 September 2021 |
| | | | | EP | 3908323 | A2 | 17 November 2021 |
| | | | | IL | 284673 | A | 31 August 2021 |
| | | | | JP | 2022517767 | A | 10 March 2022 |
| | | | | WO | 2020146541 | A2 | 16 July 2020 |
| | | | | WO | 2020146541 | A3 | 13 August 2020 |
| | | | | AU | 2020205662 | A1 | 22 July 2021 |
| | | | | MA | 53772 | A1 | 31 October 2022 |
| | | | | CL | 2021001790 | A1 | 01 July 2022 |
| | | | | CO | 2021010315 | A2 | 19 August 2021 |
| CN | 109476699 | A | 15 March 2019 | JP | 2021088582 | A | 10 June 2021 |
| | | | | DK | 3464318 | T3 | 28 June 2021 |
| | | | | RU | 2018145728 | A | 10 July 2020 |
| | | | | RU | 2018145728 | A3 | 04 August 2020 |
| | | | | RU | 2745748 | C2 | 31 March 2021 |
| | | | | CR | 20180594 | A | 29 July 2019 |
| | | | | PE | 20190622 | A1 | 26 April 2019 |
| | | | | KR | 20190014542 | A | 12 February 2019 |
| | | | | KR | 102435599 | B1 | 29 August 2022 |
| | | | | US | 2018126000 | A1 | 10 May 2018 |
| | | | | MY | 194619 | A | 07 December 2022 |
| | | | | PH | 12018502539 | A1 | 30 September 2019 |
| | | | | MX | 2018014927 | A | 09 April 2019 |
| | | | | US | 2022354959 | A1 | 10 November 2022 |
| | | | | AU | 2021269433 | A1 | 16 December 2021 |
| | | | | PL | 3464318 | T3 | 08 November 2021 |
| | | | | TW | 201801749 | A | 16 January 2018 |
| | | | | TWI | 728118 | B | 21 May 2021 |
| | | | | EP | 3901162 | A1 | 27 October 2021 |
| | | | | SG | 10202001787 | QA | 29 April 2020 |
| | | | | WO | 2017210471 | A1 | 07 December 2017 |
| | | | | ECSP | 18094857 | A | 31 January 2019 |
| | | | | BR | 112018074922 | A2 | 12 March 2019 |
| | | | | BR | 112018074922 | B1 | 25 October 2022 |
| | | | | KR | 20220119529 | A | 29 August 2022 |
| | | | | MX | 2020012562 | A | 09 February 2021 |
| | | | | DOP | 2018000261 | A | 31 December 2018 |
| | | | | IL | 263214 | A | 31 December 2018 |
| | | | | ZA | 201808623 | B | 28 August 2019 |
| | | | | CL | 2018003406 | A1 | 15 February 2019 |
| | | | | SG | 11201810678 | WA | 28 December 2018 |
| | | | | UY | 37269 | A | 02 January 2018 |
| | | | | LT | 3464318 | T | 25 June 2021 |
| | | | | JP | 2019524645 | A | 05 September 2019 |
| | | | | JP | 6843891 | B2 | 17 March 2021 |
| | | | | PT | 3464318 | T | 02 July 2021 |
| | | | | HUE | 054804 | T2 | 28 September 2021 |

Form PCT/ISA/210 (patent family annex) (July 2022)

**INTERNATIONAL SEARCH REPORT**
**Information on patent family members**

International application No.
**PCT/CN2023/073057**

| Patent document cited in search report | Publication date (day/month/year) | Patent family member(s) | Publication date (day/month/year) |
|---|---|---|---|
| | | CO 2018012996 A2 | 18 January 2019 |
| | | SI 3464318 T1 | 30 July 2021 |
| | | HRP 20210924 T1 | 03 September 2021 |
| | | MA 51586 A | 10 April 2019 |
| | | ES 2877548 T3 | 17 November 2021 |
| | | EP 3464318 A1 | 10 April 2019 |
| | | EP 3464318 B1 | 21 April 2021 |
| | | AU 2017274442 A1 | 13 December 2018 |
| | | AU 2017274442 B2 | 19 August 2021 |
| | | US 2020338208 A1 | 29 October 2020 |
| | | RS 62040 B1 | 30 July 2021 |
| | | US 2019262465 A1 | 29 August 2019 |
| | | US 10668167 B2 | 02 June 2020 |
| | | CA 3025377 A1 | 07 December 2017 |
| WO 2021148003 A1 | 29 July 2021 | AU 2021210079 A1 | 25 August 2022 |
| | | BR 112022014398 A2 | 13 September 2022 |
| | | EP 4094779 A1 | 30 November 2022 |
| | | TW 202140078 A | 01 November 2021 |
| | | JP 2023511163 A | 16 March 2023 |
| | | KR 20220130160 A | 26 September 2022 |
| | | CA 3168654 A1 | 29 July 2021 |
| US 2015056221 A1 | 26 February 2015 | UY 35712 A | 27 March 2015 |
| | | NZ 716612 A | 29 July 2022 |
| | | EA 201600190 A1 | 31 August 2016 |
| | | AU 2014308920 A1 | 03 March 2016 |
| | | AU 2014308920 B2 | 06 February 2020 |
| | | EP 3036257 A1 | 29 June 2016 |
| | | EP 3036257 B1 | 16 October 2019 |
| | | US 2016251442 A1 | 01 September 2016 |
| | | US 9688764 B2 | 27 June 2017 |
| | | CL 2016000376 A1 | 19 May 2017 |
| | | TW 201542592 A | 16 November 2015 |
| | | TWI 641620 B | 21 November 2018 |
| | | IL 243974 A0 | 21 April 2016 |
| | | IL 243974 B | 28 June 2018 |
| | | PH 12016500349 B1 | 16 May 2016 |
| | | JP 2020023511 A | 13 February 2020 |
| | | KR 20160036630 A | 04 April 2016 |
| | | KR 102343315 B1 | 27 December 2021 |
| | | US 2018094066 A1 | 05 April 2018 |
| | | US 10106616 B2 | 23 October 2018 |
| | | SG 11201601211 TA | 30 March 2016 |
| | | HK 1220206 A1 | 28 April 2017 |
| | | BR 112016003441 A2 | 05 December 2017 |
| | | US 9302015 B2 | 05 April 2016 |
| | | WO 2015026907 A1 | 26 February 2015 |
| | | CA 2920369 A1 | 26 February 2015 |
| | | CA 2920369 C | 14 March 2023 |
| | | JP 2016532703 A | 20 October 2016 |
| | | JP 6621412 B2 | 18 December 2019 |

Form PCT/ISA/210 (patent family annex) (July 2022)

**INTERNATIONAL SEARCH REPORT**
**Information on patent family members**

International application No.
**PCT/CN2023/073057**

| Patent document cited in search report | Publication date (day/month/year) | Patent family member(s) | Publication date (day/month/year) |
|---|---|---|---|
| | | MX 2016002150 A | 28 June 2016 |
| | | MX 370787 B | 06 January 2020 |
| | | MY 179851 A | 18 November 2020 |
| | | US 2019241667 A1 | 08 August 2019 |
| | | US 10683354 B2 | 16 June 2020 |
| US 2019167804 A1 | 06 June 2019 | LT 3658192 T | 12 July 2021 |
| | | CA 3082356 A1 | 06 June 2019 |
| | | CL 2020001452 A1 | 11 September 2020 |
| | | PT 3658192 T | 25 June 2021 |
| | | ZA 202003325 B | 28 July 2021 |
| | | UY 37991 A | 28 June 2019 |
| | | JP 2021054845 A | 08 April 2021 |
| | | SI 3658192 T1 | 31 August 2021 |
| | | CL 2021001075 A1 | 15 October 2021 |
| | | RS 61994 B1 | 30 July 2021 |
| | | MA 49796 A | 03 June 2020 |
| | | KR 20200095477 A | 10 August 2020 |
| | | PE 20201286 A1 | 24 November 2020 |
| | | JP 6813712 B2 | 13 January 2021 |
| | | JP 2021501124 A | 14 January 2021 |
| | | AU 2018374634 A1 | 28 May 2020 |
| | | US 2021015938 A1 | 21 January 2021 |
| | | HRP 20210917 T1 | 03 September 2021 |
| | | DK 3658192 T3 | 21 June 2021 |
| | | PL 3658192 T3 | 18 October 2021 |
| | | IL 274651 A | 30 June 2020 |
| | | IL 274651 B | 29 April 2021 |
| | | ECSP 20029001 A | 30 June 2020 |
| | | DOP 2020000116 A | 31 August 2020 |
| | | CO 2020006446 A2 | 09 June 2020 |
| | | EP 3658192 A1 | 03 June 2020 |
| | | EP 3658192 B1 | 28 April 2021 |
| | | MX 2020005583 A | 25 November 2020 |
| | | ES 2877659 T3 | 17 November 2021 |
| | | TW 201924723 A | 01 July 2019 |
| | | WO 2019106609 A1 | 06 June 2019 |
| | | BR 112020010694 A2 | 10 November 2020 |
| | | US 10772970 B2 | 15 September 2020 |
| | | CR 20200239 A | 21 September 2020 |
| | | EP 3884962 A1 | 29 September 2021 |
| | | RU 2020117698 A3 | 04 January 2022 |
| | | HUE 054428 T2 | 28 September 2021 |
| | | SG 11202004867 WA | 29 June 2020 |
| US 2020164085 A1 | 28 May 2020 | US 11510993 B2 | 29 November 2022 |
| | | US 2023099074 A1 | 30 March 2023 |
| | | EP 3359194 A2 | 15 August 2018 |
| | | EP 3359194 A4 | 24 April 2019 |
| | | WO 2017062271 A2 | 13 April 2017 |
| | | WO 2017062271 A3 | 01 June 2017 |
| WO 2020038454 A1 | 27 February 2020 | BR 112021002989 A2 | 11 May 2021 |

Form PCT/ISA/210 (patent family annex) (July 2022)

**INTERNATIONAL SEARCH REPORT**
**Information on patent family members**

International application No.
**PCT/CN2023/073057**

| Patent document cited in search report | Publication date (day/month/year) | Patent family member(s) | | | Publication date (day/month/year) |
|---|---|---|---|---|---|
| | | KR | 20210049792 | A | 06 May 2021 |
| | | AU | 2019324403 | A1 | 28 January 2021 |
| | | CA | 3105527 | A1 | 27 February 2020 |
| | | MX | 2021001143 | A | 12 April 2021 |
| | | EP | 3808774 | A1 | 21 April 2021 |
| | | EP | 3808774 | A4 | 18 August 2021 |
| | | JP | 2021533792 | A | 09 December 2021 |
| | | TW | 202023612 | A | 01 July 2020 |
| | | US | 2021238294 | A1 | 05 August 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010053751 A
- WO 2001092340 A
- WO 2008054606 A
- WO 2014031610 A
- WO 2020038454 A
- WO 2017210471 A
- WO 2019106609 A
- WO 2019136487 A
- US 20050238649 A1
- US 5208020 A
- WO 2020038454 A1
- WO 2020200960 A1


### Non-patent literature cited in the description


- *J. Biol. Chem*, 1968, vol. 243, 3558
- **CHARI et al.** *Cancer Research*, 1992, vol. 52, 127-131
- *J. biol. chem*, 1968, vol. 243, 3558
- Chinese Pharmacopoeia 0542. 2015
- **KABAT, E. A. et al.** Sequences of Proteins of Immunological Interest. 1991
- **QUEEN et al.** *Proc. Natl. Acad. Sci. USA*, 1991, vol. 88, 2869
- **JONES et al.** *Nature*, 1986, vol. 321, 522
- **RIECHMANN et al.** *Nature*, 1988, vol. 332, 323-327
- **VERHOEYEN et al.** *Science*, 1988, vol. 239, 1534
- **WARD et al.** *Nature*, 1989, vol. 341, 544-546
- **BIRD et al.** *Science*, 1988, vol. 242, 423-426
- **HUSTON et al.** *Proc. Natl. Acad. Sci. USA*, 1988, vol. 85, 5879-5883
- **HOLLIGER**. *Proc. Natl. Acad. Sci. USA*, 1993, vol. 90, 6444-6448
- **ALFTHAN et al.** *Protein Eng*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol*, 2001
- **KABAT E.A. et al.** *Sequences of proteins of immunological interest*, 1991
- **KABAT et al.** Sequences of Proteins of Immunological Interest. Public Health Service, National Institutes of Health, 1991
- **AL-LAZIKANI et al.** *JMB*, 1997, vol. 273, 927-948
- **LEFRANC M.P**. *Immunologist*, 1999, vol. 7, 132-136
- **LEFRANC, M.P. et al.** *Dev. Comp. Immunol.*, 2003, vol. 27, 55-77
- Epitope Mapping Protocols in Methods in Molecular Biology. 1996, vol. 66
- Antibodies: A Laboratory Manual. Cold Spring Harbor Press
- **ED. T. W. GREENE ; P. G. M. WUTS**. Protective Groups in Organic Synthesis
- *Tetrahedron*, 2018, vol. 74 (15), 1951-1956